# EUROPEAN PATENT SPECIFICATION

(11) **EP 1 220 935 B1**
(45) Date of publication and mention of the grant of the patent: **19.07.2006**
(21) Application number: 00960923.1
(22) Date of filing: 28.09.2000
(51) Int. Cl.: C12N 15/82, C12N 15/29, A01H 5/00

(54) **SYNTHETIC AND CHIMERIC PROMOTERS, EXPRESSION CASSETTES, PLASMIDS, VECTORS, TRANSGENIC PLANTS AND SEEDS CONTAINING THEM, AND METHOD FOR PRODUCING THEM**
SYNTHETISCHE UND CHIMERE PROMOTOREN, EXPRESSIONSKASSETTEN, PLASMIDE, VEKTORE, TRANSGENE PFLANZEN UND DIESE ENTHALTENDE SAMEN, SOWIE VERFAHREN ZU IHRER HERSTELLUNG
PROMOTEURS SYNTHETIQUES ET CHIMERIQUES; CASSETTES D'EXPRESSION, PLASMIDES, VECTEURS, PLANTES ET GRAINES TRANSGENIQUES LES CONTENANT, ET LEUR PROCEDE D'OBTENTION

(30) Priority: 30.09.1999 FR 9912373
(43) Date of publication of application: 10.07.2002
(73) Proprietor: MERISTEM THERAPEUTICS, 63100 Clermont-Ferrand (FR)
(72) Inventor: GRUBER, Véronique, F-63400 Chamalières (FR); NORRE, Frédéric, F-63730 Mirefleurs (FR); THEISEN, Manfred, F-63400 Chamalières (FR)
(74) Representative: Thurgood, Alexander John
(86) International application number: PCT/IB2000/001383
(87) International publication number: WO 2001/023593

(56) References cited:
- WO-A-00/31274
- WO-A-00/31282
- WO-A-00/55331
- WO-A-97/20056
- WO-A-97/25419
- WO-A-98/03655
- WO-A-98/07747
- WO-A-99/03985
- WO-A-99/16890
- THOMAS M S ET AL: "IDENTIFICATION OF AN ENHANCER ELEMENT FOR THE ENDOSPERM-SPECIFIC EXPRESSION OF HIGH MOLECULAR WEIGHT GLUTENIN" PLANT CELL,US,AMERICAN SOCIETY OF PLANT PHYSIOLOGISTS, ROCKVILLE, MD, vol. 2, no. 12, 1 December 1990 (1990-12-01), pages 1171-1180, XP002049700 ISSN: 1040-4651
- P HILSON ET AL: "Fos and Jun oncogenes transactivate chimeric or native promoters containing AP1-GCN4 binding sites in plant cells" PLANT CELL,US,AMERICAN SOCIETY OF PLANT PHYSIOLOGISTS, ROCKVILLE, MD, vol. 2, no. 7, July 1990 (1990-07), pages 651-658-658, XP002103874 ISSN: 1040-4651
- HALFORD N G ET AL: "FUNCTIONAL ANALYSIS OF THE UPSTREAM REGIONS OF A SILENT AND AN EXPRESSED MEMBER OF A FAMILY OF WHEAT SEED PROTEIN GENES IN TRANSGENIC TOBACCO" PLANT SCIENCE,IE,LIMERICK, vol. 62, no. 2, 1989, pages 207-216, XP002049701 ISSN: 0168-9452
- DATABASE BIOSIS [Online] BIOSCIENCES INFORMATION SERVICE, PHILADELPHIA, PA, US; 1994 BLECHL ANN E ET AL: "A transient assay for promoter activity of wheat seed storage proteins genes and other genes expressed in developing endosperm." Database accession no. PREV199598005497 XP002144304 & PLANT SCIENCE (LIMERICK), vol. 102, no. 1, 1994, pages 69-80, ISSN: 0168-9452
- DATABASE EMBL [Online] ACCESSION NO:X12928, 28 September 1998 (1998-09-28) ANDERSON, O.D., ET AL.: "ANDERSON, O.D., ET AL." XP002144299 -& ANDERSON, O.D., ET AL.: "Nucleotide sequence of the two high-molecular-weight glutenin genes from the D-genome of a hexaploid bread wheat, Triticum aestivum L. cv Cheyenne" NUCLEIC ACIDS RESEARCH, vol. 17, 1989, pages 461-462, XP002144300
- DATABASE EMBL [Online] ACCESSION NO: Y10956, 10 February 1997 (1997-02-10) SCHLUMBAUM, A., ET AL.: "T.aestivum promoter region of HMW glutenin subunit gene, allele Glu-D1-1cs" XP002144301
- DATABASE BIOSIS [Online] BIOSCIENCES INFORMATION SERVICE, PHILADELPHIA, PA, US; April 1998 (1998-04) ANDERSON O D ET AL: "Conservation in wheat high-molecular-weight glutenin gene promoter sequences: Comparisons among loci and among alleles of the GLU-B1-1 locus." Database accession no. PREV199800271856 XP002144305 & THEORETICAL AND APPLIED GENETICS, vol. 96, no. 5, April 1998 (1998-04), pages 568-576, ISSN: 0040-5752 -& DATABASE EMBL [Online] ACCESSION NO:X13927, 19 February 1990 (1990-02-19) ANDERSON, O.D., ET AL.: "Wheat Glu-B1-1b gene for HMW glutenin subunit" XP002144302 -& DATABASE EMBL [Online] ACCESSION NO:M22209, 22 April 1989 (1989-04-22) ANDERSON, O.D., ET AL.: "Tricticum aestivum, high MW glutenin subunit (Bx7) gene, complete cds." XP002144303

### SEQUENCE LISTING

<110> MERISTEM THERAPEUTICS
<120> SYNTHETIC AND CHIMERIC PROMOTERS, EXPRESSION CASSETTES, PLASMIDS, VECTORS, TRANSGENIC PLANTS ET SEEDS INCLUDING THEM AND PROCESSES FOR PRODUCING THE SAME
<130> PrHMWG1
<140>
   <141>
<160> 37
<170> Patentln Ver. 2.1
<210> 1
   <211>417
   <212> DNA
   <213> Triticum aestivum
<220>
   <221> misc_feature
   <222> (22)..(29)
   <223> Prolamine- like box
<220>
   <221> misc_feature
   <222> (70)..(73)
   <223> GATA box
<220>
   <221> misc_feature
   <222> (87)..(90)
   <223> GATA box
<220>
   <221> misc_feature
   <222> (127)..(133)
   <223> Prolamine-like box
<220>
   <221> misc_feature
   <222> (161)..(168)
   <223> G-like box
<220>
   <221> enhancer
   <222> (193)..(230)
   <223> Enhancer box
<220>
   <221> TATA_signal
   <222> (349)..(355)
   <223> TATA box
<220>
   <221> misc_feature
   <222> (379)
   <223> Transcription initiation site
<400> 1
   agctttgagt ggccgtagat ttgcaaaagc aatggctaac agacacatat tctgccaaac 60
   cccaagaagg ataatcactt ttcttagata aaaaagaaca gaccaatata caaacatcca 120
   cacttctgca aacaatacat cagaactagg attacgccga ttacgtggct ttagcagact 180
   gtccaaaaat ctgttttgca aagctccaat tgctccttgc ttatccagct tcttttgtgt 240
   tggcaaactg cgcttttcca accgattttg ttcttctcgc gctttcttct taggctaaac 300
   aaacctcacc gtgcacgcag ccatggtcct gaaccttcac ctcgtcccta taaaagccta 360
   gccaaccttc acaatcttat catcacccac aacaccgagc accacaaact agagatc 417
<210> 2
   <211> 181
   <212> DNA
   <213> Artificial Sequence
<220>
   <223> Description of Artificial Sequence:MPr1126 promoter
<220>
   <221> TATA_signal
   <222> (113)..(119)
   <223> TATA box
<220>
   <221> misc_feature
   <222>(143)
   <223> Transcription Initiation site
<400> 2
   gtgttggcaa actgcgcttt tccaaccgat tttgttcttc tcgcgctttc ttcttaggct 60
   aaacaaacct caccgtgcac gcagccatgg tcctgaacct tcacctcgtc cctataaaag 120
   cctagccaac cttcacaatc ttatcatcac ccacaacacc gagcaccaca aactagagat 180
   c 181
<210> 3
   <211> 244
   <212> DNA
   <213> Artificial Sequence
<220>
   <223> Description of Artificial Sequence:MPr1 127 promoter
<220>
   <221> enhancer
   <222> (20)..(57)
   <223> Enhancer box
<220>
   <221> TATA_signal
   <222> (176)..(182)
   <223> TATA box
<220>
   <221> misc_feature
   <222> (206)
   <223> Transcription Initiation site
<400> 3
   gcagactgtc caaaaatctg ttttgcaaag ctccaattgc tccttgctta tccagcttct 60
   tttgtgttgg caaactgcgc ttttccaacc gattttgttc ttctcgcgct ttcttcttag 120
   gctaaacaaa cctcaccgtg cacgcagcca tggtcctgaa ccttcacctc gtccctataa 180
   aagcctagcc aaccttcaca atcttatcat cacccacaac accgagcacc acaaactaga 240
   gatc 244
<210> 4
   <211> 277
   <212> DNA
   <213> Artificial Sequence
<220>
   <223> Description of Artificial Sequence:MPr1128 promoter
<220>
   <221> misc_feature
   <222> (21)..(28)
   <223> G-like box
<220>
   <221> enhancer
   <222> (53)..(90)
   <223> Enhancer box
<220>
   <221> TATA_signal
   <222> (209)..(215)
   <223> TATA box
<220>
   <221> misc_feature
   <222> (239)
   <223> Transcription Initiation Site
<400> 4
   cagaactagg attacgccga ttacgtggct ttagcagact gtccaaaaat ctgttttgca 60
   aagctccaat tgctccttgc ttatccagct tcttttgtgt tggcaaactg cgcttttcca 120
   accgattttg ttcttctcgc gctttcttct taggctaaac aaacctcacc gtgcacgcag 180
   ccatggtcct gaaccttcac ctcgtcccta taaaagccta gccaaccttc acaatcttat 240
   catcacccac aacaccgagc accacaaact agagatc 277
<210> 5
   <211> 472
   <212> DNA
   <213> Artificial Sequence
<220>
   <223> Description of Artificial Sequence:MPr1130 promoter
<220>
   <221> misc_feature
   <222> (22)..(29)
   <223> Prolamine-like box
<220>
   <221> misc_feature
   <222> (70)..(73)
   <223> GATA box
<220>
   <221> misc_feature
   <222> (87)..(90)
   <223> GATA box
<220>
   <221> misc_feature
   <222> (127)..(133)
   <223> Prolamine-like box
<220>
   <221> misc_feature
   <222> (161)..(168)
   <223> G-like box
<220>
   <221 > enhancer
   <222> (193)..(230)
   <223> Enhancer box
<220>
   <221> misc_feature
   <222> (314)..(368)
   <223> As2/As2/As1 box
<220>
   <221> TATA_signat
   <222> (404)..(410)
   <223> TATA box
<220>
   <221> misc_feature
   <222> (434)
   <223> Transcription Initiation Site
<400> 5
   agctttgagt ggccgtagat ttgcaaaagc aatggctaac agacacatat tctgccaaac 60
   cccaagaagg ataatcactt ttcttagata aaaaagaaca gaccaatata caaacatcca 120
   cacttctgca aacaatacat cagaactagg attacgccga ttacgtggct ttagcagact 180
   gtccaaaaat ctgttttgca aagctccaat tgctccttgc ttatccagct tcttttgtgt 240
   tggcaaactg cgcttttcca accgattttg ttcttctcgc gctttcttct taggctaaac 300
   aaacctcacc gtgattgatg tgatatcaag attgatgtga tatctccact gacgtaaggg 360
   atgacgcaca cgcagccatg gtcctgaacc ttcacctcgt ccctataaaa gcctagccaa 420
   ccttcacaat cttatcatca cccacaacac cgagcaccac aaactagaga tc 472
<210> 6
   <211> 455
   <212> DNA
   <213> Artificial Sequence
<220>
   <223> Description of Artificial Sequence:MPr1131 promoter
<220>
   <221> misc_feature
   <222> (22)..(29)
   <223> Prolamine-like box
<220>
   <221> misc_feature
   <222> (70)..(73)
   <223> GATA box
<220>
   <221> misc_feature
   <222> (87)..(90)
   <223> GATA box
<220>
   <221> misc_feature
   <222> (127)..(133)
   <223> Prolamine-like box
<220>
   <221> misc_feature
   <222> (161)..(168)
   <223> G-like box
<220>
   <221> enhancer
   <222> ()..)
   <223> Enhancer box
<220>
   <221> misc feature
   <222> ()..)
   <223> As2/As1 box
<220>
   <221> TATA_signal
   <222> ()..(393)
   <223> TATA box
<220>
   <221> misc_feature
   <222> ()
   <223> Transcription Initiation Site
<400> 6
   agctttgagt ggccgtagat ttgcaaaagc aatggctaac agacacatat tctgccaaac 60
   cccaagaagg ataatcactt ttcttagata aaaaagaaca gaccaatata caaacatcca 120
   cacttctgca aacaatacat cagaactagg attacgccga ttacgtggct ttagcagact 180
   gtccaaaaat ctgttttgca aagctccaat tgctccttgc ttatccagct tcttttgtgt 240
   tggcaaactg cgcttttcca accgattttg ttcttctcgc gctttcttct taggctaaac 300
   aaacctcacc gtgattgatg tgatatctcc actgacgtaa gggatgacgc acacgcagcc 360
   atggtcctga accttcacct cgtccctata aaagcctagc caaccttcac aatcttatca 420
   tcacccacaa caccgagcac cacaaactag agatc 455
<210>7
   <211> 332
   <212> DNA
   <213> Artificial Sequence
<220>
   <223> Description of Artificial Sequence:MPr1135 promoter
<220>
   <221> misc_feature
   <222> (21)..(28)
   <223> G-like box
<220>
   <221> enhancer
   <222> (53)..(90)
   <223> Enhancer box
<220>
   <221> misc_feature
   <222> (174)..(228)
   <223> As2/As2/As1 box
<220>
   <221> TATA_signal
   <222> (264)..(270)
   <223> TATA box
<220>
   <221> misc_feature
   <222> (294)
   <223> Transcription Initiation Site
<400> 7
   cagaactagg attacgccga ttacgtggct ttagcagact gtccaaaaat ctgttttgca 60
   aagctccaat tgctccttgc ttatccagct tcttttgtgt tggcaaactg cgcttttcca 120
   accgattttg ttcttctcgc gctttcttct taggctaaac aaacctcacc gtgattgatg 180
   tgatatcaag attgatgtga tatctccact gacgtaaggg atgacgcaca cgcagccatg 240
   gtcctgaacc ttcacctcgt ccctataaaa gcctagccaa ccttcacaat cttatcatca 300
   cccacaacac cgagcaccac aaactagaga tc 332
<210> 8
   <211> 219
   <212> DNA
   <213> Artificial Sequence
<220>
   <223> Description of Artificial Sequence:MPr1136 promoter
<220>
   <221> misc_feature
   <222> (78)..(115)
   <223> As2/As1 box
<220>
   <221> TATA_signal
   <222> (151)..(157)
   <223> TATA box
<220>
   <221> misc_feature
   <222>(181)
   <223> Transcription Initiation Site
<400> 8
   gtgttggcaa actgcgcttt tccaaccgat tttgttcttc tcgcgctttc ttcttaggct 60
   aaacaaacct caccgtgatt gatgtgatat ctccactgac gtaagggatg acgcacacgc 120
   agccatggtc ctgaaccttc acctcgtccc tataaaagcc tagccaacct tcacaatctt 180
   atcatcaccc acaacaccga gcaccacaaa ctagagatc 219
<210>9
   <211> 282
   <212> DNA
   <213> Artificial Sequence
<220>
   <223> Description of Artificial Sequence:MPr1 137 promoter
<220>
   <221> enhancer
   <222> (20)..(57)
   <223> Enhancer box
<220>
   <221> misc_feature
   <222> (141)..(178)
   <223> As2/As1 box
<220>
   <221> TATA_signal
   <222> (214)..(220)
   <223> TATA box
<220>
   <221> misc_feature
   <222> (244)
   <223> Transcription Initiation Site
<400> 9
   gcagactgtc caaaaatctg ttttgcaaag ctccaattgc tccttgctta tccagcttct 60
   tttgtgttgg caaactgcgc ttttccaacc gattttgttc ttctcgcgct ttcttcttag 120
   gctaaacaaa cctcaccgtg attgatgtga tatctccact gacgtaaggg atgacgcaca 180
   cgcagccatg gtcctgaacc ttcacctcgt ccctataaaa gcctagccaa ccttcacaat 240
   cttatcatca cccacaacac cgagcaccac aaactagaga tc 282
<210> 10
   <211> 315
   <212> DNA
   <213> Artificial Sequence
<220>
   <223> Description of Artificial Sequence:MPr1138 promoter
<220>
   <221> misc_feature
   <222> (21)..(28)
   <223> G-like box
<220>
   <221> enhancer
   <122> (53)..(90)
   <223> Enhancer box
<220>
   <221> misc_feature
   <222> (174)..(211)
   <223> As2/As1 box
<220>
   <221> TATA_signal
   <222> (247)..(253)
   <223> TATA box
<220>
   <221> misc_feature
   <222> (277)
   <223> Transcription Initiation Site
<400> 10
   cagaactagg attacgccga ttacgtggct ttagcagact gtccaaaaat ctgttttgca 60
   aagctccaat tgctccttgc ttatccagct tcttttgtgt tggcaaactg cgcttttcca 120
   accgattttg ttcttctcgc gctttcttct taggctaaac aaacctcacc gtgattgatg 180
   tgatatctcc actgacgtaa gggatgacgc acacgcagcc atggtcctga accttcacct 240
   cgtccctata aaagcctagc caaccttcac aatcttatca tcacccacaa caccgagcac 300
   cacaaactag agatc                                                  315
<210> 11
   <211> 505
   <212> DNA
   <213> Artificial Sequence
<220>
   <223> Description of Artificial Sequence:MPr1139 promoter
<220>
   <221> misc_feature
   <222> (4)..(26)
   <223> Cereal box
<220>
   <221> misc_feature
   <222> (39)..(61)
   <223> Cereal box
<220>
   <221> misc_feature
   <222> (120)..(123)
   <223> GATA box
<220>
   <221> misc_feature
   <222> (137)..(140)
   <223> GATA box
<220>
   <221> misc_feature
   <222> (72)..(79)
   <223> Prolamine-like box
<220>
   <221> misc_feature
   <222> (177)..(183)
   <223> Prolamine like box
<220>
   <221> misc_feature
   <222> (211)..(218)
   <223> G-like box
<220>
   <221> enhancer
   <222> (243)..(280)
   <223> Enhancer box
<220>
   <221> misc_feature
   <222> (364)..(401)
   <223> As2/As1 box
<220>
   <221> TATA_signal
   <222> (437)..(443)
   <223> TATA box
<220>
   <221> misc_feature
   <222> (467)
   <223> Transcription Initiation Site
<400> 11
   ctcgacatgg ttagaagttt tgagtgccgc cactactcga catggttaga agttttgagt 60
   ggccgtagat ttgcaaaagc aatggctaac agacacatat tctgccaaac cccaagaagg 120
   ataatcactt ttcttagata aaaaagaaca gaccaatata caaacatcca cacttctgca 180
   aacaatacat cagaactagg attacgccga ttacgtggct ttagcagact gtccaaaaat 240
   ctgttttgca aagctccaat tgctccttgc ttatccagct tcttttgtgt tggcaaactg 300
   cgcttttcca accgattttg ttcttctcgc gctttcttct taggctaaac aaacctcacc 360
   gtgattgatg tgatatctcc actgacgtaa gggatgacgc acacgcagcc atggtcctga 420
   accttcacct cgtccctata aaagcctagc caaccttcac aatcttatca tcacccacaa 480
   caccgagcac cacaaactag agatc 505
<210> 12
   <211> 96
   <212> DNA
   <213> Artificial Sequence
<220>
   <223> Description of Artificial Sequence:MPr1197 promoter
<220>
   <221> TATA_signal
   <222> (28)..(34)
   <223> TATA box
<220>
   <221> misc_feature
   <222> (58)
   <223> Transcription Initiation Site
   <400> 12
   catggtcctg aaccttcacc tcgtccctat aaaagcctag ccaaccttca caatcttatc 60
   atcacccaca acaccgagca ccacaaacta gagatc 96
<210> 13
   <211> 187
   <212> DNA
   <213> Artificial Sequence
<220>
   <223> Description of Artificial Sequence:MPr1198 promoter
<220>
   <221> misc_feature
   <222> (8)..(15)
   <223> G-like box
<220>
   <221> enhancer
   <222> (40)..(77)
   <223> Enhancer box
<220>
   <221> TATA_signal
   <222> (119)..(125)
   <223> TATA box
<220>
   <221> misc_feature
   <222>(149)
   <223> Transcription Initiation Site
<400> 13
   acgccgatta cgtggcttta gcagactgtc caaaaatctg ttttgcaaag ctccaattgc 60
   tccttgctta tccagcttct tttgtgttgg ccatggtcct gaaccttcac ctcgtcccta 120
   taaaagccta gccaaccttc acaatcttat catcacccac aacaccgagc accacaaact 180
   agagatc 187
<210> 14
   <211> 290
   <212> DNA
   <213> Artificial Sequence
<220>
   <223> Description of Artificial Sequence:MPr1199 promotoer
<220>
   <221> misc_feature
   <222> (6)..(25)
   <223> GC rich box
<220>
   <221> misc_feature
   <222> (34)..(41)
   <223> G-like box
<220>
   <221> enhancer
   <222> (66)..(103)
   <223> Enhancer box
<220>
   <221> TATA_signal
   <222> (222)..(228)
   <223> TATA box
<220>
   <221> misc_feature
   <222> (252)
   <223> Transcription Initiation Site
<400> 14
   caaatgggcc ggaccgggcc ggcccagcgc cgattacgtg gctttagcag actgtccaaa 60
   aatctgtttt gcaaagctcc aattgctcct tgcttatcca gcttcttttg tgttggcaaa 120
   ctgcgctttt ccaaccgatt ttgttcttct cgcgctttct tcttaggcta aacaaacctc 180
   accgtgcacg cagccatggt cctgaacctt cacctcgtcc ctataaaagc ctagccaacc 240
   ttcacaatct tatcatcacc cacaacaccg agcaccacaa actagagatc 290
<210> 15
   <211> 381
   <212> DNA
   <213> Artificial Sequence
<220>
   <223> Description of Artificial Sequence:MPr1200
<220>
   <221> misc_feature
   <222> (4)..(26)
   <223> Cereal box
<220>
   <221> misc_feature
   <222> (39)..(61)
   <223> Cereal box
<220>
   <221> misc_feature
   <222> (87)..(94)
   <223> G-like box
<220>
   <221> enhancer
   <222> (119)..(156)
   <223> Enhancer box
<220>
   <221> misc_feature
   <222> (240)..(277)
   <223> As2/As1 box
<220>
   <221> TATA_signal
   <222> (313)..(319)
   <223> TATA box
<220>
   <221> misc_feature
   <222> (343)
   <223> Transcription Initiation Site
<400> 15
   ctcgacatgg ttagaagttt tgagtgccgc cactactcga catggttaga agttttgagt 60
   ggccgtagat ttgctctaga cgccgattac gtggctttag cagactgtcc aaaaatctgt 120
   tttgcaaagc tccaattgct ccttgcttat ccagcttctt ttgtgttggc aaactgcgct 180
   tttccaaccg attttgttct tctcgcgctt tcttcttagg ctaaacaaac ctcaccgtga 240
   ttgatgtgat atctccactg acgtaaggga tgacgcacac gcagccatgg tcctgaacct 300
   tcacctcgtc cctataaaag cctagccaac cttcacaatc ttatcatcac ccacaacacc 360
   gagcaccaca aactagagat c 381
<210> 16
   <211> 343
   <212> DNA
   <213> Artificial Sequence
<220>
   <223> Description of Artificial Sequence:MPr1213 Promoter
<220>
   <221> misc_feature
   <222> (4)..(26)
   <223> Cereal box
<220>
   <221> misc_feature
   <222> (39)..(61)
   <223> Cereal box
<220>
   <221> enhancer
   <222> (119)..(156)
   <223> Enhancer box
<220>
   <221> misc_feature
   <222> (87)..(94)
   <223> G-like box
<220>
   <221> TATA_signal
   <222> (275)..(281)
   <223> TATA box
<220>
   <221> misc_feature
   <222> (305)
   <223> Transcription Initiation Site
<400> 16
   ctcgacatgg ttagaagttt tgagtgccgc cactactcga catggttaga agttttgagt 60
   ggccgtagat ttgctctaga cgccgattac gtggctttag cagactgtcc aaaaatctgt 120
   tttgcaaagc tccaattgct ccttgcttat ccagcttctt ttgtgttggc aaactgcgct 180
   tttccaaccg attttgttct tctcgcgctt tcttcttagg ctaaacaaac ctcaccgtgc 240
   acgcagccat ggtcctgaac cttcacctcg tccctataaa agcctagcca accttcacaa 300
   tcttatcatc acccacaaca ccgagcacca caaactagag atc                  343
<210> 17
   <211> 358
   <212> DNA
   <213> Artificial Sequence
<220>
   <223> Description of Artificial Sequence:MPr1216 promoter
<220>
   <221> misc_feature
   <222> (7)..(14)
   <223> G-like box
<220>
   <221> enhancer
   <222> (39)..(76)
   <223> Enhancer box
<220>
   <221> misc_feature
   <222> (102)..(109)
   <223> G-like box
<220>
   <221> enhancer
   <222> (134)..(171)
   <223> Enhancer box
<220>
   <221> TATA_signal
   <222> (290)..(296)
   <223> TATA box
<220>
   <221> misc_feature
   <222> (320)
   <223> Transcription Initiation Site
<400> 17
   cgccgattac gtggctttag cagactgtcc aaaaatctgt tttgcaaagc tccaattgct 60
   ccttgcttat ccagcttctt ttgtgttggt ctagacgccg attacgtggc tttagcagac 120
   tgtccaaaaa tctgttttgc aaagctccaa ttgctccttg cttatccagc ttcttttgtg 180
   ttggcaaact gcgcttttcc aaccgatttt gttcttctcg cgctttcttc ttaggctaaa 240
   caaacctcac cgtgcacgca gccatggtcc tgaaccttca cctcgtccct ataaaagcct 300
   agccaacctt cacaatctta tcatcaccca caacaccgag caccacaaac tagagatc 358
<210> 18
   <211> 25
   <212> DNA
   <213> Artificial Sequence
<220>
   <223> Description of Artificial Sequence:Oligodesoxynucleotide
<400> 18
   atcggaattc gtgttggcaa actgc 25
<210> 19
   <211> 29
   <212> DNA
   <213> Artificial Sequence
<220>
   <223> Description of Artificial Sequence:Oligodesoxynucleotide
<400> 19
   atcgggaatt cgcagactgt ccaaaaatc 2 9
<210> 20
   <211> 29
   <212> DNA
   <213> Artificial Sequence
<220>
   <223> Description of Artificial Sequence:Oligodesoxynucleotide
<400> 20
   atcggaattc cagaactagg attacgccg 29
<210> 21
   <211>29
   <212> DNA
   <213> Artificial Sequence
<220>
   <223> Description of Artificial Sequence:Oligodesoxynucleotide
<400> 21
   tacgaattcc cagctttgag tggccgtag 29
<210> 22
   <211> 35
   <212> DNA
   <213> Artificial Sequence
<220>
   <223> Description of Artificial Sequence:Oligodesoxynucleotide
<400> 22
   atcggaattc tagacgccga ttacgtggct ttagc 35
<210> 23
   <211> 83
   <212> DNA
   <213> Artificial Sequence
<220>
   <223> Description of Artificial Sequence:Oligodesoxynucleotide
<400> 23
   tacgaattcc tcgacatggt tagaagtttt gagtgccgcc actactcgac atggttagaa 60
   gttttgagtg gccgtagatt tgc 83
<210> 24
   <211> 30
   <212> DNA
   <213> Artificial Sequence
<220>
   <223> Description of Artificial Sequence:Oligodesoxynucleotide
<400> 24
   atcggaattc gccgattacg tggctttagc 30
<210> 25
   <211> 29
   <212> DNA
   <213> Artificial Sequence
<220>
   <223> Description of Artificial Sequence:Oligodesoxynucleotide
<400> 25
   atcggaattc gcagccatgg tcctgaacc 29
<210> 26
   <211> 19
   <212> DNA
   <213> Artificial Sequence
<220>
   <223> Description of Artificial Sequence:Oligodesoxynucleotide
<400> 26
   tacgaattcc tcgacatgg 19
<210> 27
   <211> 63
   <212> DNA
   <213> Artificial Sequence
<220>
   <223> Description of Artificial Sequence:Oligodesoxynucleotide
<400> 27
   attgatgtga tatctccact gacgtaaggg atgacgcaca cgcagccatg gtcctgaacc 60
   ttc 63
<210> 28
   <211> 80
   <212> DNA
   <213> Artificial Sequence
<220>
   <223> Description of Artificial Sequence:Oligodesoxynucleotide
<400> 28
   attgatgtga tatcaagatt gatgtgatat ctccactgac gtaagggatg acgcacacgc 60
   agccatggtc ctgaaccttc 80
<210> 29
   <211> 33
   <212> DNA
   <213> Artificial Sequence
<220>
   <223> Description of Artificial Sequence:Oligodesoxynucleotide
<400> 29
   tacggatccc cggggatctc tagtttgtgg tgc 33
<210> 30
   <211> 26
   <212> DNA
   <213> Artificial Sequence
<220>
   <223> Description of Artificial Sequence:Oligodesoxynucleotide
<400> 30
   gctctagagc aaatctacgg ccactc 26
<210> 31
   <211> 27
   <212> DNA
   <213> Artificial Sequence
<220>
   <223> Description of Artificial Sequence:Oligodesoxynucleotide
<400> 31
   gctctagacc aacacaaaag aagctgg 27
<210> 32
   <211> 29
   <212> DNA
   <213> Artificial Sequence
<220>
   <223> Description of Artificial Sequence:Oligodesoxynucleotide
<400> 32
   catgccatgg ccaacacaaa agaagctgg 29
<210> 33
   <211> 63
   <212> DNA
   <213> Artificial Sequence
<220>
   <223> Description of Artificial Sequence:Oligodesoxynucleotide
<400> 33
   tgcgtcatcc cttacgtcag tggagatatc acatcaatca cggtgaggtt tgtttagcct 60
   aag 63
<210> 34
   <211> 80
   <212> DNA
   <213> Artificial Sequence
<220>
   <223> Description of Artificial Sequence:Oligodesoxynucleotide
<400> 34
   tgcgtcatcc cttacgtcag tggagatatc acatcaatct tgatatcaca tcaatcacgg 60
   tgaggtttgt ttagcctaag 80
<210> 35
   <211> 236
   <212> DNA
   <213> Artificial Sequence
<220>
   <223> Description of Artificial Sequence:MPr1133 promoter
<220>
   <221 > misc_feature
   <222> (78)..(132)
   <223> As2/As21As1 box
<220>
   <221> misc_feature
   <222> (198)
   <223> Transcription Initiation Site
<400> 35
   gtgttggcaa actgcgcttt tccaaccgat tttgttcttc tcgcgctttc ttcttaggct 60
   aaacaaacct caccgtgatt gatgtgatat caagattgat gtgatatctc cactgacgta 120
   agggatgacg cacacgcagc catggtcctg aaccttcacc tcgtccctat aaaagcctag 180
   ccaaccttca caatcttatc atcacccaca acaccgagca ccacaaacta gagatc 236
<210> 36
   <211> 299
   <212> DNA
   <213> Artificial Sequence
<220>
   <223> Description of Artificial Sequence:MPr1134
<220>
   <221> enhancer
   <222> (20)..(57)
   <223> Enhancer box
<220>
   <221> misc_feature
   <222> (141)..(195)
   <223> As2/As2/As1 box
<220>
   <221> misc_feature
   <222> (261)
   <223> Transcription Initiation Site
<400> 36
   gcagactgtc caaaaatctg ttttgcaaag ctccaattgc tccttgctta tccagcttct 60
   tttgtgttgg caaactgcgc ttttccaacc gattttgttc ttctcgcgct ttcttcttag 120
   gctaaacaaa cctcaccgtg attgatgtga tatcaagatt gatgtgatat ctccactgac 180
   gtaagggatg acgcacacgc agccatggtc ctgaaccttc acctcgtccc tataaaagcc 240
   tagccaacct tcacaatctt atcatcaccc acaacaccga gcaccacaaa ctagagatc 299
<210> 37
   <211> 453
   <212> DNA
   <213> Artificial Sequence
<220>
   <223> Description of Artificial Sequence:MPr1217 promoter
<220>
   <221> misc_feature
   <222> (8)..(15)
   <223> G-like box
<220>
   <221> enhancer
   <222> (40)..(77)
   <223> Enhancer box
<220>
   <221> misc_feature
   <222> (102)..(109)
   <223> G-like box
<220>
   <221> enhancer
   <222> (134)..(171)
   <223> Enhancer box
<220>
   <221> misc_feature
   <222> (197)..(204)
   <223> G-like box
<220>
   <221> enhancer
   <222> (229)..(266)
   <223> Enhancer box
<220>
   <221> TATA_signal
   <222> (385)..(391)
   <223> TATA box
<220>
   <221> misc_feature
   <222> (415)
   <223> Transcription Initiation Site
<400> 37
   acgccgatta cgtggcttta gcagactgtc caaaaatctg ttttgcaaag ctccaattgc 60
   tccttgctta tccagcttct tttgtgttgg tctagacgcg attacgtggc tttagcagac 120
   tgtccaaaaa tctgttttgc aaagctccaa ttgctccttg cttatccagc ttcttttgtg 180
   ttggtctaga cgccgattac gtggctttag cagactgtcc aaaaatctgt tttgcaaagc 240
   tccaattgct ccttgcttat ccagcttctt ttgtgttggc aaactgcgct tttccaaccg 300
   attttgttct tctcgcgctt tcttcttagg ctaaacaaac ctcaccgtgc acgcagccat 360
   ggtcctgaac cttcacctcg tccctataaa agcctagcca accttcacaa tcttatcatc 420
   acccacaaca ccgagcacca caaactagag atc                             453

## Description

The present invention relates to chimeric promoters of expression, intended in particular for use in the field of plant biotechnology.

In general, the promoters of expression are known in the field of biotechnology and genetic manipulation. With regard more particularly to plant biotechnology, the level of expression of a gene encoding a polypeptide to be produced in a host cell is often dependent on the promoter used. The various promoters commonly used are often limited to specific applications or tissues, because of their tissue specificity or strength of expression. Mention may be made, for example, of the 35S promoter of the cauliflower mosaic virus, as a promoter which is relatively strong compared, for example, with the promoter originating from the *nos* gene, these two promoters being more particularly used in the field of plant biotechnology. There is thus a need for novel promoters which make it possible to overcome the drawbacks of using the current promoters.

An attempt at satisfying this need has been reported in the PCT patent application published under the number WO 97/20056, which describes increasing the level of gene expression by using enhancers, (i.e. having a positive effect on the activity of a promoter), in known promoters. The nucleotide sequences of enhancers are rich in A and T bases, the total amount of these bases constituting more than 50% of the nucleotide sequence of the enhancer. In particular, the Applicants of this application recommend the use of an enhancer region originating from the plastocyanine promoter of pea.

The expressions used in the description and claims have the following meaning:
- the term "nucleic acid" means DNA or RNA;
- the term "nucleic acid sequence" means a single- or doublestranded oligomer or polymer of nucleotide bases read from the 5' end towards the 3' end, which comprises self-replicating plasmids, genes and DNA or RNA polymers which are or are not infectious, and functional or nonfunctional DNA or RNA. In the nucleotide notation used in the present application, except where specifically mentioned, the left-hand end of a single-stranded nucleotide sequence is the 5' end;
- the phrase "nucleic acid sequence derived" means that the sequence is derived directly or indirectly from the sequence to which reference is made, for example by substitution, deletion, addition, mutation, fragmentation and/or synthesis of one or more nucleotides;
- the term "promoter" or the phrase "promoter nucleic acid sequence" means a nucleic acid region which is upstream of the translation start codon, and which is involved in the recognition and binding of RNA polymerase and of other proteins for transcription;
- "plant promoter" is a promoter which is capable of initiating transcription in plant cells;
- "constitutive promoter" is a promoter which is capable of expressing nucleic acid sequences which are linked in a functional manner to said promoter, in all or practically all the tissues of the host organism throughout the development of said organism;
- "specific tissue promoter" is a promoter which is capable of selectively expressing nucleic acid sequences which are linked in functional manner to said promoter, in certain specific tissues of the host organism;
- the phrase "linked in a functional manner" or "functionally linked" means the linking of a nucleic acid sequence, for example a promoter or a regulatory or functional box, to another nucleic acid sequence, for example another regulatory or functional box or a gene to be expressed which encodes a protein to be produced, such that the sequences exert their primary functions in a cell, a genome, a vector or an expression cassette into which they have been introduced, i.e. that they are functional in such environments. It should also be understood that, in the case of a promoter, the sequence of the promoter also includes sequences transcribed between the transcription start site and the translation start site;
- the term "expression cassette" means nucleotide sequences which are capable of directing the expression of a nucleic acid sequence, or of a gene, encoding a polypeptide to be produced in a host organism which is compatible with such sequences. Such cassettes include at least one promoter and one transcription termination signal, and optionally other factors which are required or useful for expression;
- the term "vector" means expression systems, for example DNA-coated projectiles, nucleic acid-based transit vehicles and nucleic acid molecules which have been adapted to deliver the nucleic acid, and autonomous self-replicating circular DNA, for example plasmids, cosmids, phagemids, etc. If a recombinant cell culture or microorganism is described as being the host of an "expression vector", this includes extrachromosomal circular DNA (such as, for example, mitochondrial or chloroplastic DNA) and DNA which has been integrated into the host chromosome(s), the vector being able to be either replicated stabily by the cells during mitosis, as an autonomous structure which is integrated into the genome of the host, or maintained in the nucleus or cytoplasm of the host;
- the term "plasmid" means an autonomous circular DNA molecular capable of replicating in a cell, and comprises both the so-called "expression" plasmids and the so-called "nonexpression" plasmids. If a recombinant cell culture or microorganism is described as being the host of an "expression" plasmid, this comprises both extrachromosomal circular DNA molecules and DNA which has been integrated into the host chromosome(s). If the plasmid is maintained by a host cell, the plasmid is either stabily replicated by the cells during mitosis, as an autonomous structure, or integrated into the genome of the host;
- the term "heterologous sequence" or "heterologous nucleic acid sequence" means a sequence originating from a source, or from a species, which is foreign to its environment or, if it originates from the same environment, has been modified with respect to its original form. The modification of the nucleic acid sequence can take place, for example, by treating the nucleic acid with a restriction enzyme so as to generate a nucleic acid fragment which is capable of being linked in a functional manner to a promoter. The modification can also take place via techniques such as site-direct mutagenesis;
- the term "box" means a nucleic acid sequence to which a regulatory function is attributed;
- the term "like" means that the box and/or the nucleic acid sequence with which this term is associated comprises a certain sequence identity or a consensus with a box and/or a known so-called reference nucleic acid sequence, preferably a sequence identity of at least 50%, more preferably a sequence identity of at least 75%, and more particularly a sequence identity of at least 90% with the reference sequence. The percentage sequence identity is calculated on the basis of a window of comparison of at least 6 nucleotide bases. The determination of a window of comparison can be carried out using sequence alignment algorithms in order to determine homology with a reference sequence, for example the local homology algorithm, the homology alignment algorithm, and the similarity search algorithm, these algorithms also existing in computer form, known under the names GAP, BESTFIT, FASTA and TFASTA. The percentage sequence identity is obtained by comparing the reference sequence with the box and/or the nucleic acid sequence;
- the term "located" means the position on a nucleic acid sequence of an identified element, such as a "box", a restriction site or a codon having a specific function. The position, which is given by a number, refers to the position of the start of the element in the nucleic acid sequence, except where specifically mentioned, in the direction of reading of the latter, i.e. in the 5'->3' direction;
- the term "Element 300", "EM", "endosperm motif", "P-box" or "Prolamine-like" box means a regulatory or functional motif or element which encodes storage proteins of many cereals, and which is involved in the common regulatory mechanism mediating the coordinated expression of zein genes during the development of the maize albumen;
- the term "G-like" box means an ACGT core motif, the functional contribution to transcriptional regulation of which has been defined in few cases, but which appears to be necessary for maximum expression of a promoter;
- the term "enhancer" box means regulatory DNA sequences which can act in cis at a distance, independently of their orientation and upstream or downstream of their target promoter, and which can generally consist of multiple short motifs which bind a combination of trans factors so as to possibly confer inductibility, tissue specificity or a general improvement in the activity of a promoter;
- the term "GATA" box means a regulatory or functional motif or element which may be required for the activity of many light-regulated promoters;
- the term "as1" or "activating sequence 1" box means a regulatory or functional motif or element preferably originating from the 35S promoter of the CaMV (cauliflower mosaic virus), which can confer expression in roots and which can play a more complex role in promoter regulation through synergistic interactions with other cis-activating elements, and which can optionally be salicylic acid-inducible;
- the term "as2" or "activating sequence 2" box means a regulatory or functional motif or element preferably originating from the 35S promoter of the CaMV (cauliflower mosaic virus), which can confer expression in the photosynthetic tissues, and which can have transcriptional activator activity;
- the term "cereal" box means means a regulatory or functional motif or element which may be involved in specific expression in wheat seeds;
- the term "GC-rich" box means a regulatory or functional motif or element which is rich in G or C nucleotides, for example originating from a geminivirus;
- the term "transgenic plant" means a plant which has been obtained by genetic manipulation techniques, and covers whole plants obtained by such manipulations, regenerated plants which integrate into their genome, or which express, such manipulations, their progeny, and the plant organs, for example roots, stalks and leaves, obtained by these techniques. The transgenic plants according to the present invention can have various levels of ploidy, and can in particular be polyploid, diploid or haploid;
- the term "propagule" means a mass or group of plant cells which is structured or unstructured, and which enables the regeneration of a whole plant, for example explants, calluses, stalks, leaves, roots, cuttings and even seeds.

The Applicant of the present invention has taken an approach which is different from that of the Applicant of the PCT application previously discussed. Specifically, the Applicant of the present invention has succeeded, surprisingly, in producing chimeric promoters which make it possible to satisfy the need described above, and in particular which make it possible to increase the level of expression of a gene or of a nucleic acid sequence encoding a polypeptide to be produced, in a host cell, and in particular a plant cell, with respect to the existing promoters most commonly used. Moreover, the Applicant has succeeded, at the same time, in producing a complete range of promoters so as to be able to choose the one which is suitable for use according to the use envisaged and the environment of its implementation, and thus to be able to control in some way the level of expression of a gene to be expressed which encodes a polypeptide to be produced. One example of use of this principle would be to use one of the weaker promoters of the invention to direct and/or control the expression of a protein or enzyme, for example an agent for selection in a plant, for example, resistance to antibiotics or to herbicides, or a coenzyme or cofactor required for assembling a more important protein, and to use a stronger promoter in accordance with the invention to, for example, control the expression of a polypeptide having a therapeutic effect.

Yet another advantage of the present invention is that the promoters prepared in accordance with the invention allow both a specific expression in the seeds, but also a deregulation in order to favour expression in other organs, for example, leaves, stalks and the plant vascular system.

Consequently, a main subject of the present invention is a chimeric promoter comprising at least one nucleic acid sequence derived from a gene encoding a high molecular weight wheat glutenin, and preferably a nucleic acid sequence derived from the wheat Dx5 or Bx7 gene encoding a high molecular weight wheat glutenin.

Preferably, the chimeric promoter comprises at least one nucleic acid sequence derived from a gene encoding a high molecular weight wheat glutenin, the sequence of which is identified under the number SEQ.ID01.

More preferably, the nucleic acid sequence derived from a gene encoding a high molecular weight wheat glutenin corresponds to a sequence chosen from the group consisting of the sequences identified under the numbers SEQ. ID02, SEQ. ID03, SEQ. ID04, SEQ. ID05, SEQ. ID06, SEQ. ID07, SEQ. ID08, SEQ. ID09, SEQ. ID10, SEQ. ID11, SEQ. ID12, SEQ. ID13, SEQ. ID16, SEQ. ID17, SEQ. ID18, SEQ. ID19, SEQ. ID20, SEQ. ID21 and SEQ. ID22.

In addition, the Applicant has noted that it is possible to construct promoters, and in particular plant promoters, which have an advantageous promoter activity both in dicotyledonous plants and in monocotyledonous plants, by combining a certain number of regulatory or functional boxes, using a nucleic acid sequence derived from a gene encoding a high molecular weight wheat glutenin as defined above.

Thus, another subject of the present invention is a chimeric promoter of expression which comprises a nucleic acid sequence derived from a gene encoding a high molecular weight wheat glutenin, and which comprises, in the 3' position, a "TATA" box and a transcription start site (+1).

Preferably, the promoter also comprises at least one "enhancer" box functionally linked in the 5' position upstream of the "TATA" box and the transcription start site (+1).

More preferably, the promoter also comprises at least one "G-like" box functionally linked in the 5' position upstream of the "enhancer" box.

Even more preferably, the promoter also comprises at least one "P-like" box functionally linked in the 5' position upstream of the "enhancer" box.

Advantageously, the promoter also comprises at least one "GATA" box functionally linked in the 5' position upstream of at least the "enhancer" box.

Preferably, the promoter also comprises at least one cereal box functionally linked in the 5' position upstream of the "enhancer" box.

Even more preferably, the promoter comprises two contiguous "cereal" boxes functionally linked in the 5' position upstream of the "enhancer" box.

According to one preferred embodiment of the promoters according to the present invention, they also comprise at least one "as1" box or at least one "as2" box, or an "as1/as2" or "as2/as1" combination of boxes, or repeat permutations of these, functionally linked in the 5' position upstream of the transcription start site.

According to another preferred embodiment of the invention, the "as1", "as2", "as1/as2" or "as2/as1" box(es), or its(their) repeat permutations, is(are) functionally linked downstream, in the 3' position, of the enhancer box.

According to yet another preferred embodiment of the invention, the promoters also comprise a "GC" box functionally linked upstream of the "enhancer" box.

Particularly preferably, the promoter comprises two "cereal" boxes functionally linked in the 5' position upstream of the "enhancer [lacuna] box, which is itself functionally linked in the 5' position upstream of an "as2/as1" box.

According to yet another preferred embodiment of the promoters of the invention, they comprise at least one element chosen from the group consisting of an "enhancer" box, a "G-like" box, a "P-like" box, a "GATA" box, a "cereal" box, an "as1" box, an "as2" box and/or an as1/as2 or "as2/as1" combination of boxes, optionally repeated, and a "GC-rich" box, functionally linked ir reverse orientation and/or downstream, in the 3' position, of the transcription start site.

Finally, the chimeric promoters according to the present invention advantageously comprise at least one nucleic acid sequence chosen from the group consisting of SEQ.ID02, SEQ.ID03, SEQ.ID04, SEQ.ID05, SEQ.ID06, SEQ.ID07, SEQ.ID08, SEQ.ID09, SEQ.ID10, SEQ.ID11, SEQ.ID12, SEQ.ID13, SEQ.ID16, SEQ.ID17, SEQ.ID18, SEQ.ID19, SEQ.ID20, SEQ.ID21 and SEQ.ID22.

Yet another subject of the present invention is an expression cassette comprising at least one promoter nucleic acid sequence which is derived from a gene encoding a high molecular weight wheat glutenin, and which is linked in a functional manner to a nucleic acid sequence to be expressed encoding a polypeptide to be produced, itself linked to a transcription termination nucleic acid sequence.

Preferably, this expression cassette comprises at least one promoter nucleic acid sequence, derived from a gene encoding a high molecular weight wheat glutenin, the sequence of which is identified under the number SEQ.ID01.

Even more preferably, the expression cassette comprises at least one promoter nucleic acid sequence which is derived from a gene encoding a high molecular weight wheat glutenin, and which is chosen from the group consisting of the sequences identified under the numbers SEQ.ID02, SEQ.ID03, SEQ.ID04, SEQ.ID05, SEQ.ID06, SEQ.ID07, SEQ.ID08, SEQ.ID09, SEQ.ID10, SEQ.ID11, SEQ.ID12, SEQ.ID13, SEQ.ID16, SEQ.ID17, SEQ.ID18, SEQ.ID19, SEQ.ID20, SEQ.ID21 and SEQ.ID22.

Another subject of the present invention is an isolated promoter nucleic acid sequence, characterized in that it corresponds to a sequence derived from the sequence identified under the number SEQ.ID01.

Preferably, the isolated promoter nucleic acid sequence corresponds to a sequence chosen from the group consisting of the sequences identified under the numbers SEQ.ID02,SEQ.ID03, SEQ.ID04, SEQ.ID05, SEQ.ID06, SEQ.ID07, SEQ.ID08, SEQ.ID09, SEQ.ID10, SEQ.ID11, SEQ.ID12, SEQ.ID13, SEQ.ID16, SEQ.ID17, SEQ.ID18, SEQ.ID19, SEQ.ID20, SEQ.ID21 and SEQ.ID22.

Another subject of the present invention is a vector comprising a promoter, or a promoter nucleic acid sequence, which is capable of initiating the transcription of a nucleic acid sequence encoding a polypeptide to be produced, characterized in that the promoter or the promoter nucleic acid sequence corresponds to a chimeric promoter or to a promoter nucleic acid sequence as defined above.

Preferably, the vector is chosen from the group consisting of the binary vectors identified under the numbers pMRT1207, pMRT1177, pMRT1178, pMRT1179, pMRT1180 and pMRT1181.

Yet another subject of the present invention is a transgenic plant which has stably integrated into its genome at least one promoter or at least one promoter nucleic acid sequence as defined above.

Preferably, the transgenic plant is chosen from dicotyledonous species, such as potato, tobacco, cotton, lettuce, tomato, melon, cucumber, pea, rapeseed, beetroot or sunflower, or monocotyledonous species, such as wheat, barley, oat, rice or maize.

Another subject of the present invention is a propagule of a transgenic plant as defined above, and preferably, this propagule is a seed.

Yet another subject of the present invention is a cell containing a promoter or a promoter nucleic acid sequence as defined above, and preferably, this cell is a plant cell.

Among the preferred subjects of the invention, there is also a method for expressing a nucleic acid sequence, or gene, encoding a polypeptide to be produced, in a cell, characterized in that it comprises the steps consisting in:
- transforming the cell with a vector comprising at least one promoter or at least one promoter nucleic acid sequence as defined above;
- preparing a culture of the cell under conditions which allow the expression of the nucleic acid sequence, or gene, encoding the polypeptide.

Preferably, the polypeptide to be produced is an enzyme or protein, or derivative of the latter, which has activity in vitro and/or in humans and/or in animals, said activity comprising digestive, pancreatic, biliary, antiviral, antiinflammatory, pulmonary, antimicrobial, nutritive, cosmetic and structural activity, activity in the blood, cardiovascular, ophthalmic, antigenic and immunostimulating activity, and activity in the brain. Examples of such proteins are, for example, insulins, interferons, gastric, pancreatic or biliary lipases, elastases, antiproteases such as alpha-1 antitrypsin, structure-forming proteins such as collagen, transferrins such as lactoferrin, blood-derived proteins, such as human haemoglobin or albumin, and blood cofactors, and antioxidants such as superoxide dismutase.

Preferably, the cell used in this method is a procaryotic or eucaryotic cell.

Even more preferably, the cell is a cell chosen from the group consisting of microbial cells, fungal cells, insect cells, animal cells and plant cells, and even more preferably it is a plant cell.

Finally, another subject of the present invention is a method for obtaining a transgenic plant or a propagule as defined above, characterized in that it comprises the steps consisting in:
- transforming a plant cell with a vector comprising at least one promoter or at least one promoter nucleic acid sequence as defined above;
- selecting the plant cell which has integrated the promoter or promoter nucleic acid sequence;
- propagating the transformed and selected plant cell either in culture or by regenerating chimeric or transgenic whole plants.

### DESCRIPTION OF THE FIGURES

The invention will be more clearly understood through the detailed description of the various embodiments given hereafter by way of non-limiting examples, and with reference to the attached drawings, in which:

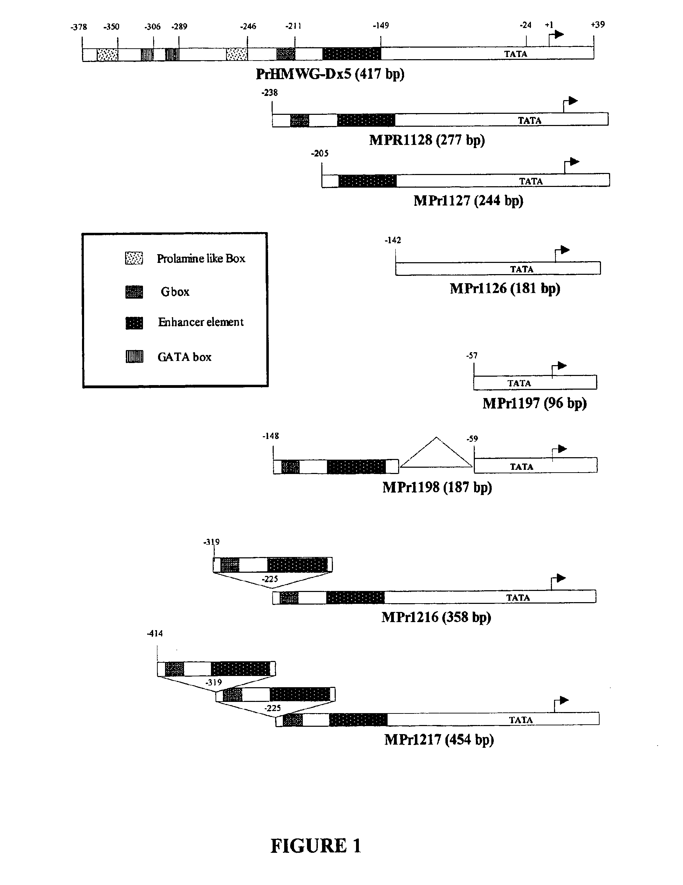

- Figures 1, 2, 3 and 4 represent, schematically, synthetic and chimeric promoter constructs in accordance with the invention. In Figure 1, they are constructs comprising a series of deletions starting from the whole promoter originating from the Dx5 gene which is from wheat and which encodes a high molecular weight wheat glutenin, and a series of constructs comprising repeats of elements which comprise an "enhancer" box combined with a "G-like" box;

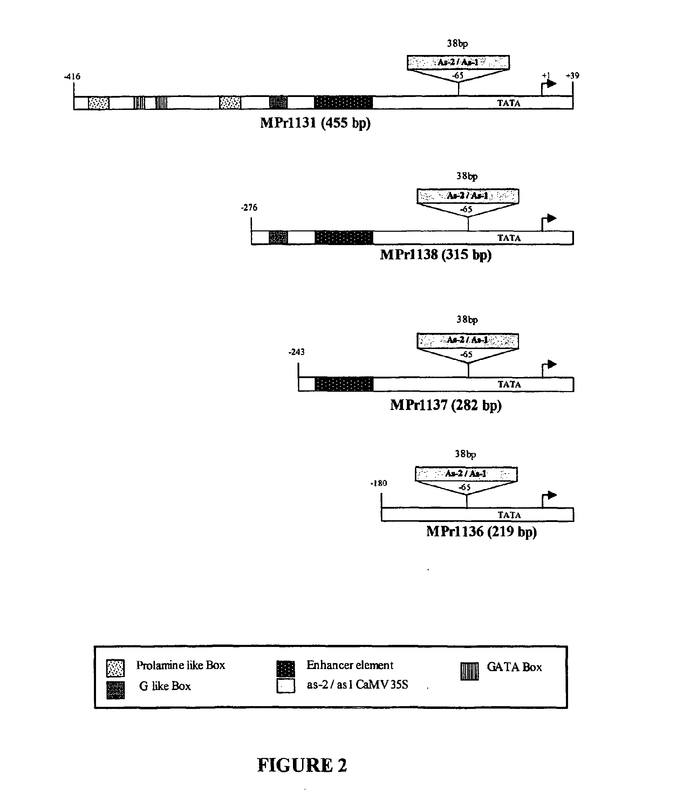

- Figure 2 represents, schematically, constructs of other synthetic and chimeric promoters in accordance with the invention, containing insertions of regulatory and/or functional elements which comprise combined "as2/as1" boxes;

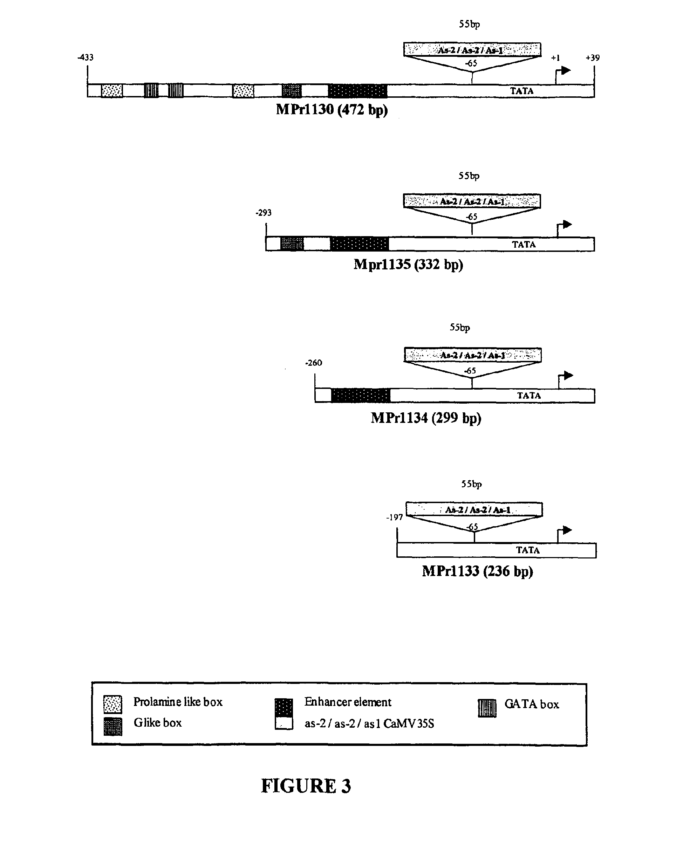

- Figure 3 represents, schematically, constructs of other synthetic and chimeric promoters in accordance with the invention, containing insertions of regulatory and/or functional elements which comprise combined "as2/as2/as1" boxes;

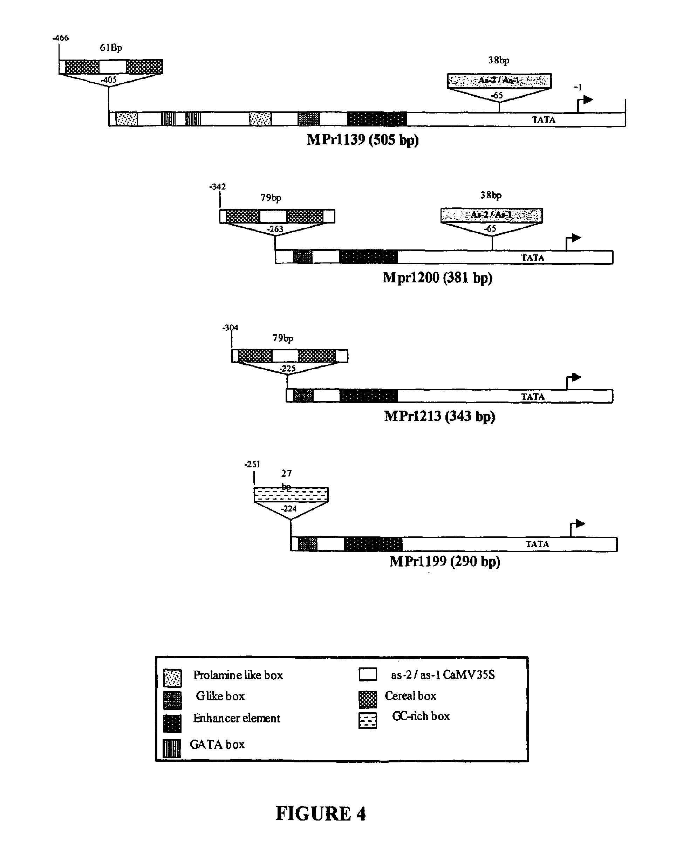

- Figure 4 represents, schematically, constructs of other synthetic and chimeric promoters in accordance with the invention, containing insertions of regulatory and/or functional elements which comprise combined "cereal" boxes, originating from the Bx7 gene encoding a high molecular weight wheat glutenin, alone or in combination with "as2/asl" boxes, and a construction comprising a "GC-rich" box;

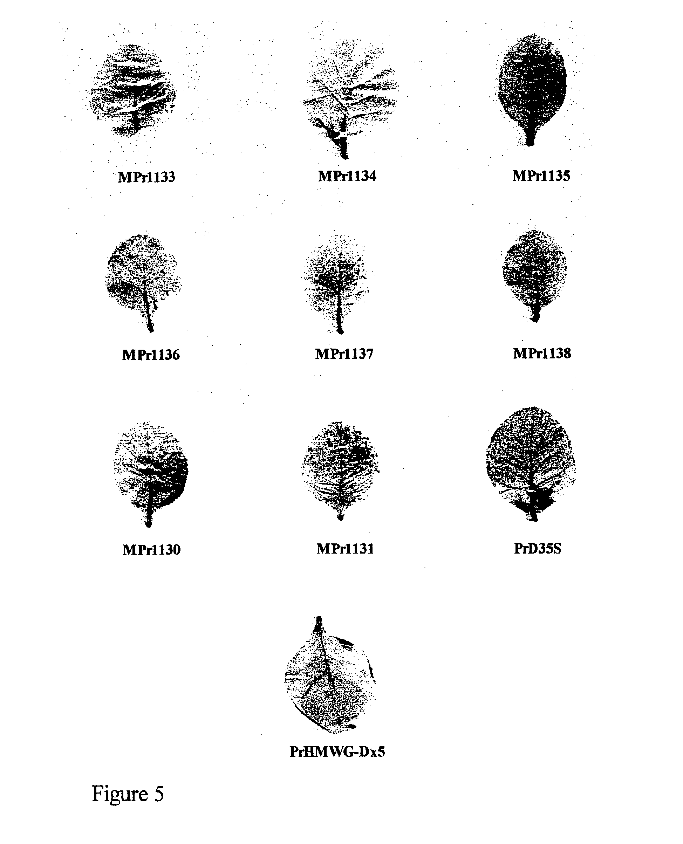

- Figure 5 represents photographs of tobacco leaves which have been transformed with vectors containing the promoters or [lacuna] nucleic acid sequences described above functionally linked to the gene encoding GUS (beta-glucuronidase), and after histochemical revelation, the blue dots indicating the presence of cells transformed with said constructs, and thus the activity of the promoter;

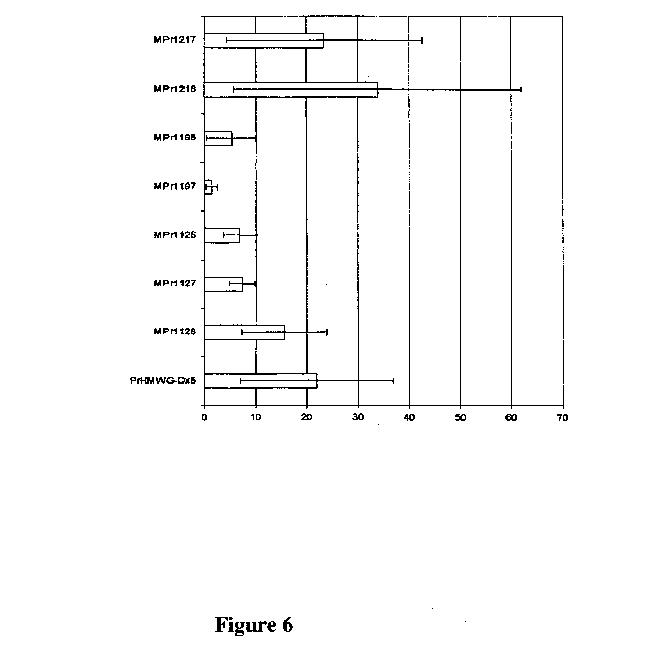

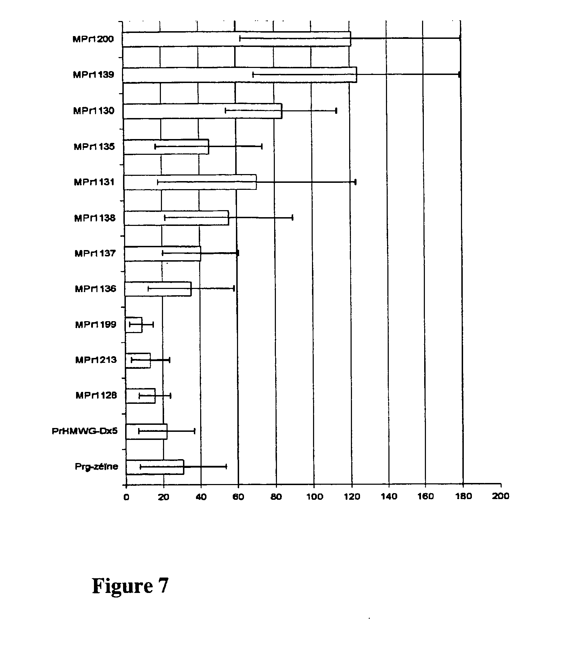

- Figures 6 and 7 represent graphs comparing the promoter activity relating to the various constructs after transient expression in maize albumen. Three days after bombarding, the albumen are ground, and then the crude extract is clarified by centrifugation. The β-glucuronidase activity and the luciferase activity are measured by luminometry on an aliquot of the crude extract, and then the GUS activity/LUC activity ratio is determined. The histograms correspond to the mean of ratios for the same construct +/- Standard Error of the Mean (SEM).

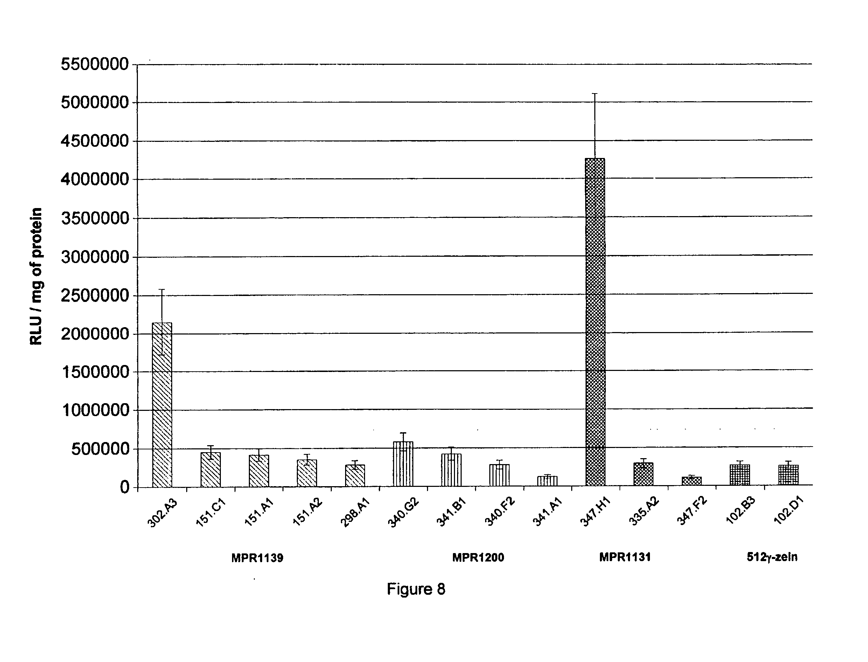

- Figure 8 represents a graph comparing the promoter activity of MPr1139, MPr1200 and MPr1131 to that of the 512 gamma-zein promoter in stable expression in maize albumen, 30 days after pollinisation (30 DAP). The β-glucuronidase activity and the total quantity of proteins were determined respectively by luminometry and spectrometry. The histograms correspond to the average ratios of GUS activity / total proteins measured seed by seed for each plant, +/- standard mean error. The name of each transformant is indicated.

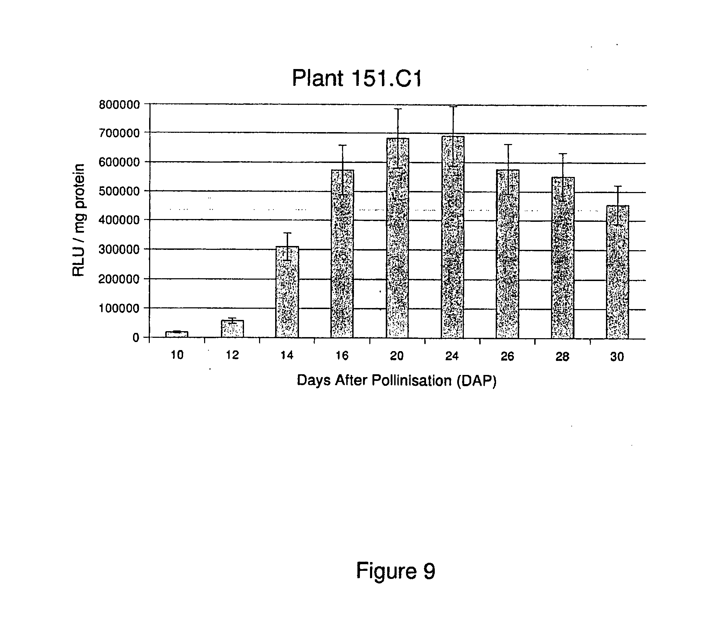

- Figure 9 represents the temporal β-glucuronidase activity controlled by the promoter MPr1139 in stable expression in maize albumen. The β-glucuronidase activity and the quantity of total proteins were determined by luminometry and spectrometry. The histograms correspond to the average ratiosn of GUS activity / total proteins measured seed per seed for the plant 151.C1 at different stages of development, +/- standard mean error.

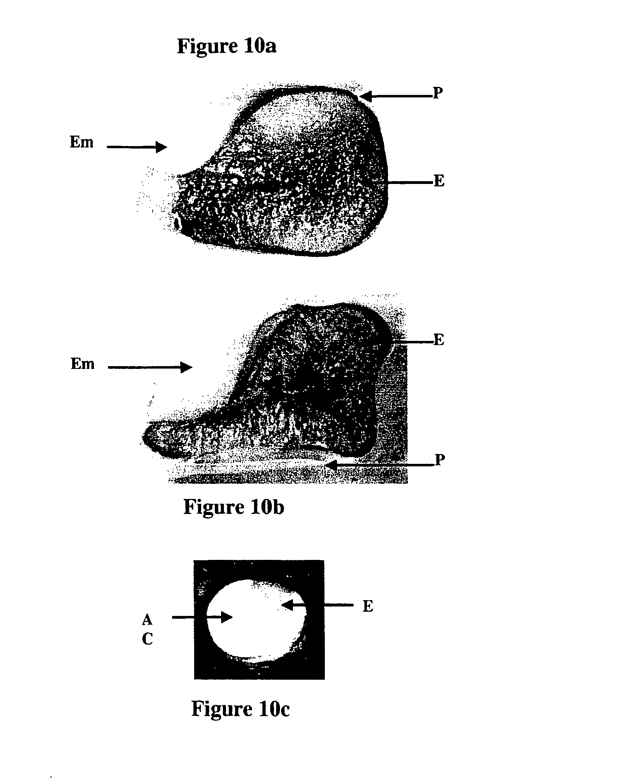

- Figure 10a represents a longitudinal section of a maize seed at 13 DAP, figure 10b represents a longitudinal section of a maize seed at 20 DAP, and figure 10c is a top plan view of a dissected maize seed. All reveal the β-glucuronidase activity under the control of the promoter MPr1139 in stable expression in maize seeds, visualized by histochemical staining (blue color), where the letters indicate the following : E (embryo) ; Em (endosperm) ; AC (aleurone cells) ; P (pericarp)

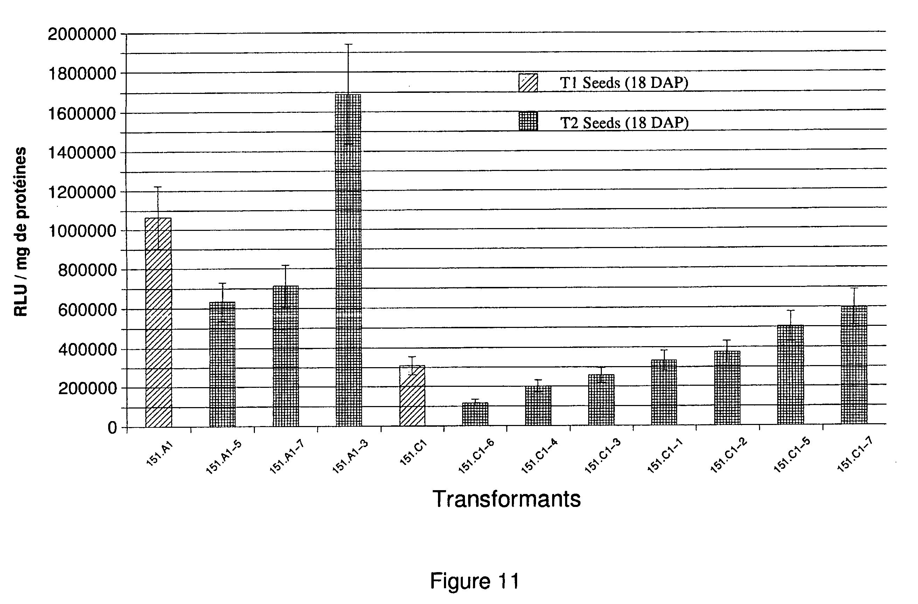

- Figure 11 represents a graph comparing the promoter activity of MPr1139 in first generation maize seeds (T1) to that of second generation transgenic maize seeds (T2), 18 days after pollinisation (18 DAP). The β-glucuronidase activity and the quantity of total proteins were measured by luminometry and spectrometry respectively. The histograms correspond to the average ratios of GUS activity / total proteins measured seed per seed for each plant, +/- standard mean error. The name of each transformant is indicated.

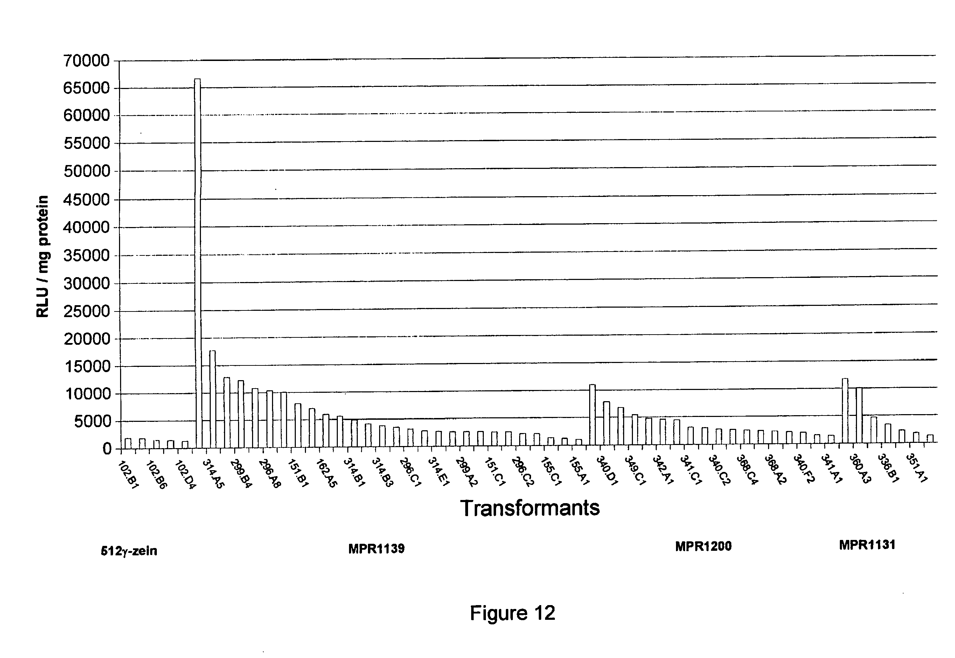

- Figure 12 represents a graph comparing the promoter activity of MPr1139, MPr1200 and MPr1131 to that of the 512 gamma-zein promoter in stable expression of maize leaves, 3 weeks after acclimatization in a greenhouse. The β-glucuronidase activity and the quantity of total proteins were measured by luminometry and spectrometry respectively. The histograms correspond to the ratios of GUS activity / total proteins measured in the leaves of different maize leaves. The name of each transformant is indicated.

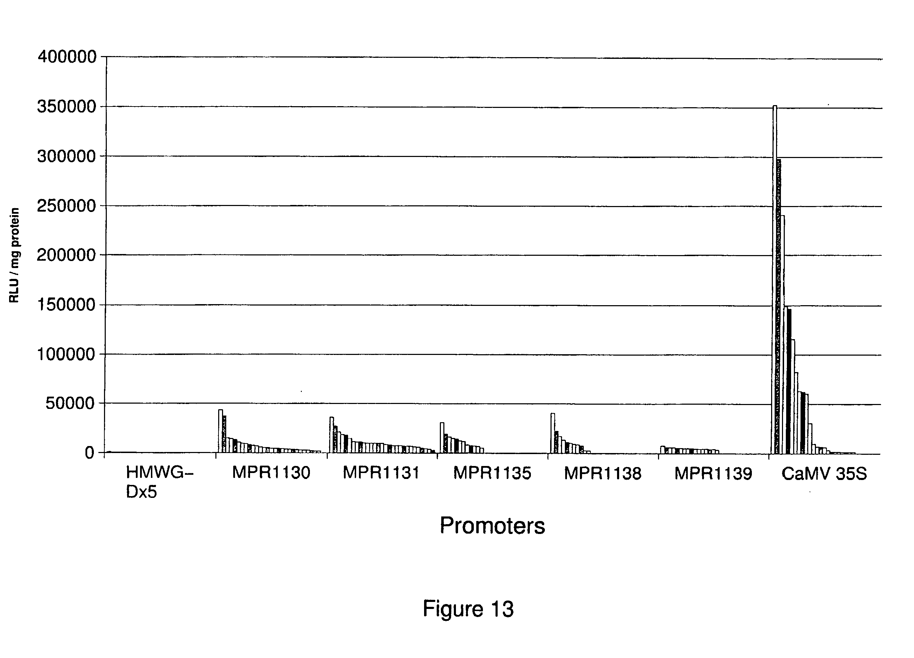

- Figure 13 represents a graph comparing the promoter activity of MPr1130 MPr1131, MPr1135, MPr1138 and MPr1139 to that of the reference promoters CaMV D35S and HMWG-Dx5 in stable expression in tobacco leaves, at the 11 week stage of development after acclimatization in a greenhouse. The β-glucuronidase activity and the quantity of total proteins were measured by luminometry and spectrometry respectively. The histograms correspond to the ratios of GUS activity / total proteins measured in the leaves of different tobacco plants.

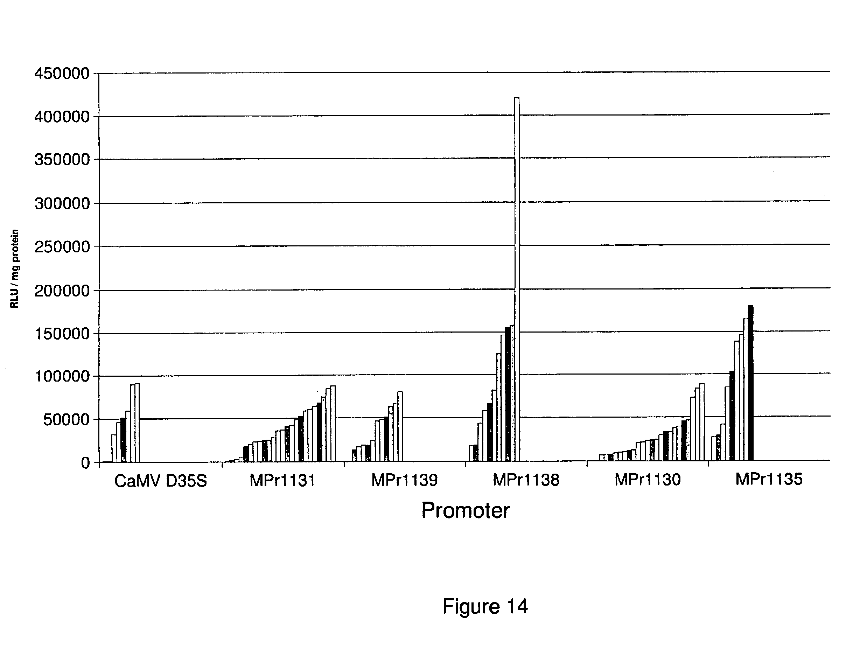

- Figure 14 represents a graph comparing the promoter activity promotrice of MPr1130, MPr1131, MPr1135, MPr1138 and MPr1139 to that of the reference promoter CaMV D35S, in stable expression in mature first generation tobacco seeds. The β-glucuronidase activity and the quantity of total proteins were measured by luminometry and spectrometry respectively. The histograms correspond to the ratios of GUS activity / total proteins measured in the seeds of different tobacco plants.

In the detailed description which follows, the enzymatic treatments performed with the restriction enzymes and the DNA modification enzymes were carried out according to the recommendations of the supplier New England Biolabs. Following each enzymatic treatment, the DNA was systematically purified with the aid of the "QIAquick PCR Purification" (QIAGEN) or "Concert Rapid PCR Purification System" (GIBCO BRL Life Technologies), or, if specified, with the aid of the "QIAquick Gel Extraction" (QIAGEN) or "Concert Rapid Gel Extraction System" (GIBCO BRL Life Technologies) kits according to the manufacturer's instructions. The "GeneAmp PCR System 9700" thermocycler used is sold by Perkin Elmer Applied Biosystems.

### EXAMPLES

### Example 1

### Constructs for comparative purpose (controls).

In order to enable the comparison of the chimeric promoters described in this patent, the *uidA* gene encoding b-glucuronidase (Jefferson et al., 1986) containing the sequence of intron IV2 of the potato patatin gene *ST-LSI* (Vancanneyt et al., 1990) (*uidA*-IV2) was placed under the control of one of the reference promoters and of the nopaline synthase gene terminator (term-nos) of *Agrobacterium tumefaciens,* in the plasmid pGEM3Z sold by Promega Corp. (Madison, USA).

### 1.1. Construction of the negative control pMRT1144.

The plasmid pMRT1144, devoid of any promoter sequence, is used as a negative control. It is derived from the plasmid pGEM3Z into which the sequence "uidA-IV2/term-nos" has been introduced.

Firstly, 5 µg of the plasmid pB1221 (Clonetech, CA, USA) were digested for 1 h at 37°C with the restriction enzymes EcoRI and BamHI. The *uidA* sequence under the control of the nopaline synthase terminator was then isolated on a 0.8% agarose gel with the aid of the "QIAquick Gel Extraction" kit.

In parallel, 5 µg of plasmid pGEM3Z were digested with the restriction enzyme pair EcoRI and BamHI for 1 h at 37°C. The vector fragment was then isolated on a 0.8% agarose gel with the aid of the "QIAquick Gel Extraction" kit, and dephosphorylated with 40 U of calf intestine alkaline phosphatase (New England Biolabs) in the presence of buffer 3 (1X) at 37°C for 1 h.

The ligation reaction was carried out with 50 ng of the "*uidA*-IV2/term-*nos*" fragment and 100 ng of plasmid pGem3Z, thus treated, in a 10 µl reaction mixture, in the presence of T4 DNA ligase buffer (1X) and of 400 units of T4 DNA ligase (New England Biolabs) in the "GeneAmp PCR System 9700" thermocycler. It consists of one cycle at 10°C for 30 sec. and of 200 identical cycles each consisting of the following steps: 30 sec. at 30°C, 30 sec. at 10°C and 30 sec. at 30°C. *Escherichia coli* DH5a bacteria, which had been made competent beforehand, were transformed with all of the ligation reaction mixture. The plasmid DNA of the clones obtained, which were selected on Luria-Bertani medium (LB, 10 g/l bactotryptone, 5 g/l yeast extract, 10 g/l NaCl, pH 7.2 and 15 g/l Agar-Agar) supplemented with ampicillin (50 mg/l), was extracted according to the alkaline lysis method (Birnboim and Doly, 1983) and analysed with enzymatic digestions. The resulting plasmid was called pGEM3Z/uidA/term-*nos*.

Secondly, in order to insert the 192-bp intron IV2 of the potato patatin gene into the *uidA* coding sequence of pGEM3Z/uidA/term-nos, an internal portion of this gene (710-bp SnaBI/BstBI fragment) was excised and then replaced with the equivalent sequence containing intron IV2 (902-bp SnaBI/BstBI fragment).

In order to do this, 10 µg of the plasmid *pGEM3Z*/*uidA*/*term-nos* were digested for 1 h at 37°C with SnaBI (restriction site located at position +383 bp downstream of the ATG start codon of the *uidA* gene), and then for 1 h at 65°C with BstBI (site located at position +1093 bp). The plasmid thus deleted of the 710-bp fragment was isolated on a 0.8% agarose gel with the aid of the "QIAquick Gel Extraction" kit, and dephosphorylated with 40 U of calf intestine alkaline phosphatase (New England Biolabs) in the presence of buffer 3 (1X) at 37°C for 1 h.

The 902-bp BstBI/SnaBI fragment corresponding to the sequence of intron IV2 followed by the *uidA* sequence was obtained by digesting 10 µg of the plasmid p35S GUS INT (Vancanneyt et al., 1990) with the restriction enzyme SnaBI (restriction site located at position +383 bp downstream of the ATG start codon of the *uidA* gene) for 1 h at 37°C, and restriction enzyme BstBI (site located at position +1285 bp) for 1 h at 37°C. The 902-bp fragment was then isolated on a 1% agarose gel with the aid of the "Concert Rapid Gel Extraction System" kit.

The ligation reaction was carried out with 100 ng of vector pGEM3Z/uidA/term-nos and 50 ng of the 902-bp BstBI/SnaBI fragment thus treated, in a 10 µl reaction mixture, in the presence of the T4 DNA ligase buffer (1X) and of 400 units of T4 DNA ligase (New England Biolabs) in the "GeneAmp PCR System 9700" thermocycler as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was extracted according to the alkaline lysis method and analysed with enzymatic digestions. The plasmid obtained was called pMRT1144.

### 1.2. Construction of the positive control pMRT1218.

In order to have a reference sequence which is a promoter in the maize albumen SN 87 165 (L2), the 1.7-kb whole g-zein promoter (Prg-zein) contained in the plasmid p63 described by Reina et al. (1990) was placed upstream of the sequence *uidA-*IV2/term-*nos*.

The 1.7-kb g-zein promoter was obtained by digesting 15 µg of plasmid p63 with the restriction enzymes HindIII and BamHI for 1 h at 37°C. The 1.7-kb Prg-zein fragment thus released was isolated on a 0.8% agarose gel with the aid of the "Concert Rapid Gel Extraction System" kit.

In parallel, 10 µg of plasmid pMRT1126 (described in section 3.4 of Example 3) were also digested with the restriction enzymes HindIII and BamHI for 1 h at 37°C. The vector fragment was then isolated on a 0.8% agarose gel with the aid of the "Concert Rapid Gel Extraction System" kit, and dephosphorylated with 40 U of calf intestine alkaline phosphatase (New England Biolabs) in the presence of buffer 3 (1X) at 37°C for 1 h.

The ligation reaction was carried out with 50 ng of the g-zein promoter fragment and 100 ng of plasmid pMRT1126, thus treated, in a 10 µl reaction mixture, in the presence of the T4 DNA ligase buffer (1X) and of 400 units of T4 DNA ligase (New England Biolabs), in the "GeneAmp PCR System 9700" thermocycler as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/1), was extracted according to the alkaline lysis method and analysed with enzymatic digestions. The resulting plasmid was called pMRT1218.

### 1.3. Construction of the positive control pMRT1092.

In order to have a reference sequence which is a promoter in the photosynthetic tissues of tobacco *(Nicotiana tabacum* L., cultivar PBD6), the double 35S promoter of the cauliflower mosaic virus (CaMV PrD35S) was placed upstream of the sequence *ui*dA-IV2/term-nos.

Firstly, the 192-bp intron IV2 of the potato patatin gene was inserted into the *uidA* coding sequence at position +383 bp as described in section 1.1. In order to do this, 1 µg of plasmid pBI221 (Clontech, CA, USA) was digested for 1 h 30 min. at 37°C with SnaBI, and then for 1 h 30 min. at 65°C with BstBI. The plasmid deleted of a 710-bp fragment was isolated on a 0.8% agarose gel, and then purified on a Qiaquick affinity column. A 20 ng amount of BstBI/SnaBI pBI221 vector and 80 ng of the 902-bp BstBI/SnaBI fragment originating from p35S GUS INT as described above were ligated overnight at 18°C in a 10 µl reaction mixture, in the presence of the T4 DNA ligase buffer (1X) and of 400 units of T4 DNA ligase enzyme (New England Biolabs). *Escherichia coli* DH5a bacteria, which had been made competent beforehand, were transformed with half of the ligation reaction mixture. The DNA of the clones obtained, which were selected on LB medium supplemented with ampicillin (50 mg/l), was extracted according to the alkaline lysis method and analysed with enzymatic digestions. The plasmid obtained was called pBI221/*uidA*-IV2.

Secondly, the sequence of the CaMV 35S promoter present in the plasmid pBI221/*uidA*-IV2 was replaced with the sequence of "CaMV PrD35S". The plasmid pBI221/*uidA*-IV2 was digested for 10 h 30 min. at 37°C with HindIII, and then the sticky ends were made blunt-ended using the Klenow fragment (New England Biolabs) for 30 min at 37°C. The product of this reaction was then digested overnight at 37°C with BamHI. The plasmid DNA fragment, corresponding to the vector deleted of the 828-bp CaMV 35S promoter fragment, was isolated on a 0.8% agarose gel, and then purified on a Qiaquick affinity column.

In parallel, the CaMV D35S promoter was obtained from the plasmid pJIT163D. This plasmid is derived from the plasmid pJIT163, which is itself derived from the plasmid pJIT160 (Guérineau and Mullineaux, 1993). The plasmid pJIT163 has an ATG codon between the HindIII and SalI sites of the polylinker. In order to delete this ATG and to obtain the plasmid pJIT163D, the pJIT163 plasmid DNA was digested with HindIII and SalI, purified on a 0.8% agarose gel, electroeluted, precipitated in the presence of a 1/10 volume of 3M sodium acetate, pH 4.8, and of 2.5 volumes of absolute ethanol at -80°C for 30 min, centrifuged at 12,000 g for 30 min, washed with 70% ethanol, dried, subjected to the action of the Klenow fragment (New England Biolabs) for 30 min at 37°C, deproteinized by extraction with one volume of phenol, then one volume of phenol/chloroform/isoamyl alcohol (25/24/1 v/v/v) and finally one volume of chloroform/isoamyl alcohol (24/1 v/v), precipitated in the presence of a 1/10 volume of 3M sodium acetate, pH 4.8, and of 2.5 volumes of absolute ethanol at -80°C for 30 min, then centrifuged at 12,000 g for 30 min, washed with 70% ethanol, dried and finally ligated in the presence of the T4 DNA ligase buffer (1X) and 2.5 units of T4 DNA ligase (Amersham) at 14°C for 16 h. *Escherichia coli* DH5a bacteria, which had been made competent beforehand, were transformed. The plasmid DNA of the clones obtained, which were selected on LB medium supplemented with ampicillin (50 mg/l), was extracted according to the alkaline lysis method and analysed with enzymatic digestions. Next, ten µg of plasmid pJIT163D were digested for 10h30 at 37°C with KpnI (site located 5' of the promoter), and then the sticky ends were made blunt-ended using 6 units of T4 DNA polymerase (New England Biolabs) for 30 min at 37°C. The product of this reaction was then digested overnight at 37°C with BamHI. The resulting 761-bp DNA fragment, corresponding to the CaMV D35S promoter, was isolated on a 1% agarose gel, and then purified on a Qiaquick affinity column. The ligation was carried out with 10 ng of plasmid vector and 100 ng of the 761-bp fragment, in a 10 µl reaction mixture, in the presence of the T4 DNA ligase buffer (1X) and 400 units of T4 DNA ligase (New England Biolabs) overnight at 18°C. *Escherichia coli* DH5a bacteria, which had been made competent beforehand, were transformed with half of the ligation reaction mixture. The plasmid DNA of the clones obtained, which were selected on LB medium supplemented with ampicillin (50 mg/l), was extracted according to the alkaline lysis method and analysed with enzymatic digestions. The plasmid obtained was called pMRT1092. **1.3. Description of the reference plasmid pCaMV35Sluc.**

The plasmid used as an internal reference in the transient expression is pCaMV35Sluc (Torrent et al., 1997), which contains the cassette for expression of the luciferase (luc) reporter gene under the control of the CaMV 35S promoter and RNA terminator.

### Example 2.

### Construction of plasmids containing the whole promoter sequence and deleted or duplicated promoter sequences of a high molecular weight wheat glutenin gene.

The whole promoter (PrHMWG-Dx5 (SEQ.ID01)) of the high molecular weight glutenin gene encoding the Dx5 subunit, also called GluD1-1b of the hexaploid wheat *Triticum aestivum L. cv Cheyenne* (Anderson et al., 1989) corresponds to a 417-bp sequence (accession No. X12928) ranging from position -378 bp to position +39 bp, on which diverse potentially regulatory sequences are identified and listed on the 5' side towards the 3' side, with respect to the +1 transcription start point (Fig. I):
- a prolamine-"like" box, stretching from position -357 to position -350 bp,
- two GATA boxes, stretching from position -309 to position -306 bp, and position -292 to position -289 bp,
- a prolamine-"like" box, stretching from position -252 to position -246 bp,
- an 8-bp "G"-like box, stretching from position -218 to position -211 bp,
- a 38-bp activating element, stretching from position -186 to position -149 bp, composed of a mosaic of putative cis-activating motifs:
   - a prolamine box, stretching from position -182 to position -176 bp,
   - a sequence with imperfect symmetry, stretching from position -178 to position -161 bp,
   - a direct repeat of the pentanucleotide GCTCC between positions -176 and -163 bp,
   - an "E" box, stretching from position -172 to position -167 [lacuna],
   - a direct repeat of the pentanucleotide TTGCT between positions -169 and -158 bp,
- a "TATA" box, having the consensus TATAAAA from position
- 30 to -24 [lacuna],
- the +1 transcription start point (position 1),
- an untranslated 5' region ranging from position +1 to position +39 bp.

In order to study the effect of the various putative cis-activating elements described above, a detailed functional analysis of the HMWG-Dx5 promoter (SEQ.ID01) was carried out.

The *uidA*-IV2 reporter gene was placed under the control of the whole HMWG-Dx5 promoter (SEQ.ID01) and under the control of the synthetic HMWG-Dx5 (SEQ.ID01) promoters having either increasing deletions of the 5' regions, or an internal deletion, or duplications of an internal portion.

### 2.1. Construction of the plasmid pbMT1125.

The plasmid pMRT1125 is the result of cloning the whole promoter of the high molecular weight glutenin gene (PrHMWG-Dx5 (SEQ.ID01), Fig. I) upstream of the uidA-IV2 reporter gene, and constitutes the reference construct for all of the synthetic HMWG-Dx5 (SEQ.ID01) promoters described in this patent.

The HMWG-Dx5 promoter (SEQ.ID01) described by Anderson et al. (1989) was obtained from the expression cassette "PrHMWG-Dx5 (SEQ. ID01)/uidA/term-nos" introduced into a pUC19 plasmid (Stratagene) according to the usual cloning techniques. Ten µg of the resulting plasmid (pPUC19-HMWG) were hydrolysed with EcoRI for 1 h at 37°C, and subjected to the action of 20 U of the Klenow fragment (New England Biolabs) for 30 min at 37°C in the presence of 60 nmol of each of the dNTPs, of 12 µl of *500 mM Tris-HCL, pH 7.5*/*500 mM MgC12* buffer and 6 µl of 1 M dithiothreitol (DTT). The DNA was then digested with BamHI for 1 h at 37°C, and the HMWG-Dx5 promoter fragment (SEQ.ID01) thus released and treated was isolated on a 1.5% agarose gel with the aid of the "QIAquick Gel Extraction" kit.

In parallel, the vector fragment was prepared from the plasmid pMRT1097 (unpublished French patent application FR 9903635). Twenty µg of plasmid pMRT1097 were digested for 1 h at 37°C with SphI, and the sticky ends of the vector pMRT1097 thus linearized were made blunt-ended using 6 U of the T4 DNA polymerase enzyme (New England Biolabs) for 30 min. at 37°C. The product of this reaction was then hydrolysed with BamHI, and the vector fragment was isolated on a 0.8% agarose gel with the aid of the "QIAquick Gel Extraction" kit, before being dephosphorylated with 40 U of calf intestine alkaline phosphatase (New England Biolabs) in the presence of buffer 3 (1X) at 37°C for 1 h. The resulting cloning vector was called pGEM3Z-1.

The ligation was carried out with 100 ng of the HMWG-Dx5 promoter fragment (SEQ.ID01) thus treated and 50 ng of plasmid pGem3Z-1 overnight at 16°C in a 10 µl reaction mixture, in the presence of the T4 DNA ligase buffer (1X) and of 400 units of T4 DNA ligase (New England Biolabs). *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was extracted according to the alkaline lysis method and analysed with enzymatic digestions.

The plasmid obtained was called pMRT1125, and the HMWG-Dx5 promoter (SEQ.ID01) (shown diagrammatically in Fig. 1 was verified by sequencing.

### 2.2. Construction of the MPr1128 promoter.

The MPR1128 promoter (SEQ.ID04) is derived from PrHMWG-Dx5 (SEQ.ID01) by deleting the sequence located upstream of nucleotide -238, this sequence comprising the two prolamine-"like" boxes and the two GATA boxes. The promoter fragment was amplified by PCR from 5 ng of pMRT1125 matrix DNA (described in section 2.1 of Example 2) with the aid of 100 pmol of each of the 2 oligodeoxynucleotides 5' ATCGGAATTCCAGAACTAGGATTACGCCG 3', containing the EcoRI restriction site, and 5' TACggATCCCCggggATCTCTAgTTTgTggTgC 3', possessing the BamHI restriction site, in the presence of 50 nmol of each of the dNTPs, of the Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 5 min., the DNA was subjected to 30 cycles each consisting of the steps of denaturation at 94°C for 1 min., of hybridization at 50°C for 1 min. and elongation at 72°C for 1 min. 30 sec. During the final cycle, the elongation was continued at 72°C for 5 min.

The DNA fragment derived from the amplification was isolated on a 1.5% agarose gel with the aid of the "QIAquick Gel Extraction" kit, hydrolysed with EcoRI for 1 h at 37°C and subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 µl of 1 M DTT. The DNA thus treated was then digested with BamHI for 1 h at 37°C.

The ligation was carried out with 100 ng of the MPR1128 promoter fragment (SEQ.ID04) thus treated and 50 ng of plasmid pGEM3Z-1 (described in section 2.1 of Example 2) overnight at 16°C in a 10 µl reaction mixture, in the presence of the T4 DNA ligase buffer (1X) and of 400 units of T4 DNA ligase (New England Biolabs). *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was extracted according to the alkaline lysis method and analysed with enzymatic digestions.

The plasmid obtained was called pMRT1128, and the MPR1128 promoter sequence (SEQ.ID04) represented diagrammatically in Fig. I was verified by sequencing.

### 2.3. Construction of the MPr1127 promoter (SEQ.ID03).

The MPr1127 promoter (SEQ.ID03) is derived from the HMWG-Dx5 promoter (SEQ.ID01) by deleting the sequence located upstream of nucleotide -205, this sequence comprising the two prolamine-"like" boxes, the two GATA boxes and the "G" box. The promoter fragment was amplified by PCR and treated in the same way as the MPR1128 promoter (SEQ.ID04) (described in section 2.2 of Example 2), except that the 2 oligodeoxynucleotides used are 5' ATCGGGAATTCGCAGACTGTCCAAAAATC 3', containing the EcoRI restriction site, and 5' TACggATCCCCggggATCTCTAgTTTgTggTgC 3', possessing the BamHI restriction site.

The plasmid obtained was called pMRT1127, and the MPr1127 promoter sequence (SEQ.ID03) represented diagrammatically in Fig. I was verified by sequencing.

### 2.4. Construction of the MPr1126 promoter (SEQ.ID02).

The MPr1126 promoter (SEQ.ID02) is derived from the HMWG-Dx5 promoter (SEQ.ID01) by deleting the sequence located upstream of nucleotide -142, this sequence comprising the two prolamine-"like" boxes, the two GATA boxes, the "G" box and the activating element. The promoter fragment was amplified by PCR and treated in the same way as the MPR1128 promoter (SEQ.ID04)(described in section 2.2 of Example 2), except that the 2 oligodeoxynucleotides used are 5' ATCGGAATTCGTGTTGGCAAACTGC 3', containing the EcoRI restriction site, and 5' TACggATCCCCggggATCTCTAgTTTgTggTgC 3', possessing the BamHI restriction site. The plasmid obtained was called pMRT1126, and the MPr1126 promoter sequence (SEQ.ID02) represented diagrammatically in Fig. I was verified by sequencing.

### 2.5. Construction of the MPr1183 intermediate promoter.

The MPr1183 promoter results from the insertion of an XbaI restriction site upstream of the MPR1128 promoter (SEQ.ID04) (described in section 2.2 of Example 2). The promoter fragment was amplified by PCR from 5 ng of pMRT1128 matrix DNA with the aid of 100 pmol of each of the 2 oligodeoxynucleotides 5' ATCggAATTCTAgACgCCgATTACgTggCTTTAgC 3', containing the EcoRI and XbaI restriction sites, and 5' TACggATCCCCggggATCTCTAgTTTgTggTgC 3', possessing the BamHI restriction site, in the presence of 50 nmol of each of the dNTPs, of Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 5 min., the DNA was subjected to 30 cycles each consisting of the steps of denaturation at 94°C for 1 min., of hybridization at 50°C for 1 min. and of elongation at 72°C for 1 min. 30 sec. During the final cycle, the elongation was continued at 72°C for 5 min.

The DNA fragment derived from the amplification was isolated on a 1.5% agarose gel with the aid of the "Concert Rapid Gel Extraction System" kit, hydrolysed with EcoRI for 1 h at 37°C and then subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgC12 buffer and 6 µl of 1 M DTT, and digested with BamHI for 1 h at 37°C.

The ligation was carried out with 100 ng of the MPR1128 promoter fragment thus treated and 50 ng of plasmid pGem3Z-1 (described in section 2.1 of Example 2) overnight at 16°C in a 10 µl reaction mixture, in the presence of the T4 DNA ligase buffer (1X) and of 400 units of T4 DNA ligase (New England Biolabs). *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 25 pmol of each of the 2 oligodeoxynucleotides 5' ATCggAATTCgCAgCCATggTCCTgAACC 3' and 5' TACggATCCCCggggATCTCTAgTTTgTggTgC 3', in the presence of 15 nmol of each of the dNTPs, of the Taq DNA polymerase buffer (1X), of 75 nmol of MgCl2 and of 1.25 U of Taq DNA polymerase (Promega Corp.) in a 50 µl reaction volume. The amplification reaction was carried out in the "GeneAmp PCR System 9700" thermocycler. After a denaturation at 94°C for 3 min., the DNA was subjected to 25 cycles each consisting of the steps of denaturation at 94°C for 30 sec., of hybridization at 55°C for 1 min. and of elongation at 72°C for 1 min. During the final cycle, the elongation was continued at 72°C for 5 min.

The plasmid obtained was called pMRT1183, and the MPr1183 promoter sequence was verified by sequencing.

### 2.6. Construction of the MPr1197 promoter (SEQ. ID 12)

The MPr1197 promoter (SEQ.I D2) is derived from MPr1183 (described in section 2.5 of Example 2) by a deletion of the promoter sequence located upstream of nucleotide -57 bp, and constitutes the minimum HMWG-Dx5 (SEQ.ID01) promoter studied in this patent.

In order to do this, 5 µg of plasmid pMRT1183 were digested successively for 1 h at 37°C with XbaI and NcoI. The vector pMRT1183 thus deleted of the XbaI/NcoI fragment of the MPr1183 promoter was isolated on a 0.8% agarose gel with the aid of the "Concert Rapid Gel Extraction System" kit, and subjected to the action of 20 U of the Klenow fragment (New England Biolabs) for 30 min at 37°C in the presence of 60 nmol of each of the dNTPs, of 12 µl of 550 mM Tris-HCL, pH 7.5/500 mM MgCl2 buffer and 6 µl of 1 M DTT.

The ligation reaction was carried out with 150 ng of plasmid thus modified, in a 10 µl reaction mixture, in the presence of the T4 DNA ligase buffer (1X) and of 400 units of T4 DNA ligase (New England Biolabs) in the "GeneAmp PCR System 9700" thermocycler, as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was extracted according to the alkaline lysis method and verified with enzymatic digestions.

The plasmid obtained was called pMRT1197, and the MPr1197 promoter sequence (SEQ.ID 12) is represented diagrammatically in Fig. I.

### 2.7. Construction of the MPr1198 promoter (SEQ ID 13)

The MPr1198 promoter (SEQ ID 13) is derived from the MPR1128 promoter (SEQ.ID04) (described in section 2.2 of Example 2) by deleting the internal promoter sequence stretching from position -59 to position -135 bp, this sequence lacking the *cis-*activating elements identified above.

It was constructed by fusing, at the NcoI restriction site of pMRT1183 (described in section 2.5 of Example 2), a fragment amplified by PCR from 5 ng of pMRT1128 matrix DNA with the aid of 100 pmol each of the 2 oligodeoxynucleotides 5' ATCggAATTCTAgACgCCgATTACgTggCTTTAgC 3', containing the EcoRI and XbaI restriction sites, and 5' CATgCCATggCCAACACAAAAg-AAgCTgg 3', possessing the NcoI restriction site, in the presence of 50 nmol of each of the dNTPs, of Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 10 min., the DNA was subjected to 25 cycles each consisting of the steps of denaturation at 94°C for 1 min., of hybridization at 55°C for 1 min. and of elongation at 72°C for 1 min. 30 sec. During the final cycle, the elongation was continued at 72°C for 5 min. The DNA fragment derived from the amplification was isolated on a 1.5% agarose gel with the aid of the "Concert Rapid Gel Extraction System" kit, and hydrolysed successively with Ncol and XbaI, for 1 h at 37°C.

In parallel, the vector fragment was prepared from the plasmid pMRT1183 by deleting the MPr1183 promoter region located 5' of the NcoI restriction site. In order to do this, 5 µg of plasmid pMRT1183 were digested successively for 1 h at 37°C with XbaI and NcoI, and the vector fragment of pMRT1183 was isolated on a 0.8% agarose gel with the aid of the "Concert Rapid Gel Extraction System" kit, before being dephosphorylated with 40 U of calf intestine alkaline phosphatase (New England Biolabs) in the presence of buffer 3 (1X) at 37°C for 1 h.

The ligation reaction was carried out with 50 ng of the promoter fragment and 100 ng of plasmid thus treated, in a 10 µl reaction mixture, in the presence of the T4 DNA ligase buffer (IX) and of 400 units of T4 DNA ligase (New England Biolabs), in the "GeneAmp PCR System 9700" thermocycler, as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was extracted according to the alkaline lysis method and analysed with enzymatic digestions.

The plasmid obtained was called pMRT1198, and the MPr1198 promoter sequence (SEQ.ID13) was represented diagrammatically in Fig. I was verified by sequencing.

### 2.8. Construction of the MPr1216 promoter (SEQ.ID17).

The MPr1216 promoter (SEQ.ID17) is derived from MPR1128 (SEQ.ID04) (described in section 2.2 of Example 2) by duplicating the sequence stretching from nucleotides -225 to -136 bp, this sequence comprising the "G" box and the activating element.

It was constructed by cloning into the vector pGEM3Z-1 (described in section 2.1 of Example 2) the following two promoter fragments:
The "MPr1216 (SEQ.ID17) 5' fragment", synthesized by PCR, was amplified from 5 ng of pMRT1128 matrix DNA with the aid of 100 pmol of each of the 2 oligodeoxynucleotides 5' ATCggAATTCgCCgATTACgTggCTTTAgC 3', containing the EcoRI restriction site, and 5' gCTCTAgACCAACACAAAAgAAgCTgg 3' possessing the XbaI restriction site, in the presence of 50 nmol of each of the dNTPs, of Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 10 min., the DNA was subjected to 25 cycles each consisting of the steps of denaturation at 94°C for 1 min., of hybridization at 55°C for 1 min. and of elongation at 72°C for 1 min. 30 sec. During the final cycle, the elongation was continued at 72°C for 5 min. The DNA fragment derived from the PCR amplification was isolated on a 1.5% agarose gel with the aid of the "Concert Rapid Gel Extraction System" kit, and hydrolysed with EcoRI for 1 h at 37°C. The DNA fragment was then subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgC12 buffer and 6 µl of 1 M DTT, and digested with XbaI for 1 h at 37°C.
The "MPr1216 (SEQ.ID17) 3' fragment", obtained by the hydrolysis of 5 µg of the plasmid pMRT1183 with the restriction enzymes XbaI and BamHI, was isolated on a 1.5% agarose gel with the aid of the "Concert Rapid Gel Extraction System" kit.

The ligation reaction was carried out with 50 ng of the "MPr1216 (SEQ.ID17) 5' fragment" and 50ng of the "MPr1216 (SEQ.ID17) 3' fragment" thus treated, and 50 ng of plasmid pGem3Z-1, in a 10 µl reaction mixture, in the presence of the T4 DNA ligase buffer (1X) and of 400 units of T4 DNA ligase (New England Biolabs), in the "GeneAmp PCR System 9700" thermocycler, as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 10 pmol of each of the 2 oligodeoxynucleotides 5' ATCggAATTCgCCgATTACgTggCTTTAgC 3' and 5' TACggATCCCCggggATCTCTAgTTTgTggTgC 3' in the "GeneAmp PCR System 9700" thermocycler, as described above.

The plasmid obtained was called pMRT1216, and the MPr1216 promoter sequence (SEQ.ID17) represented diagrammatically in Fig. I was verified by sequencing.

### 2.9. Construction of the MPr1217 promoter (SEQ.ID37).

The MPr1217 promoter (SEQ. ID37) is derived from MPR1128 (SEQ.ID04) (described in section 2.2 of Example 2) by direct repeat triplication of the sequence stretching from nucleotides -225 to -136 bp, this sequence comprising the "G" box and the activating element.

The MPr1217 promoter (SEQ.ID37) was constructed by inserting, into the XbaI restriction site of pMRT1183 (described in section 2.5 of Example 2), two identical promoter fragments synthesized by PCR from 5 ng of matrix DNA with the aid of 100 pmol of each of the 2 oligodeoxynucleotides 5' ATCggAATTCTAgACgCCgATTACgTggCTTTAgC 3', containing the EcoRI and XbaI restriction sites, and 5' gCTCTAgACCAACACAAAAgAagCTgg 3', possessing the XbaI restriction site, in the presence of 50 nmol of each of the dNTPs, of Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 10 min., the DNA was subjected to 25 cycles each consisting of the steps of denaturation at 94°C for 1 min., of hybridization at 55°C for 1 min. and of elongation at 72°C for 1 min. 30 sec. During the final cycle, the elongation was continued at 72°C for 5 min. The DNA fragment derived from the PCR amplification was isolated on a 1.5% agarose gel with the aid of the "Concert Rapid Gel Extraction System" kit, and hydrolysed with XbaI for 1 h at 37°C.

In parallel, the vector fragment was prepared from 10 µg of plasmid pMRT1183 by enzymatic digestion of the XbaI restriction site, located 5' of MPr1183 for 1 h at 37°C. The vector fragment thus linearized was dephosphorylated with 40 U of calf intestine alkaline phosphatase (New England Biolabs) in the presence of buffer 3 (1X) at 37°C for 1 h.

The ligation reaction was carried out with 50 ng of promoter fragment and 100 ng of vector fragment thus prepared, in a 10 µl reaction mixture, in the presence of the 1X T4 DNA ligase buffer (New England Biolabs) and of 400 units of T4 DNA ligase (New England Biolabs) in the "GeneAmp PCR System 9700" thermocycler, as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 10 pmol of each of the 2 oligodeoxynucleotides 5' ATCggAATTCgCCgATTACgTggCTTTAgC 3' and 5' TACggATCCCCggggATCTCTAgTTTgTggTgC 3' in the "GeneAmp PCR System 9700" thermocycler, as described above.

The plasmid obtained was called pMRT1217, and the MPr1217 promoter sequence (SEQ.ID37) represented diagrammatically in Fig. I, which was verified by sequencing, has a C deleted at position -317 [lacuna], 4 bp upstream of a "G"-like box.

### Example 3.

### Construction of plasmids containing chimeric promoter sequences.

### 3.1. Construction of the MPr1130 promoter (SEQ.ID05).

The MPr1130 promoter (SEQ.ID05) results from inserting, at position -65 bp of PrHMWG-Dx5 (SEQ.ID01), a 55-bp sequence corresponding to a duplication of the as-2 motif (Lam and Chua, 1989) and to the as-1 motif (Lam et al., 1989) of CaMV 35S. It was constructed by splicing by overlap extension the "MPr1130 (SEQ.ID05) 5' fragment" and the "MPr1130 (SEQ.ID05) 3' fragment", which had been synthesized by PCR.

The "MPr1130 (SEQ.ID05) 5' fragment" was amplified by PCR from 5 ng of pUC19-HMWG matrix DNA (described in section 2.1 of Example 2) with the aid of 20 pmol of each of the 2 oligodeoxynucleotides 5' TACgAATTCCCAgCTTTgAgTggCCgTAg 3', containing the EcoRI restriction site, and 5' TgCgTCATCCCTTAC-gTCA-gTggAgATATCACATCAATCTTgATATCACATCAATCACggTgAggTTTgTTTAgCCTAAg 3' , possessing the 55-bp sequence corresponding to a duplication of the as-2 motif (Lam and Chua, 1989) and to the as-1 motif (Lam et al., 1989) of CaMV 35S, in the presence of 10 nmol of each of the dNTPs, of Vent DNA polymerase buffer (1X) and of 2 U of Vent DNA polymerase (New England Biolabs), in a 50 µl reaction volume. The PCR amplification reaction was carried out in the "GeneAmp PCR System 9700" thermocycler. After a denaturation at 94°C for 5 min., the DNA was subjected to 15 cycles each consisting of the steps of denaturation at 94°C for 1 min., of hybridization at 55°C for 1 min. and of elongation at 72°C for 1 min. 30 sec. During the final cycle, the elongation was continued at 72°C for 5 min. Forty µl of the PCR reaction medium were then subjected to the action of 12.5 U of the Klenow fragment (New England Biolabs) in the presence of 20 nmol of each of the dNTPs for 10 min. at 37°C. The PCR product thus treated was then isolated on a 1.5% agarose gel with the aid of the "QIAquick Gel Extraction" kit.

The "MPr1130 (SEQ.ID05) 3' fragment" was synthesized and treated in the same way as the "MPr1130 (SEQ.ID05) 5' fragment", except that the 2 oligodeoxynucleotides used are 5' ATTgATgTgATATCAAg-ATTgATgTgATATCTCCACTgACgTAAgggATgACgCACACgCAgCCATggTCCTgAACCTTC 3', possessing the 55-bp sequence corresponding to a duplication of the as-2 motif (Lam and Chua, 1989) and to the as-1 motif (Lam et al., 1989) of CaMV 35S, and 5' TACggATCCCCggggATCTCTAgTTTgTggTgC 3', containing the BamHI restriction site.

The "MPr1130 (SEQ.ID05) 5' fragment" and the "MPr1130 (SEQ.ID05) 3' fragment" were then assembled by overlap extension so as to generate the "MPr1130 fragment (SEQ.ID05)". In order to do this, a PCR amplification was carried out using 7.5 µl of each of the two PCR products thus treated, with the aid of 20 pmol of each of the oligodeoxynucleotides 5' TACgAATTCCCAgCTTTgAgTggCCgTAg 3', containing the EcoRI restriction site, and 5' TACggATCCCCggggATCTCTAgTTTgTggTgC 3', possessing the BamHI restriction site, in the presence of 10 nmol of each of the dNTPs, of Vent DNA polymerase buffer (1X) and of 2 U of Vent DNA polymerase (New England Biolabs), in a 50 µl reaction volume. The PCR amplification reaction was carried out in the "GeneAmp PCR System 9700" thermocycler. After a denaturation at 94°C for 5 min., the DNA was subjected to 15 cycles each consisting of the steps of denaturation at 94°C for 1 min., of hybridization at 55°C for 1 min. and of elongation at 72°C for 1 min. 30 sec. During the final cycle, the elongation was continued at 72°C for 5 min. The "MPr1130 fragment (SEQ.ID05)" thus synthesized was isolated on a 1.5% agarose gel with the aid of the "QIAquick Gel Extraction" kit. This fragment was then hydrolysed with EcoRI for 1 h at 37°C, and subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 of µl of 1 M DTT. Finally, the MPr1130 fragment (SEQ.ID05) was digested with BamHI for 1 h at 37°C.

The ligation was carried out with 100 ng of the "MPr1130 fragment (SEQ.ID05)" thus treated and 50 ng of plasmid pGem3Z-1 (described in section 2.1 of Example 2) overnight at 16°C in a 10 µl reaction mixture, in the presence of 1 µl of the T4 DNA ligase buffer (1X) and of 400 units of T4 DNA ligase (New England Biolabs). *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 25 pmol of each of the 2 oligodeoxynucleotides 5' TACgAATTCCCAgCTTTgAgTggCCgTAg 3' and 5' TACggATCCCCggggATCTCTAgTTTgTggTgC 3' in the "GeneAmp PCR System 9700" thermocycler, as described above.

The plasmid obtained was called pMRT1130, and the MPr113 promoter sequence (SEQ.ID05) represented diagrammatically in Fig. III was verified by sequencing.

### 3.2. Construction of the MPr1131 promoter (SEQ.ID06).

The MPr1131 promoter (SEQ.ID06) results from inserting, at position -65 bp of PrHMWG-Dx5 (SEQ.ID01) (described in section 2.1 of Example 2), a 38-bp sequence corresponding to the as-2 motif (Lam and Chua, 1989) and to the as-1 motif (Lam et al., 1989) of CaMV 35S. It was constructed by splicing by overlap extension the "MPr1131 (SEQ.ID06) 5' fragment" and the "MPr1131 (SEQ.ID06) 3' fragment", which had been synthesized by PCR.

The "MPr1131 (SEQ.ID06) 5' fragment" was synthesized and treated in the same way as the "MPr1130 (SEQ.ID05) 5' fragment" (described in section 3.1 of Example 3), except that the 2 oligodeoxynucleotides used are 5' TACgAATTCCCAgCTTTgAgTggCCgTAg 3', containing the EcoRI restriction site, and 5' TgCgTCATCCCTTACgTCAgTggAgATATCACATCAATCACggTgAggTTTgTTTAgCCTA Ag 3', possessing the 38-bp sequence corresponding to the as-2 motif (Lam and Chua, 1989) and to the as-1 motif (Lam et al., 1989) of CaMV 35S.

The "MPr1131 (SEQ.ID06) 3' fragment" was synthesized and treated in the same way as the "MPr1130 (SEQ.ID05) 5' fragment" (described in section 3.1 of Example 3), except that the 2 oligodeoxynucleotides used are 5' ATTgATgTgATATCTCCACTgACgTAAgggATgAC-gCACACgCAgCCATggTCCTgAACCTTC 3' possessing the 38-bp sequence corresponding to the as-2 motif (Lam and Chua, 1989) and to the as-1 motif (Lam et al., 1989) of CaMV 35S, and 5' TACggATCCCCggggATCTCTAgTTTgTggTgC 3' containing the BamHI restriction site.

The "MPr1131 (SEQ.ID06) 5' fragment" and the "MPr1131 (SEQ.ID06) 3' fragment" were then assembled by overlap extension so as to generate the "MPr1131 fragment (SEQ.ID06)". In order to do this, a PCR amplification was carried out using 7.5 µl of each of the two PCR products thus treated, with the aid of 20 pmol of each of the oligodeoxynucleotides 5' TACgAATTCCCAgCTTTgAgTggCCgTAg 3', containing the EcoRI restriction site, and 5' TACggATCCCCggggATCTCTAgTTTgTggTgC 3', possessing the BamHI restriction site, in the presence of 10 nmol of each of the dNTPs, of Vent DNA polymerase buffer (1X) and of 2 U of Vent DNA polymerase (New England Biolabs), in a 50 µl reaction volume. The PCR amplification reaction was carried out in the "GeneAmp PCR System 9700" thermocycler. After a denaturation at 94°C for 5 min., the DNA was subjected to 15 cycles each consisting of the steps of denaturation at 94°C for 1 min., of hybridization at 55°C for 1 min. and of elongation at 72°C for 1 min. 30 sec. During the final cycle, the elongation was continued at 72°C for 5 min. The "MPr1131 fragment (SEQ.ID06)" thus synthesized was isolated on a 1.5% agarose gel with the aid of the "QIAquick Gel Extraction" kit. This fragment was then hydrolysed with EcoRI for 1 h at 37°C, and subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 of µl of 1 M DTT. Finally, the MPr1131 fragment (SEQ.ID06) was digested with BamHI for 1 h at 37°C.

The ligation was carried out with 100 ng of the "MPr1130 fragment (SEQ.ID05)" thus treated and 50 ng of plasmid pGem3Z-1 (described in section 2.1 of Example 2) overnight at 16°C in a 10 µl reaction mixture, in the presence of 1 µl of the T4 DNA ligase buffer (1X) and of 400 units of T4 DNA ligase (New England Biolabs). *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 25 pmol of each of the 2 oligodeoxynucleotides 5' TACgAATTCCCAgCTTTgAgTggCCgTAg 3' and 5' TACggATCCCCggggATCTCTAgTTTgTggTgC 3' in the "GeneAmp PCR System 9700" thermocycler, as described above.

The plasmid obtained was called pMRT1131, and the MPr1131 promoter sequence (SEQ.ID06) represented diagrammatically in Fig. II was verified by sequencing.

### 3.3. Construction of the MPr1135 promoter (SEQ.ID07)

The MPr1135 promoter (SEQ.ID07) is derived from the MPr1130 promoter (SEQ.ID05) (described in section 3.1 of Example 3) by deleting the sequence located upstream of nucleotide -293, this sequence comprising the two prolamine-"like boxes and the two GATA boxes.

The promoter fragment was amplified by PCR from 5 ng of pMRT1130 matrix DNA with the aid of 100 pmol of each of the 2 oligodeoxynucleotides 5' ATCGGAATTCCAGAACTAGGATTACGCCG 3', containing the EcoRI restriction site, and 5' TACggATCCCCggggATCTCTAgTTTgTggTgC 3', possessing the BamHI restriction site, in the presence of 50 nmol of each of the dNTPs, of the Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 5 min., the DNA was subjected to 25 cycles each consisting of the steps of denaturation at 94°C for 1 min., of hybridization at 55°C for 1 min. and elongation at 72°C for 1 min. 30 sec. During the final cycle, the elongation was continued at 72°C for 1 min.

The DNA fragment derived from the amplification was isolated on a 2% agarose gel with the aid of the "QIAquick Gel Extraction" kit, hydrolysed with EcoRI for 1 h at 37°C and then subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 µl of 1 M DTT. The DNA fragment thus treated was then digested with BamHI for 1 h at 37°C.

The ligation was carried out with 100 ng of the MPR1135 promoter fragment (SEQ.ID07) thus treated and 50 ng of plasmid pGEM3Z-1 (described in section 2.1 of Example 2) overnight at 16°C in a 10 µl reaction mixture, in the presence of the T4 DNA ligase buffer (1X) and of 400 units of T4 DNA ligase (New England Biolabs). *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 25 pmol of each of the 2 oligodeoxynucleotides 5' ATCGGAATTCGTGTTGGCAAACTGC 3' and 5' TACggATCCCCggggATCTCTAgTTTgTggTgC 3', in the "GeneAmp PCR System 9700" thermocycler, as described above.

The plasmid obtained was called pMRT1135, and the MPr113 promoter sequence (SEQ.ID07) represented diagrammatically in Fig. III was verified by sequencing.

### 3.4. Construction of the MPr1138 promoter (SEQ.ID10) .

The MPr1138 promoter (SEQ.ID10) is derived from the MPr1131 promoter (SEQ.ID06) (described in section 3.2 of Example 3) by deleting the sequence located upstream of nucleotide -276, this sequence comprising the two prolamine-"like" boxes and the two GATA boxes.

The promoter fragment was amplified by PCR from 5 ng of pMRT1131 matrix DNA, treated and obtained in the same way as the MPr1135 promoter (SEQ.ID07) (described in section 3.3 of Example 3).

The plasmid obtained was called pMRT1138, and the MPr1138 promoter sequence (SEQ.ID10) represented diagrammatically in Fig. II was verified by sequencing.

### 3.5. Construction of the MPr1137 promoter (SEQID09).

The MPr1137 promoter (SEQ.ID11) is derived from the MPr1131 promoter (SEQ.ID06) (described in section 3.2 of Example 3) by deleting the sequence located upstream of nucleotide -243, this sequence comprising the two prolamine-"like" boxes, the two GATA boxes and the "G"-like box.

The promoter fragment was amplified by PCR from 5 ng of pMRT1131 matrix DNA with the aid of 100 pmol of each of the 2 oligodeoxynucleotides 5' ATCGGGAATTCGCAGACTGTCCAAAAATC 3', containing the EcoRI restriction site, and 5' TACggATCCCCggggATCTCTAgTTTgTggTgC 3', possessing the BamHI restriction site, treated and obtained in the same way as the MPr1135 promoter (SEQ.ID07) (described in section 3.3 of Example 3).

The plasmid obtained was called pMRT1137, and the MPr113 promoter sequence (SEQ.ID09) represented diagrammatically in Fig. II was verified by sequencing.

### 3.6. Construction of the MPr1134 promoter (SEQ.ID08).

The MPr113 promoter (SEQ.ID08) is derived from the MPr113 promoter (SEQ.ID05) (described in section 3.1 of Example 3) by deleting the sequence located upstream of nucleotide -260, this sequence comprising the two prolamine-"like" boxes, the two GATA boxes and the "G"-like box.

The promoter fragment was amplified by PCR from 5 ng of pMRT1130 matrix DNA with the aid of 100 pmol of each of the 2 oligodeoxynucleotides 5' ATCGGGAATTCGCAGACTGTCCAAAAATC 3', containing the EcoRI restriction site, and 5' TACggATCCCCggggATCTCTAgTTTgTggTgC 3', possessing the BamHI restriction site, treated and obtained in the same way as the MPr1135 promoter (SEQ.ID07) (described in section 3.3 of Example 3).

The plasmid obtained was called pMRT1134, and the MPr1134 promoter sequence (SEQ.ID08) represented diagrammatically in Fig. III was verified by sequencing.

### 3.7. Construction of the MPr1136 promoter (SEQ.ID08).

The MPr1136 promoter (SEQ.ID08) is derived from the MPr1131 promoter (SEQ.ID06) (described in section 3.2 of Example 3) by deleting the sequence located upstream of nucleotide -180, this sequence comprising the two prolamine-"like" boxes, the two GATA boxes, the "G"-like box and the activating element.

The promoter fragment was amplified by PCR from 5 ng of pMRT1131 matrix DNA with the aid of 100 pmol of each of the 2 oligodeoxynucleotides 5' ATCGGAATTCGTGTTGGCAAACTGC 3', containing the EcoRI restriction site, and 5' TACggATCCCCggggATCTCTAgTTTgTggTgC 3', possessing the BamHI restriction site, treated and obtained in the same way as the MPr113 promoter (SEQ.ID07) (described in section 3.3 of Example 3).

The plasmid obtained was called pMRT1136, and the MPr1136 promoter sequence (SEQ.ID08) represented diagrammatically in Fig. II was verified by sequencing.

### 3.8. Construction of the MPr1133 promoter (SEQ.ID35).

The MPr1133 promoter (SEQ.ID35) is derived from the MPr1130 promoter (SEQ.ID05) (described in section 3.2 of Example 3) by deleting the sequence located upstream of nucleotide -197, this sequence comprising the two prolamine-"like" boxes, the two GATA boxes, the "G"-like box and the activating element.

The promoter fragment was amplified by PCR from 5 ng of pMRT1130 matrix DNA with the aid of 100 pmol of each of the 2 oligodeoxynucleotides 5' ATCGGAATTCGTGTTGGCAAACTGC 3', containing the EcoRI restriction site, and 5' TACggATCCCCggggATCTCTAgTTTgTggTgC 3', possessing the BamHI restriction site, treated and obtained in the same way as the MPr1135 promoter (SEQ.ID07) (described in section 3.3 of Example 3).

The plasmid obtained was called pMRT1133, and the MPr1133 promoter sequence (SEQ.ID35) represented diagrammatically in Fig. III was verified by sequencing.

### 3.9. Construction of the MPr1139 promoter (SEQ.ID11)

The MPr1139 promoter (SEQ.ID11) results from inserting, at position -405 bp of MPr1131 (SEQ.ID06) (described in section 3.2 of Example 3), a 61-bp sequence which includes the duplication of the "cereal" box of the promoter of the high molecular weight glutenin gene encoding the Bx7 subunit (PrHMWG-Bx7) of the hexaploid wheat *Triticum aestivum L. cv Cheyenne* (Anderson et al., 1998).

The MPr1139 promoter (SEQ.ID11) was amplified by PCR from 5 ng of pMRT1131 matrix DNA with the aid of 100 pmol of each of the 2 oligodeoxynucleotides 5' TACgAATTCCTCgACATggTTAgAAgTTTTgAgTgCCgCCACTACTCgACAT-ggTTAgAAgTTTTgAgTggCCgTAgATTTgC 3', containing the EcoRI restriction site and the two "cereal" boxes described above, and 5' TACggATCCCCggggATCTCTAgTTTgTggTgC 3', possessing the BamHI restriction site, in the presence of 50 nmol of each of the dNTPs, of the Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 5 min., the DNA was subjected to 25 cycles each consisting of the steps of denaturation at 94°C for 1 min., of hybridization at 55°C for 1 min. and of elongation at 72°C for 1 min. 30 sec. During the final cycle, the elongation was continued at 72°C for 1 min.

The DNA fragment derived from the amplification was isolated on a 2% agarose gel with the aid of the "QIAquick Gel Extraction" kit and hydrolysed with EcoRI for 1 h at 37°C. The DNA fragment was then subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 µl of 1 M DTT, and digested with BamHI for 1 h at 37°C.

The ligation was carried out with 100 ng of the MPr1139 promoter fragment (SEQ.ID11) thus treated and 50 ng of plasmid pGem3Z-1 (described in section 2.1 of Example 2) overnight at 16°C, in a 10 µl reaction mixture, in the presence of the T4 DNA ligase buffer (1X) and of 400 units of T4 DNA ligase (New England Biolabs). *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 25 pmol of each of the 2 oligodeoxynucleotides 5' ATCGGAATTCCAGAACTAGGATTACGCCG 3' and 5' TACggATCCCCggggATCTCTAgTTTgTggTgC 3', in the "GeneAmp PCR System 9700" thermocycler, as described above.

The plasmid obtained was called pMRT1139, and the MPr1139 promoter sequence (SEQ.ID11) represented diagrammatically in Fig. IV was verified by sequencing.

### 3.10. Construction of the MPr1200 promoter (SEQ.ID15).

The MPr1200 promoter (SEQ.ID15) results from inserting, at position -263 bp of MPr1138 (SEQ.ID10) (described in section 3.4 of Example 3), a 79-bp sequence which includes the duplication of the "cereal" box of the promoter of the high molecular weight glutenin gene encoding the Bx7 subunit (PrHMWG-Bx7) of the hexaploid wheat *Triticum aestivum L. cv Cheyenne* (Anderson et al., 1998).

The MPr1200 promoter (SEQ.ID15) was constructed by cloning into the vector pGEM3Z-1 (described in section 2.1 of Example 2) the following two promoter fragments:
- The "MPr1200 (SEQ.ID15) 5' fragment", synthesized by PCR, was amplified from 5 ng of pMRT1139 matrix DNA (described in section 3.9 of Example 3) with the aid of 100 pmol of each of the 2 oligodeoxynucleotides 5' TACgAATTCCTCgACATgg 3', containing the EcoRI restriction site, and 5' gCTCTAgAgCAAATCTACggCCACTC 3', possessing the XbaI restriction site, in the presence of 50 nmol of each of the dNTPs, of Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 10 min., the DNA was subjected to 25 cycles each consisting of the steps of denaturation at 94°C for 1 min., of hybridization at 55°C for 1 min. and of elongation at 72°C for 1 min. 30 sec. During the final cycle, the elongation was continued at 72°C for 5 min. The DNA fragment derived from the PCR amplification was isolated on a 1.5% agarose gel with the aid of the "Concert Rapid Gel Extraction System" kit, and hydrolysed with EcoRI for 1 h at 37°C. The DNA fragment was then subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 µl of 1 M DTT, and then digested with XbaI for 1 h at 37°C.
- The "MPr1200 (SEQ.ID15) 3' fragment", synthesized by PCR, as amplified from 5 ng of pMRT1138 matrix DNA with the aid of 100 pmol of each of the 2 oligodeoxynucleotides 5' ATCggAATTCTAgACgCCgATTACgTggCTTTAgC 3', containing the EcoRI restriction site and XbaI restriction site, and 5' TACggATCCCCggggATCTCTAgTTTgTggTgC 3', possessing the BamHI restriction site, in the presence of 50 nmol of each of the dNTPs, of Vent DNA polymerase buffer (1X) and of 2 U of Vent DNA polymerase (New England Biolabs) in the "GeneAmp PCR System 9700" thermocycler, under the same conditions as the "MPr1200 (SEQ.ID15) 5' fragment". The DNA fragment derived from the PCR amplification was isolated on a 1.5% agarose gel with the aid of the "Concert Rapid Gel Extraction System" kit and hydrolysed successively with XbaI and BamHI for 1 h at 37°C.

The ligation reaction was carried out with 50 ng of the "MPr1200 (SEQ.ID15) 5' fragment", 50 ng of the "MPr1200 (SEQ.ID15) 3' fragment" thus treated, and 50 ng of plasmid pGem3Z-1, in a 10 µl reaction mixture, in the presence of the T4 DNA ligase buffer (1X) and of 400 units of T4 DNA ligase (New England Biolabs) in the "GeneAmp PCR System 9700" thermocycler, as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 10 pmol of each of the 2 oligodeoxynucleotides 5' ATCggAATTCgCAgCCATggTCCTgAACC 3' and 5' TACggATCCCCggggATCTCTAgTTTgTggTgC 3', in the "GeneAmp PCR System 9700" thermocycler, as described above.

The plasmid obtained was called pMRT1200, and the MPr1200 promoter sequence (SEQID15 )represented diagrammatically in Fig. IV was verified by sequencing.

### 3.11. Construction of the MPr1213 promoter (SEQ.ID16)

The MPr1213 promoter (SEQ.ID16) results from inserting, upstream of position -225 bp of MPR1128 (SEQ.ID04) (described in section 2.2 of Example 2), a 79-bp sequence which includes the duplication of the "cereal" box of the promoter of the high molecular weight glutenin gene encoding the Bx7 subunit (PrHMWG-Bx7) of the hexaploid wheat *Triticum aestivum L. cv Cheyenne* (Anderson et al., 1998).

The MPr1213 promoter (SEQ.ID16) was constructed by cloning into the vector pGEM3Z-1 (described in section 2.1 of Example 2), the following two promoter fragments:
The "MPr1213 (SEQ. ID16) 5' fragment", synthesized by PCR, was amplified from 5 ng of pMRT1139 matrix DNA (described in section 3.9 of Example 3) with the aid of 100 pmol of each of the 2 oligodeoxynucleotides 5' TACgAATTCCTCgACATgg 3', containing the EcoRI restriction site, and 5' gCTCTAgAgCAAATCTACggCCACTC 3', possessing the XbaI restriction site, in the presence of 50 nmol of each of the dNTPs, of Vent DNA polymerase buffer (1X) and of 2 U Vent DNA polymerase (New England Biolabs). The PCR amplification reaction was carried out in the "GeneAmp PCR System 9700" thermocycler. After a denaturation at 94°C for 10 min., the DNA was subjected to 25 cycles each consisting of the steps of denaturation at 94°C for 1 min., of hybridization at 55°C for 1 min. and of elongation at 72°C for 1 min. 30 sec. During the final cycle, the elongation was continued at 72°C for 5 min. The DNA fragment derived from the PCR amplification was isolated on a 1.5% agarose gel with the aid of the "Concert Rapid Gel Extraction System" kit, and hydrolysed with EcoRI for 1 h at 37°C. The DNA fragment was then subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 µl of 1 M DTT, and digested with XbaI for 1 h at 37°C.
The "MPr1213 (SEQ.ID16) 3' fragment", obtained by the hydrolysis of 5 µg of the plasmid pMRT1183 (described in section 2.5 of Example 2) with the XbaI and BamHI restriction enzymes, was isolated on a 1.5% agarose gel with the aid of the "Concert Rapid Gel Extraction System" kit.

The ligation reaction was carried out with 50 ng of the "MPr1213 (SEQ.ID16) 5' fragment" and 50 ng of the "MPr1213 (SEQ.ID16) 3' fragment" thus treated, and 50 ng of plasmid pGem3Z-1, in a 10 µl reaction mixture, in the presence of the T4 DNA ligase buffer (1X) and of 400 units of T4 DNA ligase (New England Biolabs) in the "GeneAmp PCR System 9700" thermocycler, as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 10 pmol of each of the 2 oligodeoxynucleotides 5' TACgAATTCCTCgACATgg 3' and 5' gCTCTAgAgCAAATCTACggCCACTC 3', in the "GeneAmp PCR System 9700" thermocycler, as described above.

The plasmid obtained was called pMRT1213, and the MPr1213 promoter sequence (SEQ.ID16) represented diagrammatically in Fig. IV was verified by sequencing.

### 3.12. Construction of the MPr1199 promoter (SEQ.ID14).

The MPr119 promoter (SEQ.ID14) results from inserting, at position -224 bp of MPR1128 (SEQ.ID04) (described in section 2.2 of Example 2), a 27-bp sequence which includes the "GC-rich" element of the intergenic region of the maize streak virus (MSV) (Fenoll et al., 1990).

The MPr1199 promoter (SEQ.ID14) was amplified by PCR from 5 ng of pMRT1128 matrix DNA with the aid of 100 pmol of each of the 2 oligodeoxynucleotides 5' ATCGGAATTCAAATGGGCCGGACCGGGCCGGCCCAGCGCCGATTACGTGGCTTTAGC 3', containing the "GC-rich" element described above and the EcoRI and XbaI restriction sites, and 5' TACggATCCCCggggATCTCTAgTTTgTggTgC 3', possessing the BamHI restriction site, in the presence of 50 nmol of each of the dNTPs, of the Vent DNA polymerase buffer (1X) and of 2 U of Vent DNA polymerase (New England Biolabs). The PCR amplification reaction was carried out in the "GeneAmp PCR System 9700" thermocycler. After a denaturation at 94°C for 5 min., the DNA was subjected to 25 cycles each consisting of the steps of denaturation at 94°C for 1 min., of hybridization at 55°C for 1 min. and of elongation at 72°C for 1 min. 30 sec. During the final cycle, the elongation was continued at 72°C for 1 min.

The DNA fragment derived from the amplification was isolated on a 1.5% agarose gel with the aid of the "Concert Rapid Gel Extraction System" kit and hydrolysed with EcoRI for 1 h at 37°C. The fragment was then subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 µl of 1 M DTT, and digested with BamHI for 1 h at 37°C.

The ligation was carried out with 100 ng of the MPr1199 promoter fragment (SEQ.ID14) thus treated and 50 ng of plasmid pGem3Z-1 (described in section 2.1 of Example 2), with PCR cycles in the "GeneAmp PCR System 9700" thermocycler under the conditions described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was analysed by PCR with the aid of 25 pmol of each of the 2 oligodeoxynucleotides 5' ATCGGAATTCCAGAACTAGGATTACGCCG 3' and 5' TACggATCCCCggggATCTCTAgTTTgTggTgC 3' in the "GeneAmp PCR System 9700" thermocycler, as described above.

The plasmid obtained was called pMRT1199mut, and the corresponding MPr1199 (SEQ.ID14) mut promoter sequence, which was verified by sequencing, has a mutation in the untranslated leader sequence at position +27 [lacuna]. To reestablish the unmutated MPr1199 sequence (SEQ.ID14), the "MPr1199 (SEQ.ID14) 5' fragment" stretching from position -251 to -58 bp was cloned in the place of the "MPr1183 5' fragment" stretching from position -225 to -58 bp.

In order to do this, 10 µg of plasmid pMRT1199mut were digested with EcoRI for 1 h at 37°C, and subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgCl2 buffer and 6 µl of 1 M DTT. After a digestion with NcoI for 1 h at 37°C, the "MPr1199 (SEQ.ID14) 5' fragment" was isolated on a 1.5% agarose gel with the aid of the "Concert Rapid Gel Extraction System" kit.

In parallel, 5 µg of plasmid pMRT1183 (described in section 2.5 of Example 2) were digested for 1 h at 37°C with XbaI, and subjected to the action of 20 U of the Klenow fragment (New England Biolabs) for 30 min at 37°C in the presence of 60 nmol of each of the dNTPs, of 12 µl of 500 mM Tris-HCL, pH 7.5/500 mM MgC12 buffer and 6 µl of 1 M DTT. After a digestion with NcoI, the vector pMRT1183 thus deleted of the "MPR1183 5' fragment" was isolated on a 0.8% agarose gel with the aid of the "Concert Rapid Gel Extraction System" kit, and dephosphorylated with 40 U of calf intestine alkaline phosphatase (New England Biolabs) in the presence of buffer 3 (1X) at 37°C for 1 h.

The ligation reaction was carried out with 100 ng of the "MPr119 (SEQ.ID14) mut 5' fragment" and 50 ng of the plasmid pMRT1183 thus treated, in a 10 µl reaction mixture, in the presence of the T4 DNA ligase buffer (1X) and of 400 units of T4 DNA ligase (New England Biolabs), in the "GeneAmp PCR System 9700" thermocycler, as described above. *Escherichia coli* DH5a bacteria, which had been made competent beforehand, were transformed with all of the ligation reaction mixture. The plasmid DNA of the clones obtained, which were selected on LB medium supplemented with ampicillin (50 mg/l), was extracted according to the alkaline lysis method and analysed by enzymatic digestion.

The plasmid obtained was called pMRT1199, and the MPr1199 promoter sequence (SEQ.ID14) is represented diagrammatically in Fig. IV.

### Example 4.

### Construction of the binary plasmids containing the MPr1130 (SEQ.ID05), MPr1131 (SEQ.ID06), MPr1135 (SEQ.ID7), MPr1138 (SEQ.ID10), MPr1139 (SEQ.ID11) and MPr1092 promoters used in the stable transformation of tobacco.

### 4.1. Construction of the binary plasmid pMRT1177.

The binary plasmid pMRT1177 was obtained by inserting the expression cassette "MPr1130 (SEQ.ID05)/*uidA*-IV2/term-*nos*" of pMRT 1130 (described in section 3.1 of Example 3) into the EcoRI site of the binary plasmid pMRT1118 (unpublished patent application FR 9911112).

In order to do this, 10 µg of plasmid pMRT1130 were digested successively with EcoRI and XmnI for 1 h at 37°C. The expression cassette was then isolated on a 0.8% agarose gel with the aid of the "Concert Rapid Gel Extraction System" kit.

In parallel, 10 µg of binary plasmid pMRT1118 were digested with EcoRI for 1 h at 37°C. The linearized vector fragment was then dephosphorylated with 40 U of calf intestine alkaline phosphatase (New England Biolabs) in the presence of buffer 3 (1X) at 37°C for 1 h.

The ligation was carried out with 50 ng of the expression cassette and 100 ng of plasmid pMRT1118 thus treated, overnight at 16°C in a 10 µl reaction mixture, in the presence of the T4 DNA ligase buffer (1X) and of 400 units of T4 DNA ligase (New England Biolabs). *Escherichia coli* DH5a bacteria, which had been made competent beforehand, were transformed with all of the ligation reaction mixture. The plasmid DNA of the clones obtained, which were selected on LB medium supplemented with kanamycin (50 mg/l), was extracted according to the alkaline lysis method and analysed with enzymatic digestions.

The plasmid obtained, called pMRT1177, was transferred into the LBA4404 *Agrobacterium tumefaciens* strain according to the technique described by Holsters et al. (1978). The plasmid DNA of the clones obtained, which were selected on 2YT medium (10 g/l bactotryptone, 10 g/l yeast extract, 5 g/l NaCl, pH 7.2 and 15 g/l Agar-Agar) supplemented with rifampicin (50 mg/l) and with kanamycin (50 mg/l), was extracted according to the alkaline lysis method, which was modified by adding lysozyme (25 mg/ml) to the cell resuspension buffer. The plasmid DNA was analysed with enzymatic digestions and the agrobacterium clone obtained was called A1177.

### 4.2. Construction of the binary plasmid pMRT1178.

The binary plasmid pMRT1178 was obtained by inserting the expression cassette "MPr1131 (SEQ.ID06)/*uidA*-IV2/term-*nos*" into the EcoRI site of the binary plasmid pMRT1118, as described in section 4.1 of Example 4, except that the expression cassette was isolated from the plasmid pMRT1131 (described in section 3.2 of Example 3).

The resulting plasmid was called pMRT1178, and was transferred into the LBA4404 *Agrobacterium tumefaciens* strain according to the protocol described above in section 4.1 of Example 4. The agrobacterium clone obtained was called A1178.

### 4.3. Construction of the binary plasmid pMRT1179.

The binary plasmid pMRT1179 was obtained by inserting the expression cassette "MPr1135 (SEQ.ID07) /uidA-IV2/term-nos" into the EcoRI site of the binary plasmid pMRT1118, as described in section 4.1 of Example 4, except that the expression cassette was isolated from the plasmid pMRT1135 (described in section 3.3 of Example 3).

The resulting plasmid was called pMRT1179, and was transferred into the LBA4404 *Agrobacterium tumefaciens* strain according to the protocol described above in section 4.1 of Example 4. The agrobacterium clone obtained was called A1179.

### 4.4. Construction of the binary plasmid pMRT1180.

The binary plasmid pMRT1180 was obtained by inserting the expression cassette "MPr1138 (SEQ.ID10)/*uidA*-IV2/term-*nos*" into the EcoRI site of the binary plasmid pMRT1138, as described in section 4.1 of Example 4, except that the expression cassette was isolated from the plasmid pMRT1138 (described in section 3.4 of Example 3).

The resulting plasmid was called pMRT1180, and was transferred into the LBA4404 *Agrobacterium tumefaciens* strain according to the protocol described above in section 4.1 of Example 4. The agrobacterium clone obtained was called A1180.

### 4.5. Construction of the binary plasmid pMRT1181.

The binary plasmid pMRT1181 was obtained by inserting the expression cassette "MPr1139 (SEQ.ID11)/*uid*A-IV2/term-*nos*" into the EcoRI site of the binary plasmid pMRT1118, as described in section 4.1 of Example 4, except that the expression cassette was isolated from the plasmid pMRT1139 (described in section 3.9 of Example 3).

The resulting plasmid was called pMRT1181, and was transferred into the LBA4404 *Agrobacterium tumefaciens* strain according to the protocol described above in section 4.1 of Example 4. The agrobacterium clone obtained was called A1181.

### 4.6. Construction of the binary plasmid pMRT1182.

The binary plasmid pMRT1182 was obtained by inserting the CaMV PrD35S promoter fragment and the sequence uidA-IV2/term-nos into the binary plasmid pMRT1118.

CaMV PrD35S was isolated by digesting 10 µg of the plasmid pJIT163D successively with KpnI and with HindIII for 1 h at 37°C. The 743-bp fragment corresponding to CaMV PrD35S was separated on a 0.8% agarose gel, and then purified with the aid of the "QIAquick Gel Extraction" kit.

The sequence *uid*A-IV2/term-*nos* was obtained by digesting 4 µg of plasmid pMRT1092 with HindIII and EcoRI for 1 h at 37°C. The 2.2-kb fragment corresponding to the sequence uidA-IV2/term-nos was separated on a 0.8% agarose gel, and then purified with the aid of the "QIAquick Gel Extraction" kit.

In parallel, 10 µg of binary plasmid pMRT1118 were digested successively with KpnI and EcoRI for 1 h at 37°C. The linearized vector fragment was then dephosphorylated with 40 U of calf intestine alkaline phosphatase (New England Biolabs) in the presence of buffer 3 (1X), for 1 h at 37°C.

The ligation was carried out in the presence of 100 ng of binary plasmid, 50 ng of the CaMV PrD35S fragment and 50 ng of the fragment corresponding to the sequence *uidA*-IV2/term-nos in a 20 µl reaction volume, in the presence of the T4 DNA ligase buffer (1X) and 400 units of T4 DNA ligase enzyme (New England Biolabs). The incubation was carried out with PCR cycles in the "GeneAmp PCR System 9700" thermocycler as described above. *Escherichia coli* DH5a bacteria, which had been made competent beforehand, were transformed with half of the ligation reaction medium. The plasmid DNA of the clones obtained, which were selected on LB medium supplemented with kanamycin (50 mg/l), was extracted according to the alkaline lysis method and analysed with enzymatic digestions.

The resulting plasmid was called pMRT1182, and was transferred into the LBA4404 *Agrobacterium* tumefaciens strain according to the protocol described above in section 4.1 of Example 4. The agrobacterium clone obtained was called A1182.

### Example 5.

### Construction of the binary plasmid pMRT1207 containing the MPr1139 promoter (SEQ.ID11), used in the stable transformation of maize.

The binary plasmid pMRT1207 was obtained by inserting the expression cassette "MPr1139 (SEQ.ID11)/*uidA*-IV2/term-*nos*" of pMRT 1139 into the HpaI site of the binary plasmid pMRT1195 (unpublished Patent application FR 9911112).

In order to do this, 7 µg of plasmid pMRT1139 were digested successively with EcoRI and XmnI for 1 h at 37°C. The expression cassette was then isolated on a 0.7% agarose gel with the aid of the "Concert Rapid Gel Extraction System" kit, and subjected to the action of 20 U of the Klenow fragment (New England Biolabs) for 30 min. at 37°C in the presence of 60 nmol of each of the dNTPs, of 12 µl of MgCl2 buffer (500 mM) and of 6 µl of DTT (1 M).

In parallel, 5 µg of binary plasmid pMRT1195 were digested with HpaI for 1 h at 37°C. The linearized vector fragment was then dephosphorylated with 40 U of calf intestine alkaline phosphatase (New England Biolabs) in the presence of buffer 3 (1X) at 37°C for 1 h.

The ligation was carried out with 100 ng of the expression cassette and 10 ng of plasmid pMRT1195 thus treated, with PCR cycles in the "GeneAmp PCR System 9700" thermocycler, as described above. *Escherichia coli* DH5a bacteria, which had been made competent beforehand, were transformed with all of the ligation reaction mixture. The plasmid DNA of the clones obtained, which were selected on LB medium supplemented with kanamycin (50 mg/l), was extracted according to the alkaline lysis method and analysed with enzymatic digestions.

The plasmid obtained, called pMRT1207, was transferred, as described in section 4.1 of Example 4, into the LBA4404-pSB1 *Agrobacterium tumefaciens* strain, this strain being derived from the LBA4404 *Agrobacterium tumefaciens* strain subsequent to the integration of the plasmid pSB1 (unpublished Patent application FR 9911112), according to the protocol described above for the production of A1177. The plasmid DNA of the clones obtained, which were selected on 2YT medium supplemented with rifampicin (50 mg/l), with kanamycin (50 mg/l) and with tetracycline (5 mg/l), was extracted according to the alkaline lysis method, which was modified by adding lysozyme (25 mg/ml) to the cell resuspension buffer. The plasmid DNA was analysed with enzymatic digestions, and the agrobacterium clone obtained was called A1207.

### Example 6.

### Measurement and comparison of the activity of the various promoters in transient expression in maize and tobacco.

### 6.1 Preparation of plant extracts.

### 6.1.1. Production and preparation of maize seeds.

The transient expression experiments were carried out on the maize albumen SN 87 165 (L2), removed from maize plants cultivated in a phytotron at 24°C, under 60% humidity and a photoperiod of 16 h light/8 h darkness.

Twelve days after pollination (DAP), the maizes were removed and sterilized in a bath of 20% domestos with stirring for 5 min. Following the removal of the domestos with successive rinses in sterile deionized water, the pericarp and the layer of aleurone cells are carefully removed under sterile conditions. Tangential sections of the albumen thus extracted were prepared and placed on filter paper soaked in minimum murashige and Skoog medium (MS 5524, Sigma).

### 6.1.2. In vitro culture of tobacco, preparation of the leaves.

The transient expression experiments were carried out on 6-week-old leaves of tobacco *(Nicotiana tabacum* L.) of the cultivar PBD6. Mature seeds of tobacco cv. PBD6 were sterilized for 10 min in a saturated solution of calcium hypochlorite (70 g/l), and then rinsed three times for 5 min in sterile deionized water. The sterile seeds were placed on MS20 medium (Murashige and Skoog, 1962) and incubated for 6 weeks in a culture chamber (constant temperature of 24°C, 16 h light/8h darkness photoperiod).

In order to minimize the destruction of the cells of the foliar mesophyll during the transformation by biolistics, the 2 main leaves of the 6-week-old PBD6 tobacco plants were removed 24 h before transformation, placed, with the upper side of the leaf facing upwards, on the BY3 gentle plasmolysis medium (4.4 g/l MS-5519 salts, 100 mg/l myoinositol, 1 mg/l thiamine, 200 mg/l KH₂PO₄, 30 g/l sucrose, 45.5 g/l sorbitol, 1 mg/l 2.4 D, 8 g/l agar, pH 5.8), and placed in a culture chamber (constant temperature of 25°C, 16 h light/ 8 h darkness photoperiod).

### 6.2. Adsorption of the plasmid DNA onto tungsten or gold microparticles.

The transformation by biolistics required the DNA to be deposited beforehand on spherical microparticles made of tungsten or of gold, sterilized for 10 min in absolute ethanol (99.98%, containing less than 0.02% of water), washed four times in sterile deionized water, and conserved at -20°C in a solution of 50% glycerol for a maximum of 4 weeks.

The concentration of all of the control and test plasmids used during the transformation was adjusted to 1 mg/ml. In each of the transformation experiments in which the activity of the promoter studied was evaluated using a luminometric assay, an internal reference control (pCaMV35Sluc) was cotransformed in order to normalize the variations in the GUS activity between the various experiments (Leckie et al., 1994). However, when the activity of the promoter studied was determined using a histochemical assay, the reference plasmid was not cotransformed.

The coating of the DNA onto the particles thus prepared was carried out in a sterile laminar flow chamber. A 1.8 mg aliquot fraction of sterile particle suspension in 30 µl of 50% glycerol was vigorously mixed by vortexing for 1 min., and then for 10 sec. with 20 µl of the DNA suspension containing 5 µg of one of the plasmids to be tested and 2 µg of the reference plasmid pCaMV35Sluc. Then 20 µl of 2.5 M CaCl₂ were added and vigorously mixed for 10 sec. Next, 20 µl of 0.1 M spermidine were added to the mixture, and all of this was stirred by vortexing for a further 30 sec. The coating of the DNA onto the beads was continued by incubating the mixture in ice for 15 min, and then the coated beads were centrifuged at low speed for 5 sec and washed twice in absolute ethanol. The particles thus coated were resuspended in 32 µl of absolute ethanol, mixed vigorously by vortexing for 15 sec., and then immediately distributed as 4 identical aliquot fractions onto the sterile "microcarrier" discs of the PDS-1000/He Biolistic system which had been prepared according to the manufacturer's recommendations (Bio-Rad, Hercule, USA). The "microcarrier support/microcarrier bearing the particle deposit" set was dried **for 5 min.**

### 6.3. Transient transformation of plant extracts by biolistics.

### 6.3.1. Bombarding the maize albumens and transient expression.

The bombarding of the maize albumens was carried out using the PDS-1000/He Biolistic system according to the general recommendations of the supplier (Bio-Rad, Hercule, USA) concerning the handling and assembly of the various elements of the equipment. Each albumen was bombarded twice successively with tungsten particles 0.6 µm in diameter, according to the following firing characteristics:
- the helium pressure chosen to accelerate the particles is 6200 kPa (900 psi),
- the plant sample is placed 6 cm from the bead acceleration zone,
- the firing is carried out under a reduced atmosphere of 27 mm of mercury.

Following the bombarding, the albumens were left in the same conditions and were incubated for 24 h in the dark in a culture chamber at 26°C, in order to allow the transient expression of the transgenes introduced into the cells.

### 6.3.2. Bombarding the tobacco foliar tissues and transient expression.

The bombarding of the tobacco leaves was carried out in the same way as the bombarding of the maize albumens, with two exceptions:
- the samples were bombarded with gold particles 1 pm in diameter,
- the samples were placed 9 cm from the bead acceleration zone.

After bombarding, the leaves were left in the same conditions and were incubated for 48 h in a culture chamber (constant temperature of 25°C, 16 h light/8 h darkness photoperiod), in order to allow the transient expression of the transgenes introduced into the cells.

### 6.4. Revelation and evaluation of the activity of the various promoters by histochemical staining.

### 6.4.1. Revelation of B-glucuronidase expression.

A revelation of b-glucuronidase expression was carried out by histochemical staining as described by Jeffersson et al. (1987). Following the incubation period in a culture chamber, the plant extracts were incubated in the presence of the β-glucuronidase substrate X-Gluc (500 mg/l 5-bromo-4-chloro-3-indolyl glucuronide), in 0.1 M phosphate buffer, 0.05% Triton X100, pH 7.0, for 24 h at 37°C.

After staining, the maize albumens were directly analysed or conserved sterilely at 4°C for several weeks, whereas the tobacco leaves were depigmented by two successive passages through 95% ethanol baths for 3 and 12 h, respectively, and then rinsed in distilled water and dried flat between two cellophane sheets.

The promoter activity of the various constructs was evaluated by estimating the number and intensity of the blue dots revealed on each plant extract.

### 6.4.2. Qualitative evaluation of the activity of the promoters in the maize albumen.

The histochemical revelation of the b-glucuronidase expression made it possible to identify three categories of promoter:
- The albumens bombarded with the pMRT1197, pMRT1126, pMRT1127 and pMRT1199 construct systematically exhibit a number of blue dots which is lower than 10. The presence of blue spots on the albumens transformed with the pMRT1197 construct indicates that the 96-bp sequence of MPr1197 (SEQ.ID12) constitutes the minimum promoter sequence of the HMWG-Dx5 promoter (SEQ.ID01), which is capable of directing basic transcriptional activity in the maize albumen. The absence of blue spots on the albumens bombarded with the pMPRT1144 construct, which lacks promoter sequence (negative control), confirms the functionality of the promoters grouped together in this category.
- The albumens bombarded with the pMRT1128, pMRT1213, pMRT1216, pMRT1217, pMRT1136, pMRT1137, pMRT1135 and pMRT1138 constructs exhibits on average a number of blue dots which is equivalent to the albumens transformed with the pMRT1125 (PrHMWG-Dx5 (SEQ.ID01)) and pMRT1218 (Prg-zein, positive control) constructs.
- Finally, the albumens bombarded with the pMRT1130, pMRT1131, pMRT1139 and pMRT1200 constructs exhibit an intensive and diffuse blue staining which makes counting the number of blue dots difficult, but which leads to the suggestion that the MPr1130 (SEQ.ID05), MPr1131 (SEQ.ID06), MPr1139 (SEQ.ID11) and MPr1200 (SEQ.ID15) promoters are very highly active in the maize albumen 12 days after pollination.

### 6.4.3. Quantitative evaluation of the activity of the promoters in the tobacco leaves.

The results of the histochemical assays carried out on the tobacco leaves transformed with the pMRT1125 (PrHMWG-Dx5 (SEQ.ID01)), pMRT1130, pMRT1131, pMRT1133, pMRT1134, pMRT1135, pMRT1136, pMRT1137, pMRT1138 and pMRT1092 (CaMV PrD35S, positive control) constructs given in Figure 5 made it possible to classify the promoters studied in four categories. No blue spot was observed on the leaves bombarded with the pMRT1125 (PrHMWG-Dx5 (SEQ.ID01)) construct. The leaves bombarded with the pMRT1133, pMRT1134, pMRT1136 and pMRT1137 constructs exhibit on average a number of blue dots which is between 50 and 100. The leaves transformed with the pMRT1135, pMRT1138 and pMRT1139 constructs (result not shown) exhibit a considerable number of blue dots, which is equivalent to that observed on the leaves bombarded with the reference construct pMRT1092 (CaMV PrD35S). Finally, the leaves bombarded with the pMRT1130 and pMRT1131 constructs exhibit a much higher number of diffuse and intense blue spots than the leaves bombarded by the reference construct pMRT1092 (CaMV PrD35S).

In light of these results, several pieces of essential information can be derived:
- the CaMV 35S as-1 and as-2 activating sequences deregulate the activity of the HMWG-Dx5 promoter (SEQ.ID01) in the tobacco leaves,
- the CaMV 35S as-1 and as-2 activating sequences act synergistically with cis-regulating motifs present in the HMWG-Dx5 promoter (SEQ.ID01). The "G"-like box appears to be one of the key elements of this combinatorial control. The GATA boxes, in combination with the "G"-like box and the CaMV 35S as-1 and as-2 activating sequences, are perhaps responsible for the very high activity of MPr1130 (SEQ.ID05) and MPr1131 (SEQ.ID06),
- duplicating the CaMV 35S as-2 activating sequence does not confer a notable positive additional effect,
- the "cereal boxes", in combination with the CaMV 35S as-1 and as-2 activating sequences, appear to confer a negative transcriptional effect in the tobacco leaves.

In conclusion, since the CaMV D35S promoter is commonly reported in the literature as being a chimeric promoter which provides an increase in the promoter activity of the GUS reporter gene which is about 8 to 12 times greater than the one provided by the CaMV 35S promoter (Kay et al., 1987), the MPr1135 (SEQ.ID07), MPr1138 (SEQ.ID10), MPr1139 (SEQ.ID11), MPr1130 (SEQ.ID05) and MPr1131 (SEQ.ID06) promoters are certainly the strongest chimeric promoters active in tobacco leaves described to date.

The MPr1133 (SEQ.ID35), MPrl134 (SEQ.ID08) , MPr1136 (SEQ.ID08) and MPr1137 (SEQ.ID09) promoters, whose activity is weaker in tobacco leaves, also have an advantage, since they can direct the expression of resistance genes in order to allow the selection of transgenic plants, in the same way as the "nos"-type promoters for example.

### 6.5. Quantification of the activity of the various promoters in the maize albumen by luminometric assay of B-glucuronidase expression.

The albumens previously transformed by biolistics were frozen in liquid nitrogen and ground with the aid of a glass rod mounted on a drill. The powder was then thawed in extraction buffer (25 mM Tris Phosphate, pH 7.8, 2 mM dithiothreitol, 2 mM 1,2-diaminocyclohexane, N,N,N',N'-tetracetic acid, 10% glycerol, 1% Triton X100) in a proportion of 1 ml of buffer per 250 mg of tissue. The mixture was homogenized and then incubated for 1h at 4°C, before being clarified by centrifugation for 5 min at 16060 g.

The GUS activity was measured on 10 µl of clarified crude extract, with the aid of the "GUS-Light chemiluminescent reporter gene assay" detection kit (Tropix Inc., Bedford, USA) according to the manufacturer's recommendations. Measurement of light emission was carried out using a Lumat LB 9507 luminometer (EGG-Berthold, Bad Wildbad, Germany).

In parallel, the luciferase activity was measured on 10 µl of clarified crude extract, with the aid of the "Luciferase assay system" detection kit (Promega Corp., Madison, USA) according to the manufacturer's recommendations. Measurement of light emission was carried out with the aid of the Lumat LB 9507 luminometer placed in a cold room at 4°C.

The results are given in Figs. 6 and 7. For each assay (three half albumens = one crude extract), the ratio between the β-glucuronidase activity and the luciferase activity measured with the luminometer was calculated. The mean of at least 5 independent assays per construct and the standard error of the mean were determined.

In order to analyse the effect of the various modifications brought to each of the promoters described in this patent, said promoters were divided up into two distinct groups. Group I (Fig. 6) consists of the promoters which make it possible to carry out a detailed functional dissection of the HMWG-Dx5 promoter (SEQ.ID01), and Group II (Fig. 7) contains the promoters which make it possible to determine the effect of the diverse *cis*-activating elements studied in this patent, in combination with the HMWG-Dx5 promoter (SEQ.ID01).

Group I contains the MPr1128, MPr1127 (SEQ.ID03), MPr1126 (SEQ.ID02), MPr1197 (SEQ.ID12), MPr1198 (SEQ.ID13), MPr1216 (SEQ.ID17) and MPr1217 (SEQ.ID37) promoters, the HMWG-Dx5 reference promoter (SEQ.ID01) and the reference construct pMRT1144 (negative control), this construct lacking promoter sequence (Fig. 6). The results of the luminometric assays make it possible to derive several observations:
- the gradual deletions of the 5' region of the HMWG-Dx5 promoter (SEQ.ID01) leads to a gradual decrease in the mean relative activity conferred by these promoters. The whole 417-bp PrHMWG-Dx5 promoter sequence (SEQ.ID01) thus appears to be required in order to allow maximum activity of the HMWG-Dx5 promoter (SEQ.ID01).
- The slight difference in activity recorded between the MPR1128 (SEQ.ID04) and PrHMWG-Dx5 (SEQ.ID01) promoters shows that the sequence located upstream of nucleotide -238 does not contain the major cis-activating elements which are responsible for the activity of the HMWG-Dx5 promoter (SEQ.ID01).
- The significant decrease in activity between the MPR1128 (SEQ.ID04) and MPr1127 (SEQ.ID03) promoters leads to the suggestion that the sequence located upstream of nucleotide -205, which contains a "G"-like box, plays a role which is capital for the activity of the HMWG-Dx5 promoter (SEQ.ID01).
- The slight difference in activity recorded between the MPr1127 (SEQ.ID03) and MPr1126 (SEQ.ID02) promoters does not allow a conclusion to be drawn regarding the real role of the enhancer element.
- The activity of the MPr1197 (SEQ.ID12) promoter, which is very weak but stronger than that obtained with the pMRT1144 construct which does not contain a promoter, indicates that the 96-bp minimum PrHMWG-Dx5 (SEQ.ID01) sequence confers a basic transcription level.
- The weak activity of MPr1198 (SEQ.ID13), compared with that of MPR1128 (SEQ.ID04), indicates that the PrHMWG-Dx5 (SEQ.ID01) sequence stretching from nucleotide -59 to nucleotide
- 135, although lacking putative *cis*-activating elements, plays a predominant role in the activity of the HMWG-Dx5 promoter (SEQ.ID01). This result implies that the functionality and, consequently, the accessibility of the trans-activating factors on the "G" box and on the activating element, depend on the distance which separates them from the TATA box.
- Duplicating the MPR1128 (SEQ.ID04) sequence stretching from nucleotide -136 to nucleotide -225, which harbours the "G" box and the activating element, confers on the MPr1216 promoter (SEQ.ID17) b-glucuronidase activity which is at least as high as that directed by PrHMWG-Dx5 (SEQ.ID01). Conversely, triplicating this same sequence in the MPr1217 promoter (SEQ.ID37) has no additional additive effect.

Group II contains the MPr1128, MPr1213 (SEQ.ID 16) MPr1199 (SEQ.ID14), MPr1136 (SEQ.ID08), MPr1137 (SEQ.ID09), MPr1138 (SEQ.ID10), MPr1131 (SEQ.ID06), MPr1135 (SEQ.ID09), MPr1130 (SEQ.ID05), MPr1139 (SEQ.ID11) and MPr1200 (SEQ.ID15) promoters, the HMWG-Dx5 reference promoter (SEQ.ID01) and the g-zein promoter which is used as a positive control (Fig. 7). In the same way as for the promoters of group I, the results of the luminometric assays make it possible to derive several observations:
- Fusing the CaMV 35S as-2 (Lam and Chua, 1989) and as-1 (Lam et al., 1989) activating sequences at position -65 bp of the HMWG-Dx5 promoter (SEQ.ID01) and of the HMWG-Dx5 promoter (SEQ.ID01) derivatives very greatly potentiates the activity of the resulting MPr1130 (SEQ.ID05), MPr1131 (SEQ.ID06), MPr1136 (SEQ.ID08) MPr1137 (SEQ.ID09), MPr1135 (SEQ.ID09) and MPr1138 (SEQ.ID10) promoters. By way of indication, the MPr1131 (SEQ.ID06) and MPr1130 (SEQ.ID05) promoters are, respectively, 3.2 and 3.8 times more active than the HMWG-Dx5 promoter (SEQ.ID01).
- The CaMV 35S as-2 and as-1 activating sequences act synergistically with the cis-activating elements of the HMWG-Dx5 promoter (SEQ.ID01). The gradual decrease in the activity of the MPr1131 (SEQ.ID06), MPr1138 (SEQ.ID10), MPr1137 (SEQ.ID09) and MPr1136 (SEQ.ID08) promoters, which coincides, respectively, with the gradual 5' deletions of the HMWG-Dx5 promoter (SEQ.ID01), confirm this.
- The comparison of the MPr1130 (SEQ.ID05) and MPr1135 (SEQ.ID07) promoters with the MPr1131 (SEQ.ID06) and MPr1138 (SEQ.ID10) promoters, respectively, indicates that duplicating the CaMV 35S as-2 activating sequence does not engender a significant additive activating effect.
- Fusing the "cereal boxes" of the promoter of the high molecular weight glutenin gene encoding the Bx7 subunit of the hexaploid wheat *Triticum aestivum L.cv Cheyenne* (Anderson et al., 1998), upstream of the MPr1131 (SEQ.ID06) and MPr1138 (SEQ.ID10) promoters very greatly improves the activity of the resulting MPr1139 (SEQ.ID11) and MPr1200 (SEQ.ID15) promoters. By way of indication, the activity of MPr1139 (SEQ. ID13) and of MPr1200 (SEQ. ID19) is approximately 5.5 times higher than that of the HMWG-Dx5 promoter (SEQ. ID01).
- The weak activity of the MPr1213 promoter (SEQ.ID16) which corresponds to the fusion of the "cereal boxes" upstream of the MPr1128 promoter, implies that the "cereal boxes" act synergistically with the CaMV 35S as-2 and as-1 activating sequences so as to potentiate the activity of the MPr1139 (SEQ.ID11) and MPr1200 (SEQ.ID15) promoters.
- Finally, fusing the "GC-rich" element of the intergenic region of the maize streak virus (MSV) upstream of the MPR1128 (SEQ.ID04) promoter does not contribute to increasing the activity of the resulting MPr1199 promoter (SEQ.ID14). On the contrary, the "GC-rich" element appears to confer a slightly inhibitory effect.

In conclusion, the MPr1139 (SEQ.ID1) and MPr1200 (SEQ.ID15) promoters, since they are, respectively, 4 and 3.9 times more active than the Prg-zein promoter, which is commonly used in plant biotechnology to direct protein expression at high levels, are unquestionably powerful tools capable of improving the level of expression of heterologous proteins in the maize albumen. Moreover, it is to be noted that the MPr1130 (SEQ.ID05), MPr1131 (SEQ. ID06), MPr1135 (SEQ.ID07), MPr1138 (SEQ.ID10) , MPr1137 (SEQ.ID09) and MPr1136 (SEQ. ID08) promoters confer b-glucuronidase activity in the maize albumen which is at least as great as that obtained with the Prg-zein promoter. Finally, the less effective promoters are also very valuable, since they can be used to provide the control of the expression of resistance genes or of genes encoding enzymatic proteins.

### Example 7.

### Expression and evaluations of the activity of the various promoters in stable expression in maize and tobacco.

### 7.1. Stable gene transformation of maize with Agrobacterium tumefaciens.

The technique used is described by Ishida et al. (1996). Immature embryos 1.0 to 1.2 mm in length (9 to 14 days after pollination) were washed in LS-inf medium, then immersed in the agrobacteria suspension, prepared as described by Ishida et al. (1996), vortexed for 30 sec., and incubated at room temperature for 5 min. The immature embryos thus treated were cultivated on LS-AS medium in the dark at 25°C for 3 days, then transferred onto LSD 1.5 medium supplemented with phosphinotricine at 5 mg/l and cefotaxime at 250 mg/l, in the dark at 25°C for 2 weeks and, finally, placed on LSD 1.5 medium supplemented with phosphinotricine at 10 mg/l and cefotaxime at 250 mg/l, in the dark at 25°C for 3 weeks. The type I calluses thus generated were isolated, fragmented and transferred onto LSD 1.5 medium supplemented with phosphinotricine at 10 mg/l and cefotaxime at 250 mg/l, in the dark at 25°C for 3 weeks. Then, the type I calluses, which had proliferated, were isolated and placed on LSZ medium supplemented with phosphinotricine at 5 mg/l and cefotaxime at 250 mg/l, under a 16 hours light/8 hours darkness photoperiod at 25°C for 2 to 3 weeks. The regenerated plantlets were then transferred onto LSF 1/2 medium under a 16 hours light/8 hours darkness photoperiod at 25°C for 1 to 2 weeks, and then to a phytotron and to a greenhouse.

### 7.2. Stable gene transformation of tobacco with Agrobacterium tumefaciens.

The transformation of the tobacco *(Nicotiana tabacum L.,* of the PBD6 cultivar) was carried out by infecting foliar discs isolated from 6-week-old tobacco plantlets in vitro, with recombinant agrobacteria according to the method described by Horsch et al. (1985).

All the in vitro cultures are prepared in an air-conditioned area in which the light intensity is 200 µE.m-2.s-1, the photoperiod is 16 hours light/8 hours darkness, and the temperature is 25°C.

Except for the initial coculturing step, the regeneration, development and rooting steps were carried out on diverse selective media supplemented with a bacteriostatic agent, namely augmentin at 400 mg/l, and with a selective agent, namely kanamycin at 200 or 100 mg/l.

The various steps and media used are as follows:
- After preculturing the agrobacteria in 5 ml of 2YT medium (10 g/l bactotryptone, 5 g/l yeast extract, 5 g/l NaCl, pH 7.2) supplemented with 6 mM final CaCl₂ and with suitable antibiotics, at 28°C for 48 hours, a culture in 10 ml of 2YT medium supplemented with CaCl2 and antibiotics is prepared at 28°C overnight. The culture is then centrifuged at 3000 g for 10 min and the bacteria are resuspended in 10 ml of liquid MS30 (4.4 g/l M0404 sold by SIGMA, supplemented with 30 g/l sucrose, pH 5.7).
- The coculturing is carried out by placing the approximately 1 cm² foliar explants, which have been cut out from in vitro plantlet leaves, in contact with the agrobacteria suspension diluted 10-fold in liquid MS30, for 20 min. Then, the explants thus treated are rapidly dried on filter paper and placed on a solid coculture medium (CM) (MS30, benzylaminopurine (BAP) at 1 mg/l, indole-3-acetic acid (IAA) at 0.1 mg/l, agar-agar at 8 g/l) for 48 hours in the air-conditioned area.
- The treated explants are then placed on a solid regeneration medium (solid CM, augmentin at 400 mg/l, kanamycin at 200 mg/l) . The explants are subcultured on the same medium after 2 weeks.
- After 2 weeks, the buds are subcultured on a solid development medium (4.4 g/l M0404 sold by SIGMA, supplemented with 20 g/l sucrose, pH 5.7 (liquid MS20), augmentin at 400 mg/l, kanamycin at 100 mg/l, agar-agar at 8 g/l).
- After 2 weeks, the transformed plantlets are subcultured on solid rooting medium which is identical to the development medium. The rooting lasts 2 to 3 weeks, at the end of which the plantlets are removed to the phytotron in jiffy pots for 10 days (16 hours light/8 hours darkness photoperiod, 23°C and 70% humidity), and then transferred to a greenhouse.

### 7.3. Measurement of β-glucuronidase activity in the maize and tobacco plants.

To measure the β-glucuronidase activity, the samples taken from the transgenic plants were frozen in liquid nitrogen and ground with the aid of a glass rod mounted on a drill. The powder was then resuspended in extraction buffer (25 mM Tris Phosphate, pH 7.8, 2 mM dithiothreitol, 2 mM 1,2-diaminocyclohexane, N,N,N',N'-tetracetic acid, 10% glycerol, 1% Triton X100) in a proportion of 1 ml of buffer per 250 mg of tissue. The mixture was homogenized and then incubated for 1h at 4°C, before being clarified by centrifugation for 5 min at 16060 g.

The GUS activity was measured on 10 µl of clarified crude extract, with the aid of the "GUS-Light chemiluminescent reporter gene assay" detection kit (Tropix Inc., Bedford, USA) according to the manufacturer's recommendations. Measurement of light emission was carried out using a Lumat LB 9507 luminometer (EGG-Berthold, Bad Wildbad, Germany).

The amount of total protein present in the crude extract was measured according to the Bradford technique (1976), using the "Bio-Rad protein assay" reagent (Bio-Rad, Munich, Germany).

### 7.4. Stable Expression and Chimeric Promoter Activity in Maize Endosperm and Leaves.

### 7.4.1. Expression in Seeds.

The β-glucuronidase activity controlled by the chimeric HMWG promoters in stable expression in maize endosperm was compared to controlled by the reference promoter 512 gamma-zein, which is known to be highly active in maize albumen (Marzabal et al., 1998). Six seeds per cob were studied, taken starting from the apex of the cob and proceeding towards its base, at different stages of growth. As an indication, the 30 DAP stage corresponds to maize seeds taken 30 days after pollinisation. The luminometric amounts of β-glucuronidase activity were determined for each seed according to the method described in section 7.3 of example 7.

The results as reported in Figure 8 refelect the β-glucuronidase activity under the control of the chimeric HMWG-Dx5 derived promoters (MPr1139, MPr1200 and MPr1131) and the reference promoter 512 gamma-zein, during stable expression in mature corn seeds, harvested at 30 DAP. The comparison of the activities of each population of plants gives a good indication of the respective strength of the different promoters. The β-glucuronidase activity under the control of promoters MPr1139, MPr1200 andn MPr1131 is on average of the order of 1.5 to 2 times as great as that under the control of the reference promoter 512 gamma-zein. Nonetheless, the seeds of plants 302.A3 and 347.H1, which respectively express the GUS protein under the control of the promoters MPr1139 and MPr1131, show a β-glucuronidase activity of 7 and 14 times respectively that of the activity controlled by the reference promoter 512 gamma-zein. No significant difference in β-glucuronidase activity was noted in plants expressing the GUS protein under the control of promoters MPr1139, MPr1200 et MPr1131. However, β-glucuronidase activity varies considerably in each population of plants. This phenomena, which has already been observed in the majority of genes introduced into plants, can be explained by positioning effects of the transgene and copy number. The luminometric determinations carried out at the 13 and 18 DAP stages of development (results not shown) indicate that the β-glucuronidase activity varies over time, but that the promoters responsible for the highest β-glucuronidase activity at the 30 DAP stage are also the strongest promoters in the earlier stages of development, at 13 and 18 DAP respectively. Thus, the classification of the promoters is maintained during development. The histogram shown in Figure 9 shows temporal fluctuations in β-glucuronidase activity under the control of the promoter MPr1139. These results indicate the the GUS activity is detectable from 10 DAP, reaches a plateau between 16 and 28 DAP and declines thereafter up to 30 DAP. The GUS activity plateau, obtained from plants taken during the summer period, was also observed for the period of development between 12 and 20 DAP (results not shown). The histochemical tests carried out on longitudinal sections of corn seeds taken at 13 DAP (Figure 10a), 18 DAP (Figure 10b) or on dissected maize seeds (Figure 10c) indicate that the promoter MPr1139, in maize seed, is specifically expressed in the albumen, no staining having been detected in the embryo, the aleurone or the pericarp. Furthermore, the histograms illustrated in Figure 11 indicate that the expression of MPr1139 is stable, or even greater in the second generation (T2).

From the preceding data, the following information can be summarized :
- the activating sequences as-1 and as-2 of the CaMV 35S promoter, fused to the promoter sequence HMWG-Dx5, appear to be very strong *cis-*activating elements in maize albumen.
- the activating sequences as-1 and as-2 of the CaMV 35S promoter do not deregulate the activity of the HMWG-Dx5 promoter in maize seeds (Figure 10).
- the cereal boxes do not have a significant cis-regulatory effect in stable expression in corn seeds, the promoter MPr1131 being at least as active as the promoter MPr1139.
- the promoter sequence HMWG-Dx5 located upstream of the "G" box, stretching from nucleotides -238 to -378pb, does not play a a key regulatory role within the the chimeric promoters derived from HMWG, the promoter MPr1200 being at least as active as the promoter MPr1139 in stable expression of maize seeds.

In conclusion, the chimeric promoters derived from HMWG (MPr1139 MPr1131 and MPr1200), being on average roughly 1.5 to 2 times as active or even greater for the best expressors 302.A3 and 347.H1, as the reference promoter 512 gamma-zein, are incontestably exceedingly useful tools capable of improving the expression of heterologous proteins in maize albumen. The chimeric promoters derived from HMWG according to the present invention can also be used to over-express endogenous proteins in monocotyledonous plant seeds, thereby representing an interest for agriculture for example, for the production of rice or wheat, in relation to starch production.

### 7.4.2 Stable Expression in Leaves.

The β-glucuronidase activity under the control of promoters MPr1139, MPr1131 and MPr1200 was determined by stable expression in maize leaves. The luminometric determinations of β-glucuronidase activity were carried out according to the method described in section 7.3 of example 7 from two leaf disks, each two centimeters in diameter, taken at 3 weeks after acclimatisation in a greenhouse from maize plants.

The comparison of the activities for each population of plants indicates that the β-glucuronidase activity controlled by the chimeric promoters MPr1139, MPr1200 and MPr1131 is at the most 30 times greater than the background noise measured in plants expressing the GUS protein under the control of the 512 gamma-zein promoter (Figure 12), with the result that the promoters MPr1139, MPr1200 and MPr1131 are slightly or not at all active in the leaves of maize plants at the three week development stage after acclimatization in a greenhouse. Nevertheless, the histochemical tests carried out on the leaves of the primary transformants (plantlets) expressing the GUS protein under the control of the chimeric promoters derived from HMWG, during rooting in in vitro cultivation, systematically reveals a blue staining (results not shown).

These results are very interesting in that the activity of the promoters MPr1139, MPr1200 and MPr1131 is low but sufficient for carrying out early tests in the leaves of transgenic maize, without any major risks of toxicity.

### 7.5. Stable Expression Activity of Chimeric Promoters in Tobacco Leaves and Seeds.

### 7.5.1. Stable Expression in Leaves.

The stable expression β-glucuronidase activity under control of the promoters MPr1130, MPr1131, MPr1135 MPr1138 and MPr1139 was compared to that controlled by the CaMV D35S promoter, in tobacco leaves. The luminometric measurements of the β-glucuronidase activity were carried out according to the method described in section 7.3 of example 7 from two leaf disks each two centimeters in diameter, taken from different leaves located at the base of the upper third of the primary transformants, at the 2, 5, 8 and 11 week stages of development after acclimatization in a greenhouse. In order to limit the variations in the degree of expression of the reporter gene, introduced mainly by random integration and the number of copies of the expression cassette, 10 to 30 independent transformants were studied for each construction.

The results illustrated in Figure 13 reflect the stable expression β-glucuronidase activity under the control of the chimeric promoters derived from HMWG and the reference promoters HMWG-Dx5 and CaMV D35S in tobacco leaves, 11 weeks after acclimatization in a greenhouse. The comparison of the activities of each plant population provides a good indication of the respective strength of the different promoters. The β-glucuronidase activity controlled by the chimeric promoters of the present invention derived from HMWG is significantly greater than that measured under the control of the HMWG-Dx5 promoter, but roughly 5 to 10 times lower than that under the control of the CaMV D35S promoter. Amongst the different HMWG chimeric promoters, no significant difference in activity was observed, except for the promoter Mpr1139, which was slightly less active. The luminometric determinations made at the 2, 5 and 8 week development stage after acclimatization in a greenhouse (results not shown) indicate that the β-glucuronidase activity increases over time in any given plant, irrespective of the promoter used. However, the strongest promoters at the 11 week development stage also confer the highest β-glucuronidase activity at earlier stages of development (2, 5 and 8 weeks after acclimatization in a greenhouse). Thus, the classification of the promoters at the 11 week stage also applies to all the other stages of development in tobacco.

It is apparent from this data that the chimeric promoters derived from HMWG are functional but only weakly active in stable expression in tobacco leaves. The applicant concludes that the activating sequences as-1 and as-2 deregulate the activity of the HMWG-Dx5 promoters in tobacco leaves, but do not confer a strong activating effect in association with the cis-regulatory elements present in the HMWG-Dx5 promoter sequence.

In order to explain this apparent contradiction in these results with those obtained in transient expression experiments, two hypotheses were raised :
- the chimeric promoters derived from HMWG are induced by injury and/or stress during biolistic transformation of tobacco leaves ;
- in stable tobacco expression, the chimeric promoters derived from HMWG are essentially expressed in the every early stages of development. The histochemical tests carried out on the leaves of the primary tobacco transformants *in vitro* (plantlets) during the regeneration step indicate high β-glucuronidase activity of the chimeric promoters derived from HMWG (results not shown).

The chimeric promoters derived from HMWG, i.e. MPr1130, MPr1131, MPr1135, MPr1138 and MPr1139, although weakly active in stable expression of tobacco leaves, can be used for example for controlling the expression of an enzyme implicated in a biosynthetic pathway of the metabolism of the plant. They can also be used to control the expression of genes conferring a resistance to the plant, for example, a resistance to an antibiotic or a herbicide, useful as a selection agent.

### 7.5.2. Stable Expression in Tobacco Seeds.

The β-glucuronidase activity was determined by luminometry in mature T1 tobacco seeds (100 mg per transformant) taken from 10 to 30 separate primary transformants obtained independently for each construction. The activity varies from plant to plant within a given construct (cf. Figure 14), which can be explained by positioning effects and the transgene copy number in the genome.

The results show that certain chimeric promoters derived from HMWG can promote β-glucuronidase activity in tobacco seeds at least as highly as the CaMV D35S promoter. Indeed, the promoters can be classified as follows:
- the promoters MPr1130, MPr1131 and Mpr1139 which promote β-glucuronidase activity to the same extent as the CaMV D35S promoter ;
- the promoters MPr113 and MPr1138, which are twice as active in tobacco seeds as the CaMV D35S promoter.

The results obtained indicate that :
- the activating sequences as-1 and as-2 of the CaMV 35S promoter confer an important cis-activating effect in a promoter sequence derived from HMWG containing only a "G -like" box and the "enhancer" element, as observed for promoters MPr1135 and MPr1138. Thus, the activating sequences as-1 and as-2 of the CaMV 35S promoter act in synergy with the cis-regulatory elements present in the HMWG-Dx5 promoter. The "G-like" box and the "enhancer" element seem to be the key elements in this combinatory control in tobacco seeds.
- the HMWG-Dx5 promoter sequence located upstream of the "G" box, stretching from nucleotides -378 to -238, confers a negative cis-regulatory effect to the promoters MPr1130, MPr1131 and MPr1139 in tobacco seeds.
- the duplication of the activating sequence as-2 of the CaMV promoter does not confer any notable positive additive effect in tobacco seeds. Indeed, no difference in activity is obtained for MPr1130 with respect to Mpr1131, nor for MPr1135 with respect ot MPr1138.
- the "cereal" boxes do not confer any notable additive cis-activating effect in tobacco seeds, since the results obtained for the promoters MPr1131 and MPr1139 are similar.
   the promoters MPr1135 and MPr1138, which are highly active in stable expression in tobacco seeds, are powerful tools for controlling the expression of heterologous proteins in dicotyledonous plants, for example, such as in those plants of high agronomic interest.

### Example 8.

### Construction of the binary plasmid pMRT1231 including the HMWG-Dx5 promoter (SEQ.IDO1), used for stable transformation of tobacco.

The binary plasmid pMRT1231 was obtained by insertion of the expression cassette "HMWG-Dx5 (SEQ.ID01) / *uidA-*IV2 / term-nos" from pMRT1125 into the resriction site HpaI from the binary plasmid pMRT1195. This is described in French patent application FR9911112, to be published, incorporated herein by reference with respect to the relevant passages.

In order to do this, 7 µg of plasmid pMRT1125 were digested successively by EcoRI and XmnI for 1 h at 37°C. The expression cassette was then isolated on 0.7% agarose gel using a "Concert Rapid Gel Extraction System" kit and subjected to the action of 20 Units of Klenow fragment (New England Biolabs) for 30 min. at 37°C in the presence of 60 nanomoles of each of the dNTPs, 12 µl of MgC12 (500 mM) and 6 µl of DTT (1M).

In parallel, 5 µg of binary plasmid pMRT1195 were digested by HpaI for 1 h at 37°C. The linearized vector frgament was then dephosphorylated by 40 Units of calf intestine alkaline phosphatase (New England Biolabs) in the presence of 3 buffer at 37°C for 1h.

The ligation was carried out with 100 ng of the expression cassette and 10 ng of pMRT1195 plasmid as obtained above, by a succession of PCR cycles in a "GeneAmp PCR System 9700" thermocycler as described previously. Previously prepared competent *Escherichia coli* DH5α, were transformed with all of the ligation reaction mixture. The plasmid DNA of the obtained clones, selected on LB media supplemented with kanamycine (50 mg/l), was extracted according to the alkaline lysis method and analyzed by enzymatic digestion.

The plamsid obtained, designated pMRT1231, was then transferred as described previously in section 4.1 of example 4, into the strain *Agrobacterium tumefaciens* LBA4404-pSB1, which strain derives from *Agrobacterium tumefaciens* LBA4404 after integration of the pSB1 plasmid according to the protocol described recently for obtaining the strain A1177. This is described in French patent application FR9911112, to be published, incorporated herein by reference for the relevant passages. The plasmid DNA of the obtained clones, selected on 2YT media supplemented with rifampicine (50 mg/l), Kanamycine (50 mg/l) and tetracycline (5 mg/l), was extracted according to the alkaline lysis method, modified by adding lysozyme (25 mg/ml) to the cell resuspension buffer. The plasmid DNA obtained was analyzed by enzymatic digestion and the agrobacteria obtained designated A1231.

The stable genetic transformation of tobacco was carried out as described in section 7.2 of example 7 except that the selection agent used in the regeneration and development media is glufosinate at 0,5 and 2mg/l respectively.

### Example 9.

### Construction of the binary plasmids including the promoters MPr1131, MPr1200 and 512 gamma-zein, used for stable genetic transformation of maize.

### 9.1. Construction of binary plasmid pMRT1263.

The binary plasmid pMRT1263 was obtained by insertion of the expression cassette "MPr1131 (SEQ. ID06) / *uidA-*IV2 / term-*nos*" into the restriction site HpaI of the binary plasmid pMRT1195. This plasmid is described in French patent application FR9911112, not yet published, and incorporated herein by reference for the relevant passages. The insertion was carried out in example 5, except that the expression cassette was isolated from plasmid pMRT1131, described in section 3.2 of example 3.

The resulting plasmid was designated pMRT1263 and was transferred into the strain *Agrobacterium tumefaciens* LBA4404-pSB1 according to the protocol described previously in section 5.1 of example 5. The agrobacteria clone obtained was designated A1263.

### 9.2. Construction of the binary plasmid pMRT1266.

The binary plasmid pMRT1266 was obtained by insertion of the expression cassette "MPr1200 (SEQ.ID15) / uidA-IV2 / term-nos" into the HpaI restriction site of binary plasmid pMRT1195. This is described in French patent application FR9911112, not yet published, the text of the relevant passages of which is hereby incorporated by reference. The insertion was carried out as described in example 5, except that the expression cassette was isolated from plamsid pMRT1200, described in section 3.10 of example 3.

The resulting plasmid was designated pMRT1266 and was transferred into the strain *Agrobacterium tumefaciens* LBA4404-pSB1 according to the protocol described previously in section 5.1 of example 5. The agrobacteria clone obtained was designated A1266.

### 9.3. Construction of binary plasmid pMRT1209.

In order to have a reference promoter sequence in stable expression in maize albumen SN 87 165 (L2), the *uidA* gene under the control of the promoter 512 gamma-zein and the nos terminator, contained in the 526 gamma-zein plasmids described by Marzabal et al. (1998), was inserted into binary plasmid pMRT1195, as described previously in example 5, except that the expression cassette was isolated from 526 gamma-zein plasmid.

The resulting plasmid pMRT1209 was transferred into the stain *Agrobacterium tumefaciens* LBA4404-pSB1 according to the protocol in section 5.1 of example 5. The agrobacteria clone obtained was designated A1209.

### References cited

Anderson O.D., Green F.C., Yip R.E., Halford N.G., Shewry P.R. and Malpica-Romero J-M. (1989). Nucleotide sequences of the two high-molecular-weight glutenin genes from the D-genome of a hexaploid bread wheat, *Triticum aestivum* L. cv Cheyenne. Nucleic Acids Research 17, 461-462.
Anderson O.D., Abraham-Pierce F.A. and Tam A. (1998). Conservation in wheat high-molecular-weight glutenin gene promoter sequences: comparisons among loci and among alleles of the *GLU-B1-1* locus. Theor Appl Genet 96, 568-576.
Birnboim H. C. and Doly J. (1979). A rapid alkaline extraction procedure for screening recombinant plasmid DNA. Nuc. Ac. Res. 7, 1513.
Bradford M. (1976). A rapid and sensitive method for the detection of microgram quantities of proteins utilizing the principle of protein-dye binding. Anal. Biochem. 72, 248-254. Fenoll C., Schwarz J.J., Black D.M., Schneider M. and Howell S.H. (1990). The intergenic region of maize streak virus contains a GC-rich element that activates rightward transcription and binds maize nuclear factors. PMB 15, 865-877. Guérineau and Mullineaux (1993). In Plant molecular biology labfax, Croy R.R.D. (Ed.), BioS Scientific Publishers, Blackwell Scientific Publications.
Jefferson R.A., Burgess S.M. and Hirsh D (1986). β-glucuronidase as a gene-fusion marker. Proc. Nat. Acad. Sci. USA, 83, 8447-8451.
Jefferson R.A., Kavanagh T.A. and Bevan M.W. (1987). GUS fusions: β-glucuronidase as a sensitive and versatile gene fusion marker in higher plants. EMBO J. 6, 3901-3907.
Holsters M., Dewaele D., Depicker A., Messenf E., Van Montagu M. and Schell J. (1978). Transfection and transformation of *Agrobacterium tumefaciens.* Mol. Gen. Genet. 136, 181-187. Horsch R.B., Fry J.E., Hoffmann N.L., Eiholtz D., Rogers S.G. and Fraley R.T. (1985). A simple and general method for transferring genes into plants. Science 227, 129-1231.
Kay R., Chan A., Daly M. and McPherson J. (1987). Duplication of CaMV 35S promoter sequences creates a strong enhancer for plant genes. Science 236, 1299-1302.
Lam E., Benfey P. N., Gilmartin P. M., Fang R. X. and Chua N. H. (1989). Site-specific mutations alter in vitro factor binding and change promoter expression pattern in transgenic plants. Proc. Natl. Acad. Sci. USA, 86, 7890-7894.
Lam E. and Chua N. H. (1989). ASF-2 : a factor that binds to the cauliflower mosaic virus 35S promoter and a conserved GATA motif in Cab promoters. Plant Cell, 1, 1147-1156.
Leckie L., Devoto A. and De Lorenzo G. (1994). Normalisation of GUS by LUC activity from the same cell extract reduces transformation variability. Biotechniques 17, 52-56.
Murashige T. and Skoog F. (1962). A revised medium for rapid growth and bioassays with tobacco tissue cultures. Physiol. Plant. 15, 473-497.
Reina M., Ponte I., Guillén P., Boronat A. and Palau J. Sequence analysis of a genomic clone encoding a Zc2 protein from *Zea mays* W64 A. Nucleic Acids Research 18, 6426.
Torrent M., Alvarez I., Geli M.I., Dalcol I. and Ludevid D. (1997). Lysine-rich modified g-zein accumulate in protein bodies of transiently transformed maize endosperms. Plant Mol. Biol. 34, 139-149.
Vancanneyt G., Schmidt R., 0'Connor-Sanchez A., Willmitzer L. and Rocha-Sosa M. (1990). Construction of an intron-containing marker gene: Splicing of the intron in transgenic plants and its use in monitoring early events in Agrobacterium-mediated plant transformation. Mol. Gen. Genet. 220, 245-250.
Marzabal P., Busk P. K., Ludevid M. D. et Torrent M. (1998). The bifactorial endosperm box of g-zein gene : characterisation and function of the Pb3 and GZM cis-acting elements. The Plant Journal 16 (1), 41-52.

## Claims

1. Chimeric promoter of expression comprising at least one nucleic acid sequence **characterised in that** the at least one nucleic acid sequence is selected from the group consisting of the sequences identified under the numbers SEQ.ID05, SEQ.ID06, SEQ.ID07, SEQ.ID09, SEQ.ID10, SEQ.ID11, SEQ.ID13, SEQ.ID15, SEQ.ID17, SEQ.ID37.

2. Expression cassette comprising a promoter according to Claim 1, which is linked in a functional manner to a nucleic acid sequence to be expressed encoding a polypeptide to be produced, itself linked to a transcription termination nucleic acid sequence.

3. Expression cassette according to Claim 2, **characterized in that** the promoter is chosen from the group consisting of the sequences identified under the numbers SEQ.ID05, SEQ.ID06, SEQ.ID07, SEQ.ID09 SEQ.ID10, SEQ.ID11, SEQ.ID13, SEQ.ID15, SEQ.ID17, SEQ.ID 37.

4. Isolated promoter nucleic acid sequence, **characterized in that** it corresponds to a sequence chosen from the group consisting of the sequences identified under the numbers SEQ.ID05, SEQ.ID06, SEQ.ID07, SEQ.ID09 SEQ.ID10, SEQ.ID11, SEQ.ID13, SEQ.ID15, SEQ.ID17, SEQ.ID 37.

5. Vector comprising a promoter or expression cassette which is capable of initiating the transcription of a nucleic acid sequence encoding a polypeptide to be produced, **characterized in that** the promoter corresponds to a promoter according to Claim 1 or an expression cassette according to any one of claim 2 to claim 3.

6. Transgenic plant which has stably integrated into its genome at least one promoter or expression cassette according to any one of Claims 1 to 4.

7. Transgenic plant according to Claim 6, **characterized in that** it is chosen from dicotyledonous species, such as potato, tobacco, cotton, lettuce, tomato, melon, cucumber, pea, rapeseed, beetroot or sunflower, or monocotyledonous species, such as wheat, barley, oat, rice or maize.

8. Propagule of a transgenic plant according to any one of Claims 6 or 7.

9. Propagule of a transgenic plant according to Claim 8, **characterized in that** it is a seed.

10. Cell containing a promoter or expression cassette according to any one of Claims 1 to 4.

11. Cell according to Claim 10, **characterized in that** it is a plant cell.

12. Method for expressing a nucleic acid sequence, or gene, encoding a polypeptide to be produced, in a cell, **characterized in that** it comprises the steps consisting in:
- transforming the cell with a vector comprising at least one promoter or expression cassette according to any one of Claims 1 to 4 ;
- preparing a culture of the cell under conditions which allow the expression of the nucleic acid sequence, or gene, encoding the polypeptide.

13. Method according to Claim 12, **characterized in that** the cell is a prokaryotic or eukaryotic cell.

14. Method according to either of Claims 12 and 13, **characterized in that** the cell is a cell chosen from the group consisting of microbial cells, fungal cells, insect cells, animal cells and plant cells.

15. Method according to any one of Claims 12 to 14, **characterized in that** the cell is a plant cell.

16. Method for obtaining a transgenic plant according to any one of Claims 6 or 7, or a propagule according to Claim 8, **characterized in that** it comprises the steps consisting in:
- transforming a plant cell with a vector comprising at least one promoter or expression cassette according to any one of Claims 1 to 4;
- selecting the plant cell which has integrated the promoter;
- propagating the transformed and selected plant cell either in culture or by regenerating chimeric or transgenic whole plants.

## Patentansprüche

1. Chimärer Promotor für die Expression, der zumindest eine Nukleinsäuresequenz aufweist, **dadurch gekennzeichnet, daß** die zumindest eine Nukleinsäuresequenz aus der Gruppe ausgewählt ist, die aus den Sequenzen besteht, die mit den Nummern SEQ.ID05, SEQ.ID06, SEQ.ID07, SEQ.ID09, SEQ.ID10, SEQ.ID11, SEQ.ID13, SEQ.ID15, SEQ.ID17, SEQ.ID37 angegeben werden.

2. Expressionskassette, die einen Promotor nach Anspruch 1 aufweist, der in funktioneller Weise mit einer zu exprimierenden auszuprägenden Nukleinsäuresequenz verbunden ist, die ein zu produzierendes Polypeptid kodiert, und selbst mit einer Transkriptionsterminations-Nukleinsäuresequenz verbunden ist.

3. Expressionskassette nach Anspruch 2, **dadurch gekennzeichnet, daß** der Promotor aus der Gruppe ausgewählt ist, die aus den Sequenzen besteht, die mit den Nummern SEQ.ID05, SEQ.ID06, SEQ.ID07, SEQ.ID09, SEQ.ID10, SEO.ID11, SEQ.ID13, SEQ.ID15, SEQ.ID17, SEQ. ID37 angegeben werden.

4. Isolierte Nukleinsäuresequenz eines Promotors, **dadurch gekennzeichnet, daß** sie einer Sequenz entspricht, die aus der Gruppe ausgewählt ist, die aus den Sequenzen besteht, die mit den Nummern SEQ.ID05, SEQ.ID06, SEQ.ID07, SEQ.ID09, SEQ.ID10, SE0.ID11, SEQ.ID13, SEQ.ID15, SEQ.ID17, SEQ.ID37 angegeben werden.

5. Vektor, der einen Promotor oder eine Expressionskassette aufweist, der bzw. die die Transkription einer Nukleinsäuresequenz einleiten kann, die ein zu produzierendes Polypeptid kodiert, **dadurch gekennzeichnet, daß** der Promotor einem Promotor nach Anspruch 1 oder einer Expressionskassette nach einem der Ansprüche 2 bis 3 entspricht.

6. Transgene Pflanze, in deren Genom zumindest ein Promotor oder eine Expressionskassette nach einem Ansprüche 1 bis 4 stabil eingebaut ist.

7. Transgene Pflanze nach Anspruch 6, **dadurch gekennzeichnet, daß** sie aus zweikeimblättrigen Spezies, wie Kartoffel, Tabak, Baumwolle, Lattich, Tomate, Melone, Gurke, Erbse, Raps, rote Rübe oder Sonnenblume, oder einkeimblättrigen Spezies, wie Weizen, Gerste, Hafer, Reis oder Mais, ausgewählt ist.

8. Vegetative Vermehrungseinheit einer transgenen Pflanze nach einem der Ansprüche 6 oder 7.

9. Vegetative Vermehrungseinheit einer transgenen Pflanze nach Anspruch 8, **dadurch gekennzeichnet, daß** sie ein Samen ist.

10. Zelle, die einen Promotor oder eine Expressionskassette nach einem der Ansprüche 1 bis 4 enthält.

11. Zelle nach Anspruch 10, **dadurch gekennzeichnet, daß** sie eine Pflanzenzelle ist.

12. Verfahren zur Expression einer Nukleinsäuresequenz oder eines Gens, die bzw. das ein zu produzierendes Polypeptid kodiert, in einer Zelle, **dadurch gekennzeichnet, daß** es die folgenden Schritte aufweist:
- Transformieren der Zelle mit einem Vector, der zumindest einen Promotor oder eine Expressionskassette nach einem der Ansprüche 1 bis 4 aufweist;
- Präparieren einer Kultur der Zelle unter Bedingungen, die die Expression der Nukleinsäuresequenz oder des Gens ermöglichen, die bzw. das das Polypeptid kodiert.

13. Verfahren nach Anspruch 12, **dadurch gekennzeichnet, daß** die Zelle eine prokaryotische oder eukaryotische Zelle ist.

14. Verfahren nach einem der Ansprüche 12 und 13, **dadurch gekennzeichnet, daß** die Zelle eine Zelle ist, die aus der Gruppe ausgewählt ist, die aus mikrobiellen Zellen, Pilzzellen, Insektenzellen, Tierzellen und Pflanzenzellen besteht.

15. Verfahren nach einem der Ansprüche 12 bis 14, **dadurch gekennzeichnet, daß** die Zelle eine Pflanzenzelle ist.

16. Verfahren für die Produktion einer transgenen Pflanze nach einem der Ansprüche 6 oder 7 oder einer vegetativen Vermehrungseinheit nach Anspruch 8, **dadurch gekennzeichnet, daß** es die folgenden Schritte aufweist:
- Transformieren einer Pflanzenzelle mit einem Vektor, der zumindest einen Promotor oder eine Expressionskassette nach einem der Ansprüche 1 bis 4 aufweist;
- Selektion der Pflanzenzelle, in die der Promotor eingebaut ist;
- Vermehren der transformierten Zelle und der ausgewählten Pflanzenzelle in einer Kultur oder durch Regenerieren von chimären oder transgenen vollständigen Pflanzen.

## Revendications

1. Promoteur d'expression chimérique comprenant au moins une séquence d'acide nucléique, **caractérisé en ce que** ladite au moins une séquence d'acide nucléique est choisie dans le groupe comprenant les séquences identifiées par le numéros SEQ ID NO : 05, SEO ID NO : 06, SEQ ID NO : 07, SEQ ID NO : 09, SEQ ID NO: 10, SEQ ID NO: 11, SEQ ID NO: 13, SEO ID NO : 15, SEQ ID NO : 17, SEQ ID NO : 37.

2. Cassette d'expression comprenant un promoteur selon la revendication 1, qui est liée d'une manière fonctionnelle à une séquence d'acide nucléique à exprimer codant pour un polypeptide à produire, lui-même lié à une séquence d'acide nucléique de terminaison de la transcription.

3. Cassette d'expression selon la revendication 2, **caractérisée en ce que** le promoteur est choisi dans le groupe comprenant les séquences identifiées par les numéros SEQ ID NO : 05, SEQ ID NO : 06, SEQ ID NO : 07, SEQ ID NO : 09, SEQ ID NO : 10, SEQ ID NO : 11, SEQ ID NO : 13, SEQ ID NO : 15, SEO ID NO : 17, SEQ ID NO : 37.

4. Séquence d'acide nucléique d'un promoteur isolé, **caractérisée en ce qu'**elle correspond à une séquence choisie dans le groupe comprenant les séquences identifiées par les numéros SEQ ID NO : 05, SEO ID NO : 06, SEQ ID NO: 07, SEQ ID NO: 09, SEQ ID NO: 10, SEQ ID NO: 11, SEQ ID NO : 13, SEQ ID NO : 15, SEQ ID NO : 17, SEQ ID NO : 37.

5. Vecteur comprenant un promoteur ou une cassette d'expression qui est capable d'initier la transcription d'une séquence d'acide nucléique codant pour un polypeptide à produire, **caractérisé en ce que** le promoteur correspond à un promoteur selon la revendication 1 ou une cassette d'expression selon l'une quelconque des revendications 2 à 3.

6. Plante transgénique qui a intégré de façon stable dans son génome au moins un promoteur ou une cassette d'expression selon l'une quelconque des revendications 1 à 4.

7. Plante transgénique selon la revendication 6, **caractérisée en ce qu'**elle est choisie parmi l'espèce dicotylédone, comme la pomme de terre, le tabac, le coton, la laitue, la tomate, le melon, le concombre, le pois, la graine de colza, la betterave ou le tournesol, ou l'espèce monocotylédone, comme le blé, l'orge, l'avoine, le riz ou le maïs.

8. Propagule d'une plante transgénique selon l'une quelconque des revendications 6 ou 7.

9. Propagule d'une plante transgénique selon la revendication 8, **caractérisée en ce qu'**elle est une graine.

10. Cellule contenant un promoteur ou une cassette d'expression selon l'une quelconque des revendications 1 à 4.

11. Cellule selon la revendication 10, **caractérisée en ce qu'**elle est une cellule végétale.

12. Procédé d'expression d'une séquence d'acide nucléique, ou d'un gène, codant pour un polypeptide à produire, dans une cellule, **caractérisée en ce qu'**elle comprend les étapes consistant à :
- transformer la cellule par un vecteur comprenant au moins un promoteur ou une cassette d'expression selon l'une quelconque des revendications 1 à 4 ;
- préparer une culture de la cellule dans des conditions qui permettent l'expression de la séquence d'acide nucléique, ou du gène, codant pour le polypeptide.

13. Procédé selon la revendication 12, **caractérisée en ce que** la cellule est une cellule procaryote ou eucaryote.

14. Procédé selon l'une ou l'autre des revendications 12 et 13, **caractérisée en ce que** la cellule est une cellule choisie dans le groupe comprenant des cellules microbiennes, des cellules fongiques, des cellules d'insectes, des cellules animales et des cellules végétales.

15. Procédé selon l'une quelconque des revendications 12 à 14, **caractérisée en ce que** la cellule est une cellule végétale.

16. Procédé pour obtenir une plante transgénique selon l'une quelconque des revendications 6 ou 7, ou une propagule selon la revendication 8, **caractérisée en ce qu'**elle comprend les étapes consistant à :
- transformer une cellule végétale par un vecteur comprenant au moins un promoteur ou une cassette d'expression selon l'une quelconque des revendications 1 à 4 ;
- sélectionner la cellule végétale qui a intégré le promoteur ;
- propager la cellule végétale transformée et sélectionnée soit en culture soit par régénération de plantes entières chimériques ou transgéniques.
